# EUROPEAN PATENT SPECIFICATION

(11) **EP 4 342 893 B1**
(45) Date of publication and mention of the grant of the patent: **20.08.2025**
(21) Application number: 22841126.0
(22) Date of filing: 20.06.2022
(51) Int. Cl.: C07F 15/00, H10K 85/30

(54) **TETRADENTATE CYCLOMETAL PLATINUM(II) AND PALLADIUM(II) COMPLEX LUMINESCENT MATERIAL CONTAINING QUINOLINE STRUCTURAL UNIT AND APPLICATION THEREOF**
VIERZÄHNIGES CYCLOMETALL-PLATIN(II)- UND PALLADIUM(II)-KOMPLEX-LUMINESZENZMATERIAL MIT EINER CHINOLIN-STRUKTUREINHEIT UND ANWENDUNG DAVON
MATÉRIAU LUMINESCENT COMPLEXE DE PLATINE (II) ET DE PALLADIUM (II) TÉTRADENTATE CYCLOMÉTAL CONTENANT UN MOTIF STRUCTURAL DE QUINOLÉINE ET SON UTILISATION

(30) Priority: 13.07.2021 CN 202110788173
(43) Date of publication of application: 27.03.2024
(73) Proprietor: Zhejiang University of Technology, Hangzhou, Zhejiang 310014 (CN); Zhejiang Huaxian Photoelectricity Technology Co., Ltd, Jiaxing, Zhejiang 314107 (CN)
(72) Inventor: LI, Guijie, Hangzhou, Zhejiang 310014 (CN); HUANG, Disheng, Hangzhou, Zhejiang 310014 (CN); SHE, Yuanbin, Hangzhou, Zhejiang 310014 (CN); ZHAO, Xiaoyu, Jiaxing, Zhejiang 314107 (CN)
(74) Representative: Loo, Chi Ching
(86) International application number: PCT/CN2022/099676
(87) International publication number: WO 2023/284486

(56) References cited:
- CN-A- 101 370 904
- CN-A- 103 097 395
- CN-A- 109 608 506
- CN-A- 109 970 714
- CN-A- 111 518 142
- CN-A- 111 518 142
- CN-A- 111 574 501
- CN-A- 111 662 336
- CN-A- 113 501 809
- US-A1- 2021 111 355

## Description

### TECHNICAL FIELD

The disclosure relates to the technical field of metal organic luminescent materials, in particular to a tetradentate cyclometalated platinum (II) and palladium (II) complex luminescent material containing quinoline structural units and application thereof.

### BACKGROUND ART

An Organic light-emitting diode (OLED) is a new generation of full-color display and lighting technologies. The OLED is a self-luminous device, which needs no backlight source and is energy-saving, with a low driving voltage, a fast response speed, high resolution and contrast, a wide viewing angle and a wide operating temperature range; and in which cheap glass can also be used as a substrate, and flexible display devices can be prepared if flexible plastic is used as the substrate. In addition, it has advantages of low cost, simple production processes and large-scale production. Therefore, the OLED has a wide and huge application prospect in high-end electronic products and aerospace; and there is also a huge potential market in the field of planar solid-state lighting.

Design and development of luminescent materials constitute a core of the OLED field. In early OLED devices, luminescent materials were mainly organic small molecule fluorescent materials. However, it is demonstrated in spin statistical quantum theory that in a case of electroluminescence, a ratio of singlet excitons to triplet excitons is 1:3, because only excitons in a singlet excited state can be used for traditional fluorescent materials, a theoretical internal quantum efficiency is only 25%. In 1998, phenomenon of phosphorescence of heavy-metal organic complex molecules at a room temperature is discovered by Professor Forrest of Princeton University and Professor Thompson of the University of Southern California. Due to strong spin-orbit coupling of heavy metal atoms, intersystem crossing (ISC) of excitons from a singlet state to a triplet state can be effectively facilitated for such complexes, so that all of singlet and triplet excitons generated by electrical excitation can be fully used in OLED devices, and the theoretical internal quantum efficiency of the luminescent materials can reach 100% (Nature, 1998, 395, 151). So far, development of OLED luminescent materials has entered a new period.

At present, heavy-metal phosphorescent organic complex molecules that can meet commercial applications are basically cyclometalated iridium (III) complex molecules, with a limited type. Content of metallic platinum in the earth's crust and its annual yield worldwide are about ten times that of metallic iridium, and cost (1,100 RMB/g) of IrCl₃·H₂O for preparing iridium (III) complex phosphorescent materials is also much higher than that (210 RMB/g) of PtCl₂ for preparing platinum (II) complex phosphorescent materials. In addition, the preparation of iridium (III) complex phosphorescent materials involves four-step reactions: iridium (III) dimer, iridium (III) intermediate ligand exchange, synthesis of mer-iridium (III) complex and mer-to-fac-iridium (III) complex isomer conversion, which greatly reduces a total yield, greatly reduces utilization of raw material IrCl₃·H₂O, and increase the preparation cost of the iridium (III) complex phosphorescent materials. In contrast, preparation of platinum (II) complex phosphorescent material only involves reaction of designing of ligand metallization of platinum salt in the last step above, with high utilization of platinum element, which can further reduce the preparation cost of the platinum (II) complex phosphorescent materials. To sum up, preparation cost of platinum (II) complex phosphorescent materials is much lower than that of iridium (III) complex phosphorescent materials.

Molecular rigidity of cyclometalated platinum (II) complex molecules based on bidentate ligands is low, and the two bidentate ligands are easy to be distorted and vibrate, resulting in non-radiation attenuation, which causes their phosphorescence quantum efficiency to be low. However, platinum (II) complex molecules based on tridentate ligands require a second complex anion (such as alkyne anion, Cl-, carbene, etc.), which also results in decrease in chemical stability and thermal stability of the complexes. All the above are not conducive to its application as a phosphorescent material in OLED devices. In contrast, cyclometalated platinum (II) complexes based on tetradentate ligands have strong molecular rigidity, high emission rate, greatly improved quantum efficiency, and high chemical stability and thermal stability, which are ideal molecules for developing new OLED phosphorescent materials. It is still of great significance on development of the OLED industry to develop new stable and efficient phosphorescent materials. Examples of platinum complexes with applications in OLED known in the art can be found in CN111518142.

### SUMMARY

The disclosure relates to the technical field of metal organic luminescent materials, in particular to a platinum (II) and palladium (II) complex containing quinoline structural units, which can be used as luminescent materials in OLED devices.

In a first aspect, a platinum (II) and palladium (II) complex containing quinoline structural units is provided in an embodiment of the present disclosure, which has a structure shown in following general formula (I): 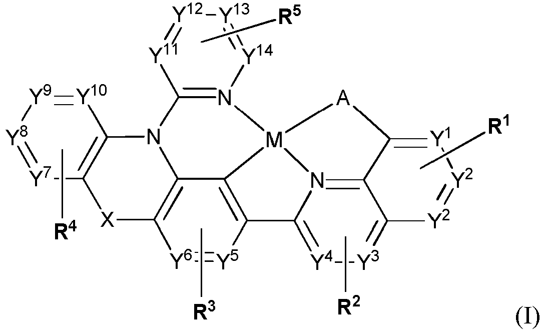 in which
M is selected from Pt or Pd;
A is selected from O, S or NR⁶;
   X can be O, S, CH₂, CHD, CD₂, CR⁷R⁸, C=O, SiR⁹R¹⁰, NH, ND, NR¹¹, PH, PD, PR¹², R¹³P=O, S=O, or SO₂;
Y¹, Y², Y³, Y⁴, Y⁵, Y⁶, Y⁷, Y⁸, Y⁹, Y¹⁰, Y¹¹, Y¹², Y¹³ and Y¹⁴ are each independently N or CH;
   substitution modes of R¹, R², R³, R⁴, R⁵, R⁶, R⁷, R⁸, R⁹, R¹⁰, R¹¹, R¹² and R¹³ are each independently expressed as single substitution, double substitution, triple substitution, four substitution or no substitution; R¹, R², R³, R⁴, R⁵, R⁶, R⁷, R⁸, R⁹, R¹⁰, R¹¹, R¹² and R¹³ each independently represent hydrogen, deuterium, alkyl, haloalkyl, cycloalkyl, alkoxy, aryl, heteroaryl, aryloxy, halogen, cycloalkenyl, heterocyclyl, alkenyl, alkynyl, hydroxyl, mercapto, nitro, cyano, amino, mono-or dialkylamino, mono-or diarylamino, ester, nitrile, isonitrile, heteroaryl, alkoxycarbonyl, amido, alkoxycarbonylamino, aryloxycarbonylamino, sulfonylamino, sulfamoyl, carbamoyl, alkylthio, sulfinyl, ureido, phosphoramido, imino, sulfo, carboxyl, hydrazino, silyl, substituted silyl, polymeric groups, or combinations thereof, and two or more adjacent R¹, R², R³, R⁴, R⁵, R⁶, R⁷, R⁸, R⁹, R¹⁰, R¹¹, R¹² and R¹³ can be selectively linked to form a condensed ring.

Further, the metal platinum (II) and palladium (II) complex containing quinoline structural units is characterized in that the platinum complex has structures shown in following general formula (II), (III), (IV) or (V): 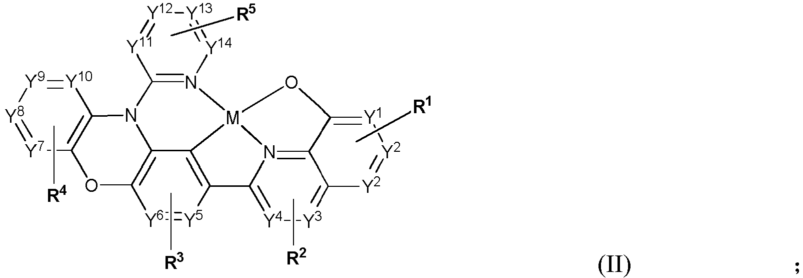 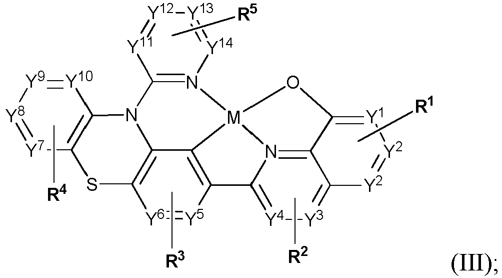 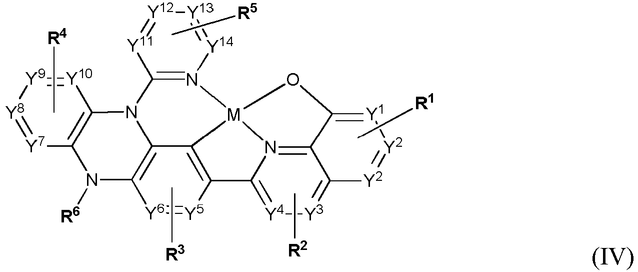 or

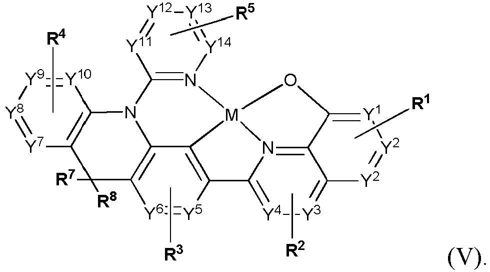

Further, the metal platinum (II) and palladium (II) complex containing quinoline structural units is characterized in that left 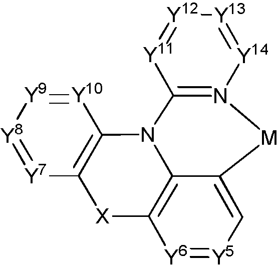 is preferably of a structure selected from one of following structures:

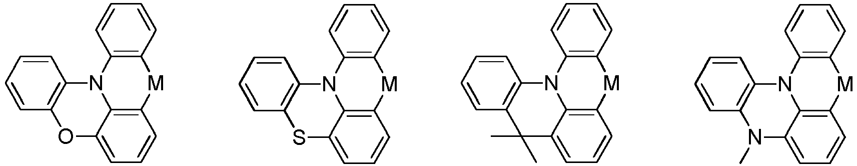

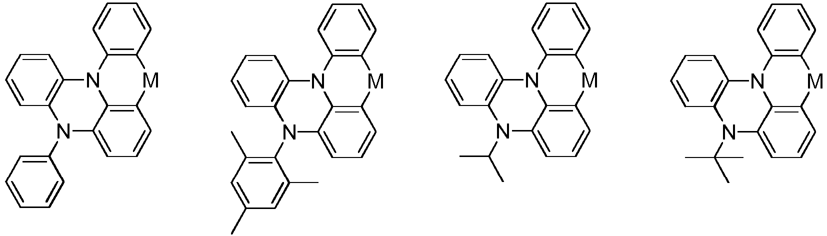

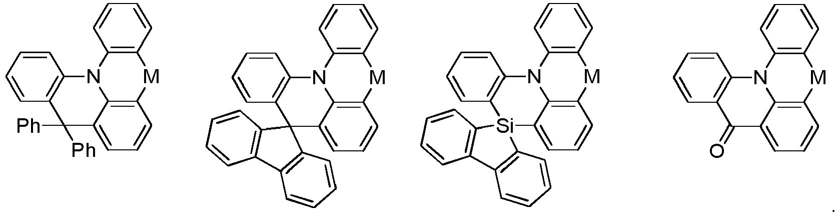

Further, the metal platinum (II) and palladium (II) complex containing quinoline structural units is characterized in that the platinum (II) and palladium (II) complex has one of following structures:

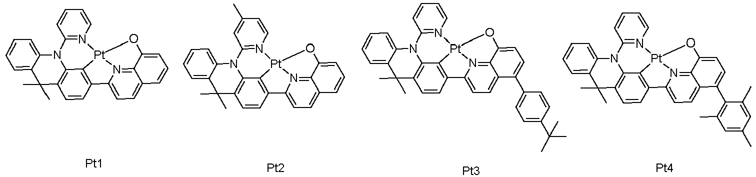

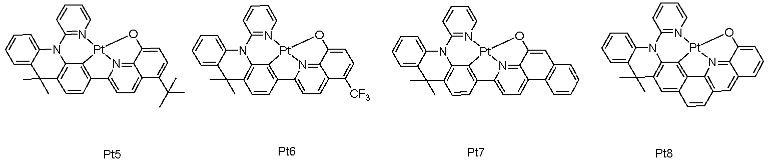

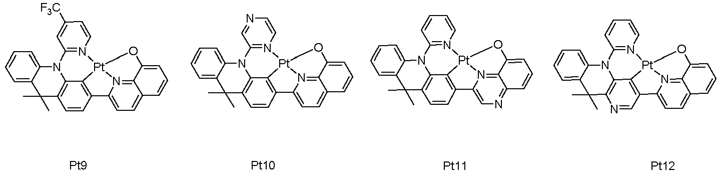

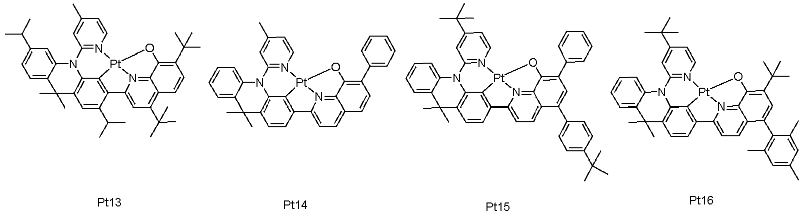

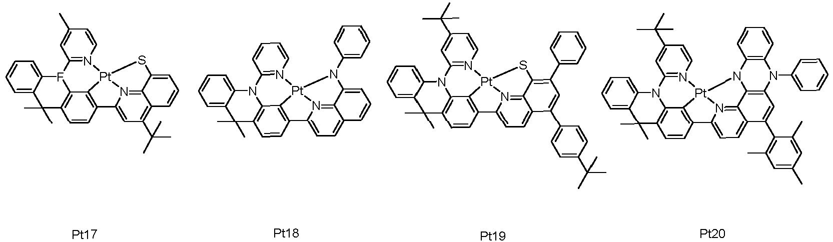

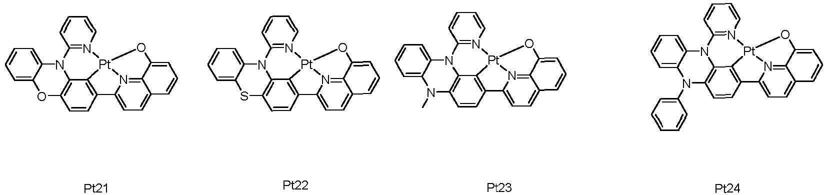

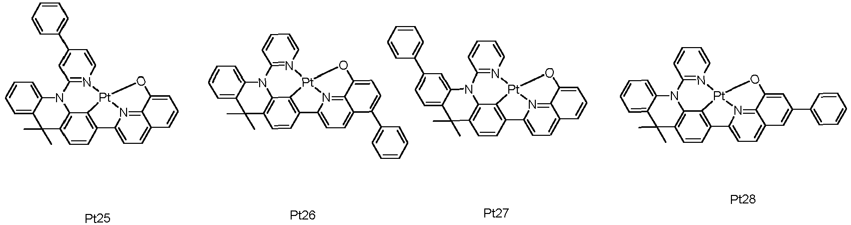

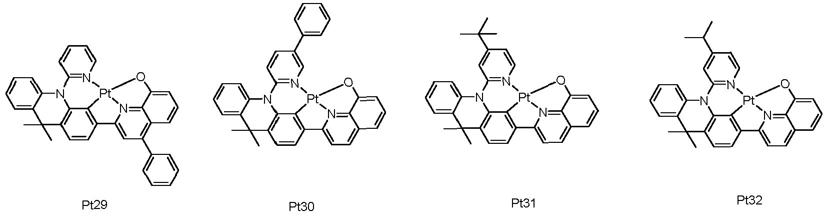

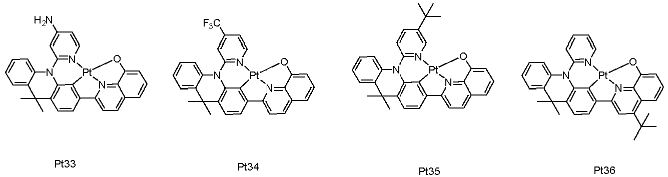

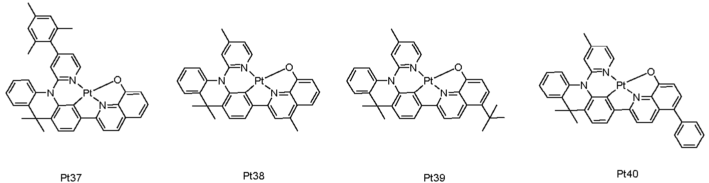

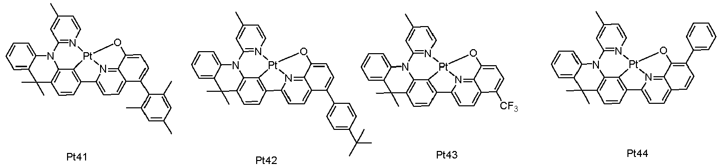

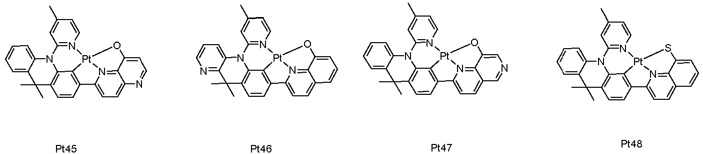

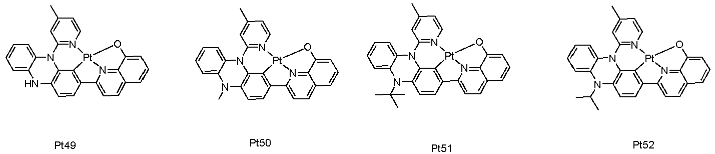

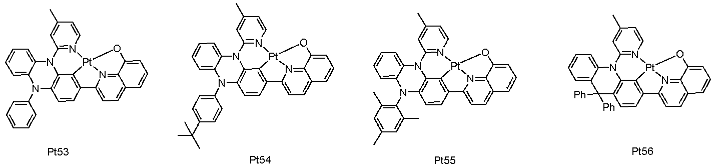

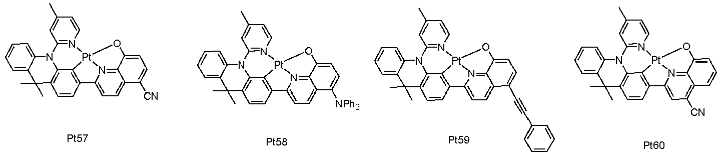

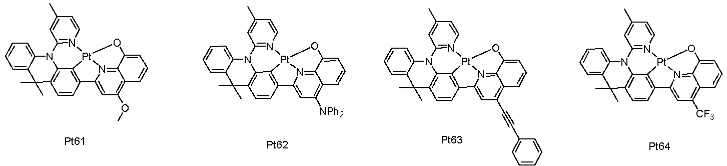

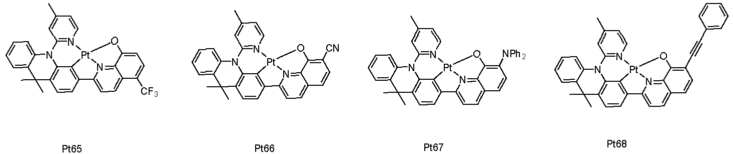

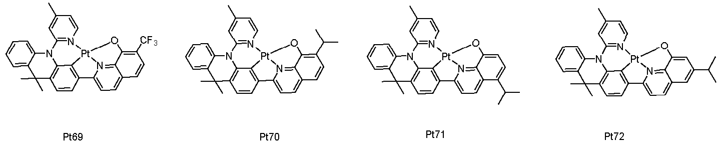

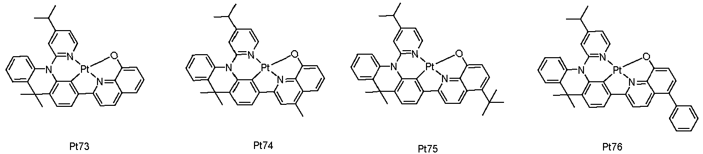

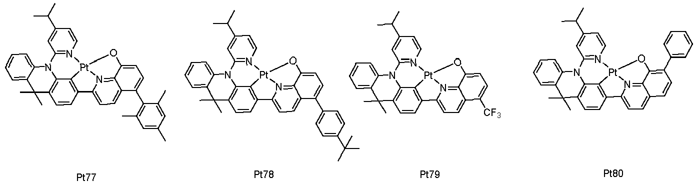

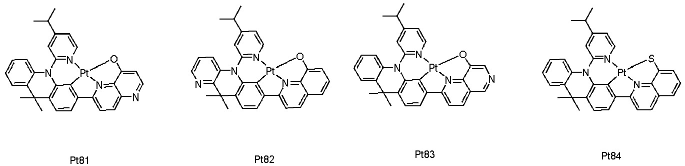

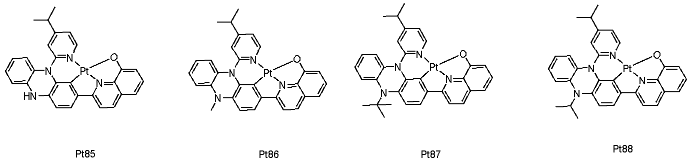

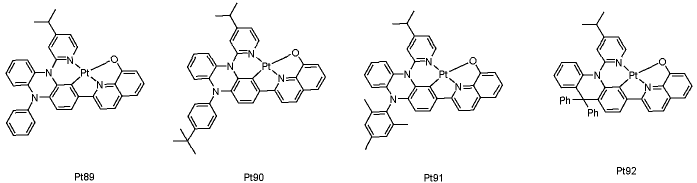

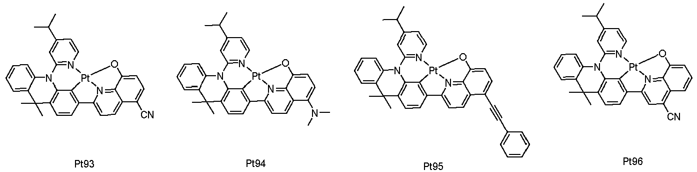

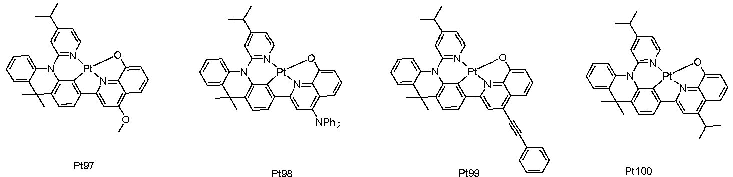

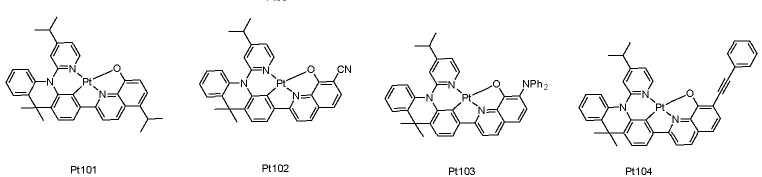

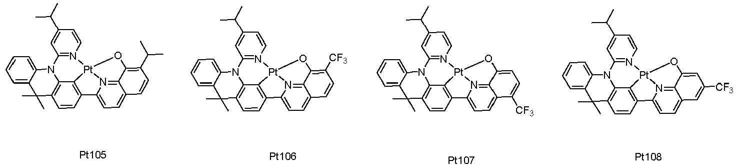

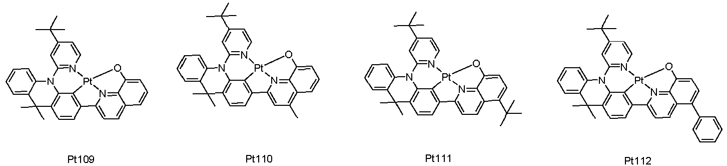

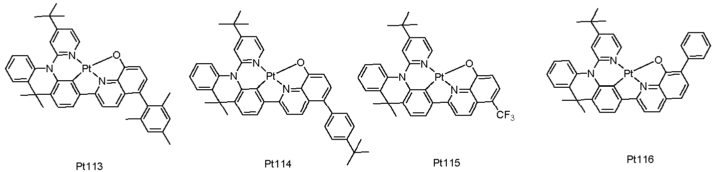

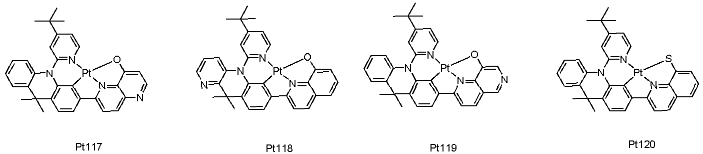

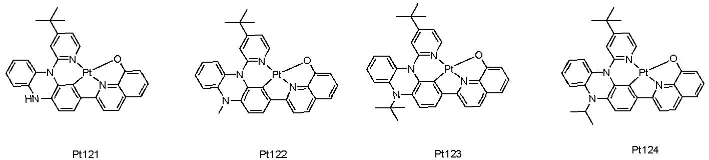

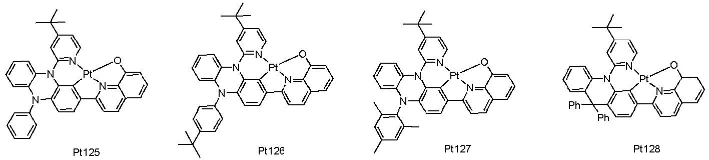

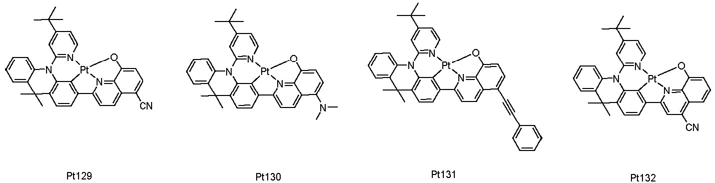

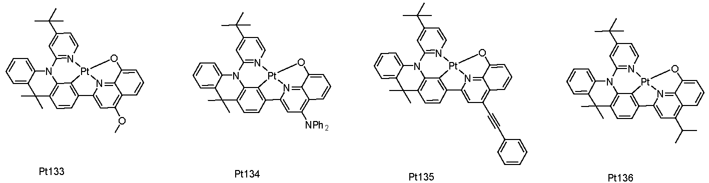

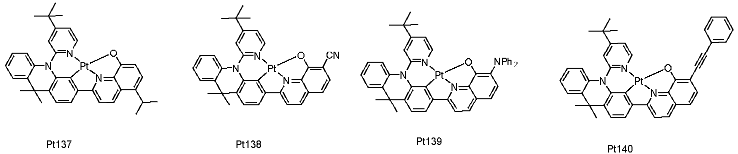

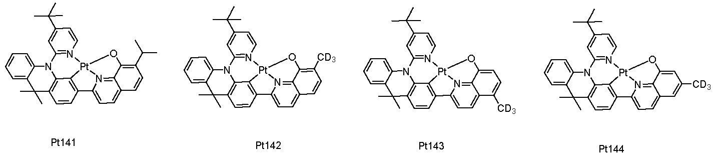

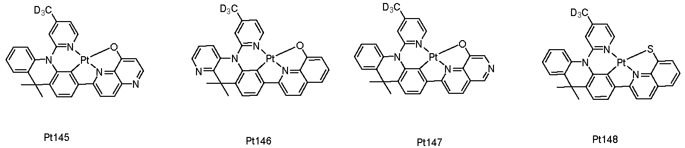

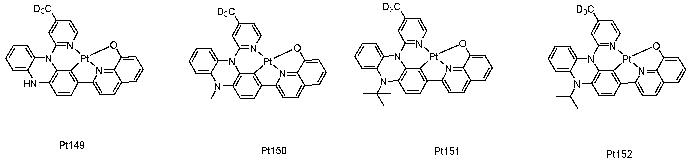

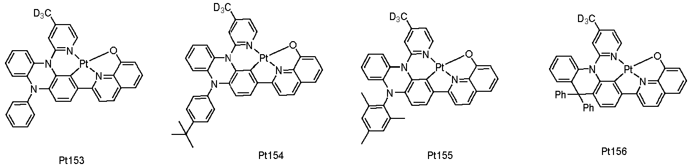

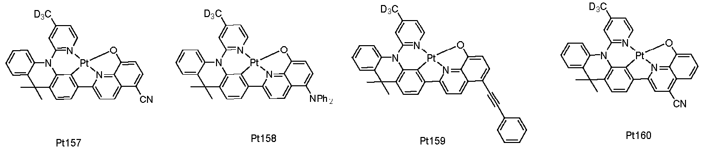

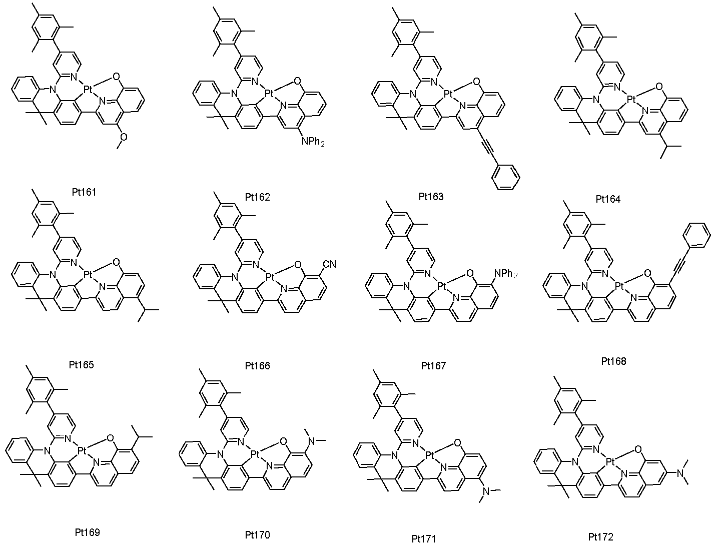

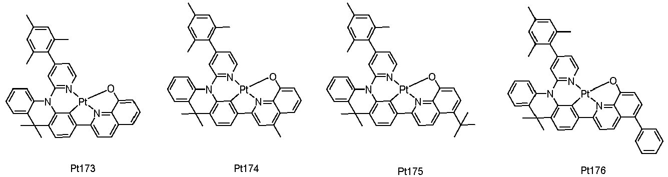

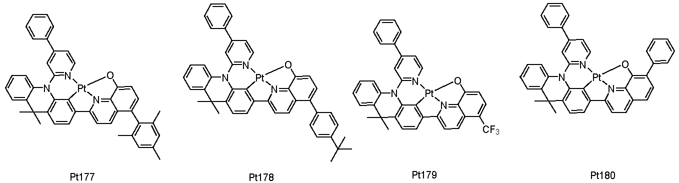

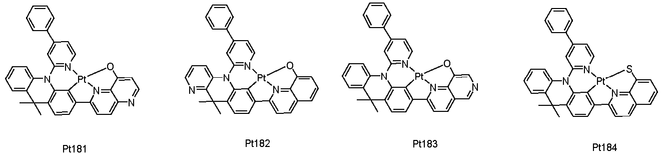

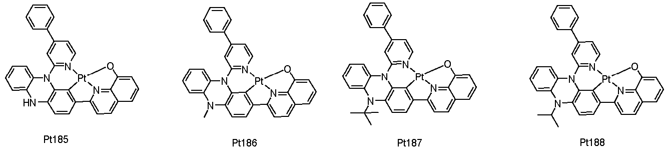

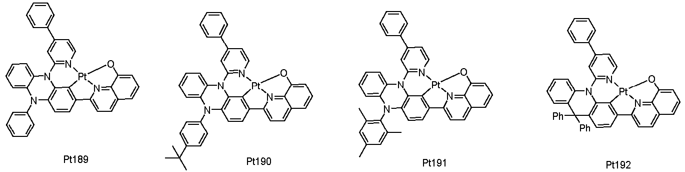

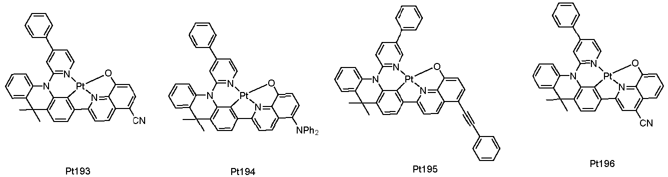

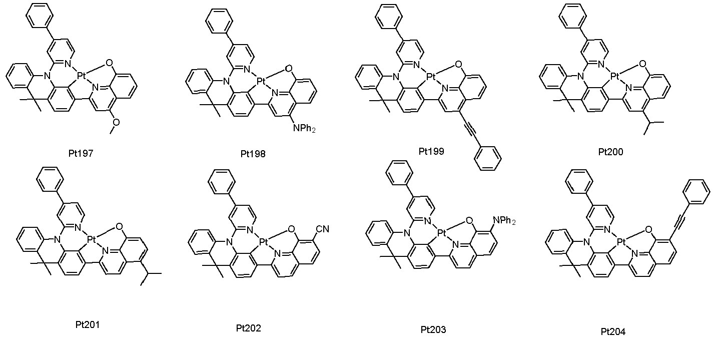

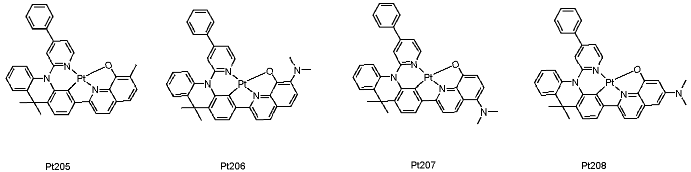

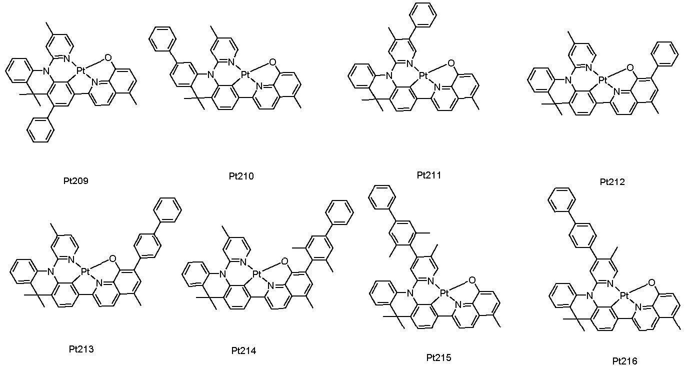

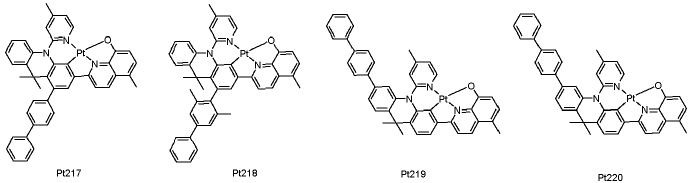

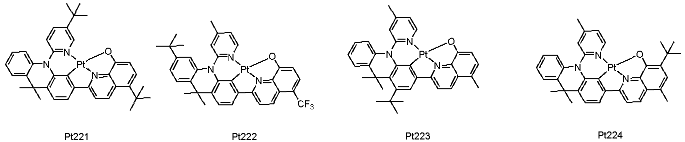

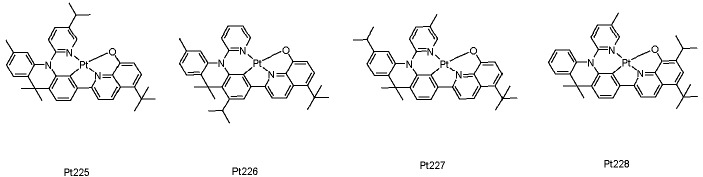

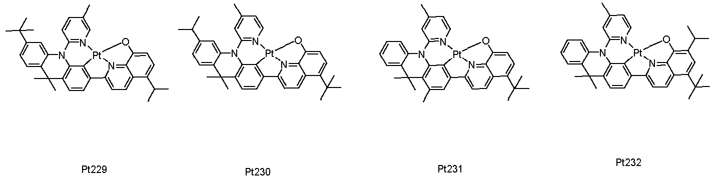

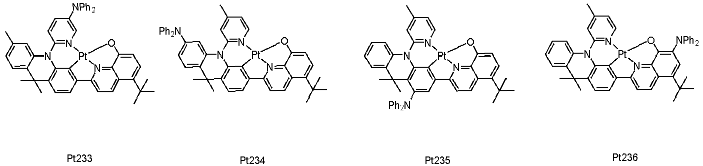

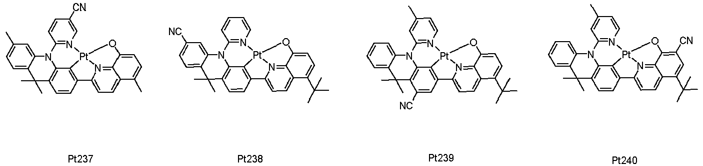

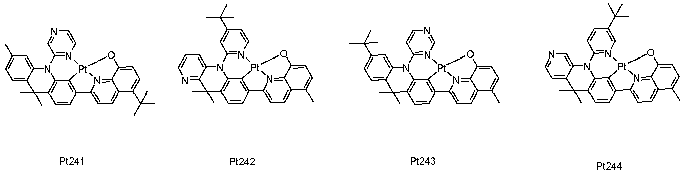

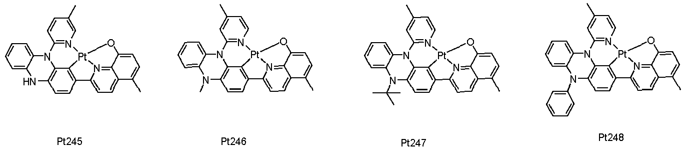

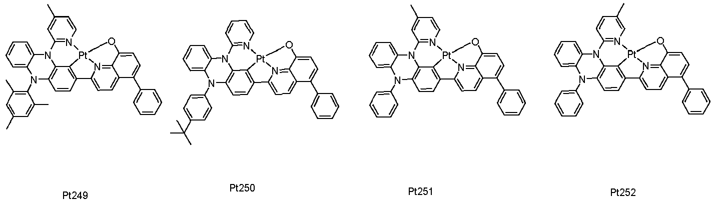

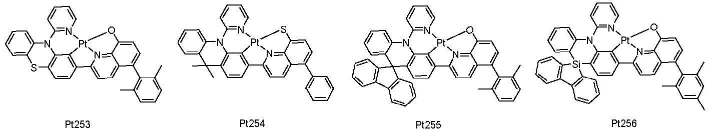

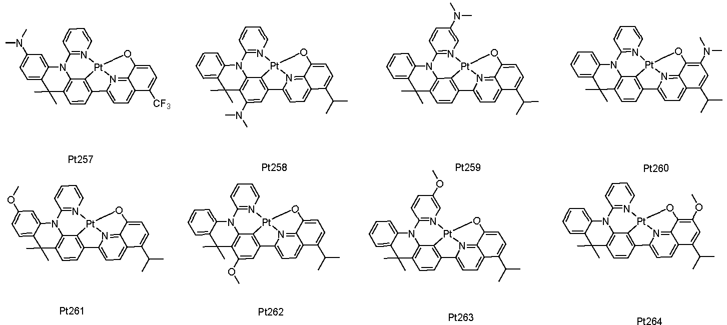

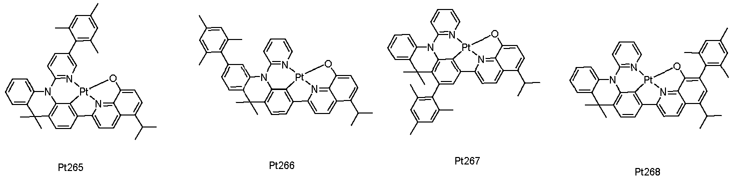

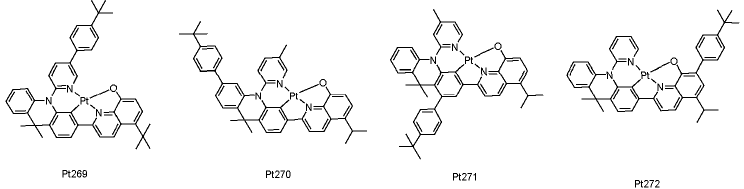

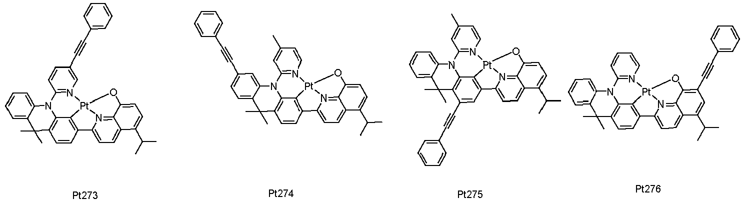

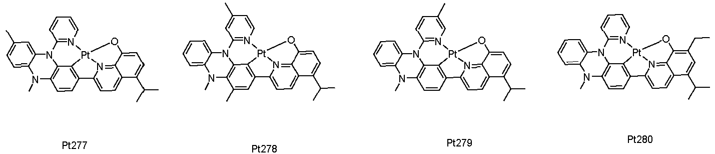

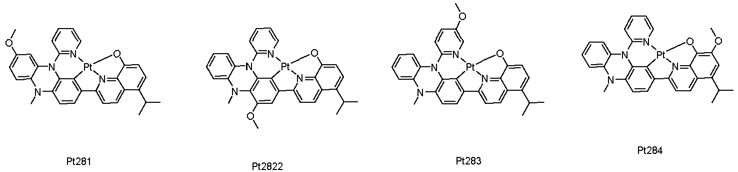

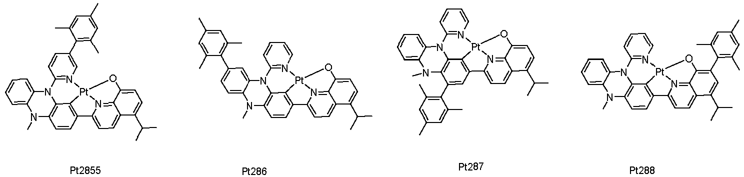

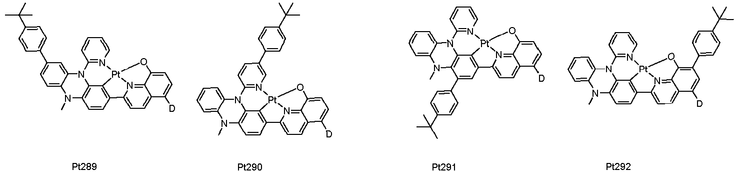

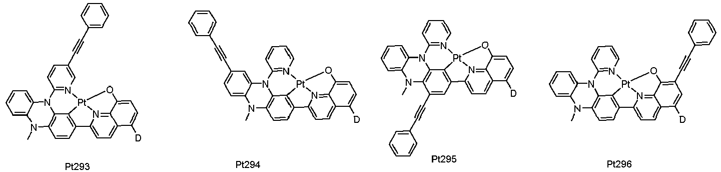

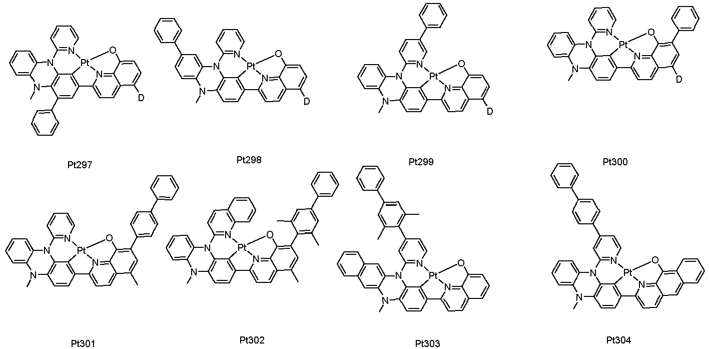

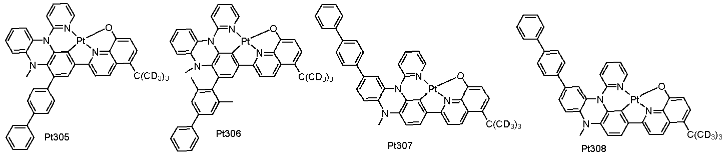

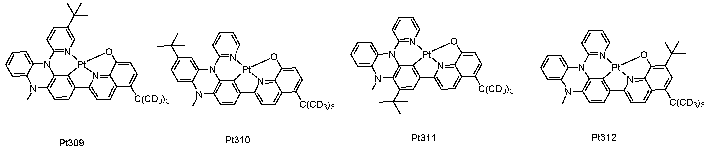

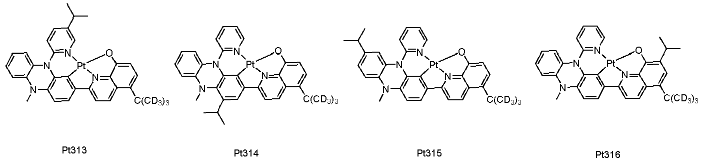

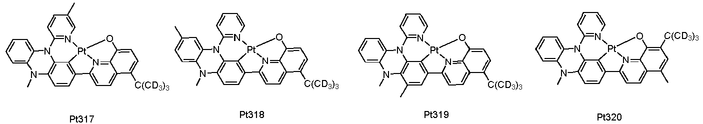

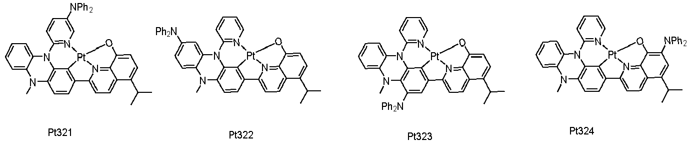

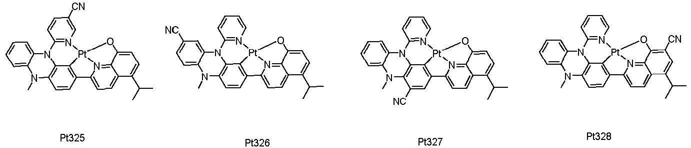

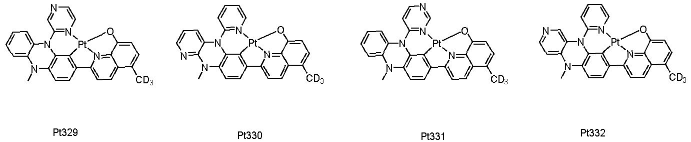

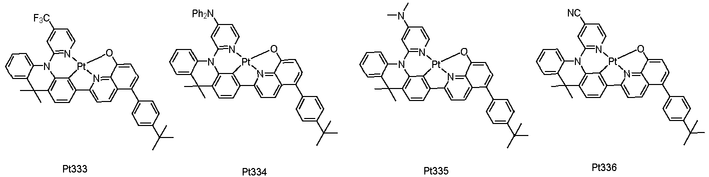

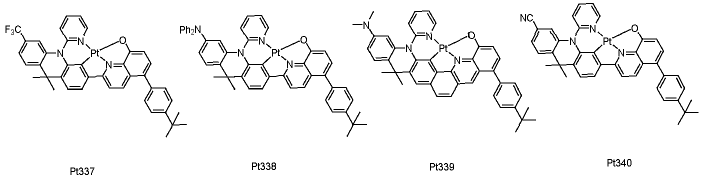

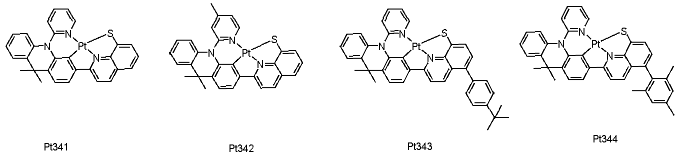

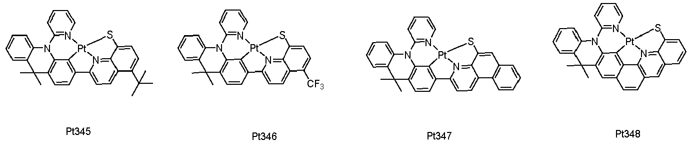

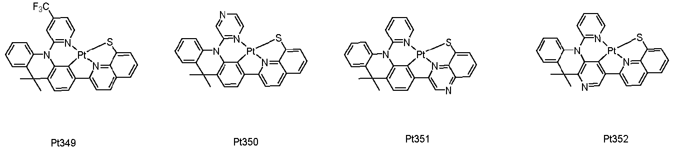

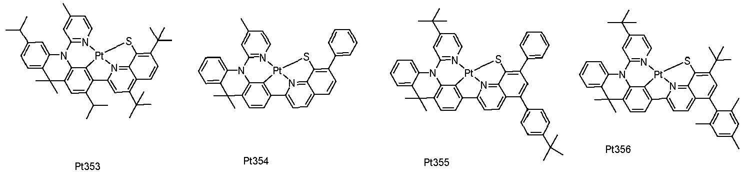

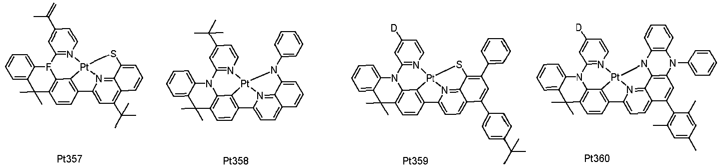

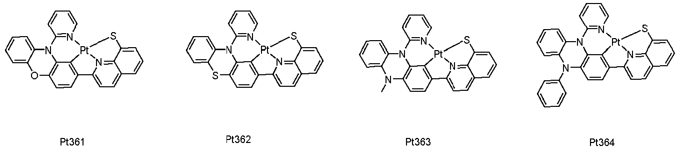

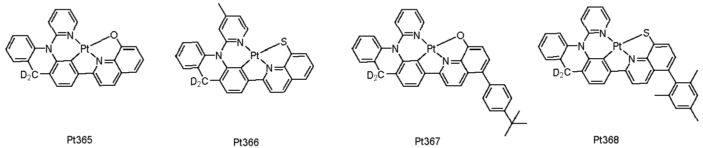

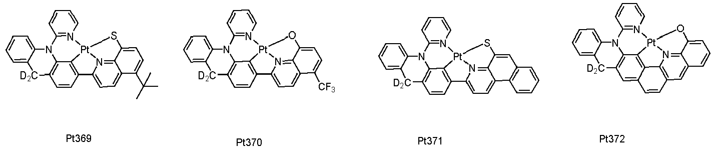

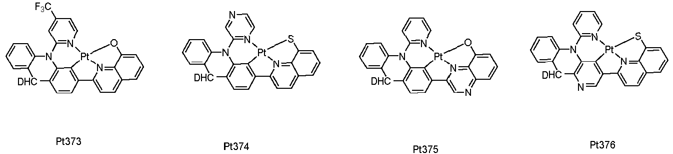

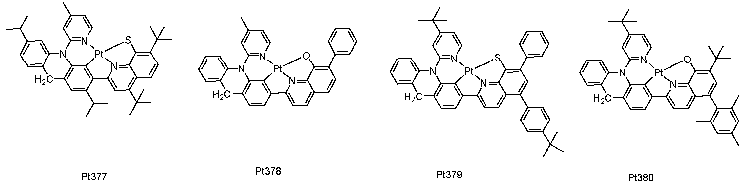

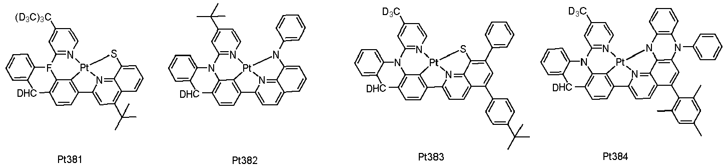

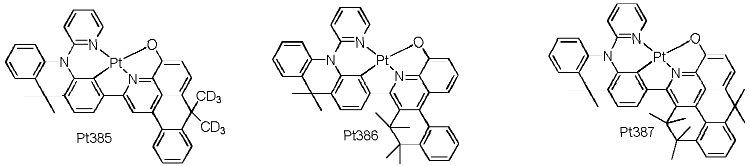

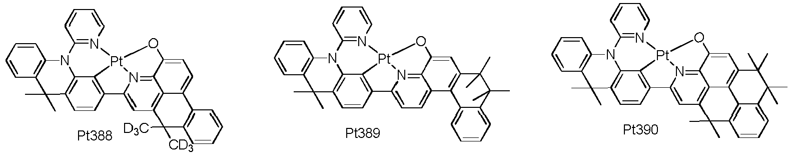

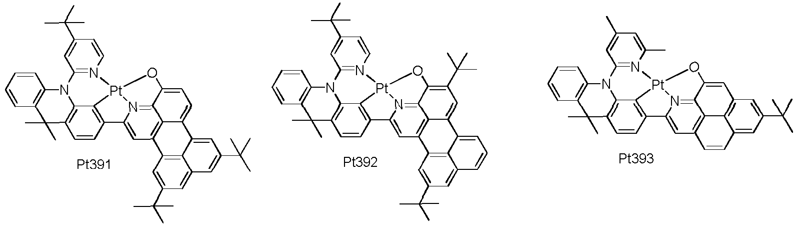

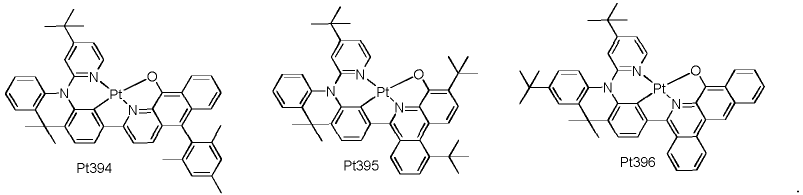

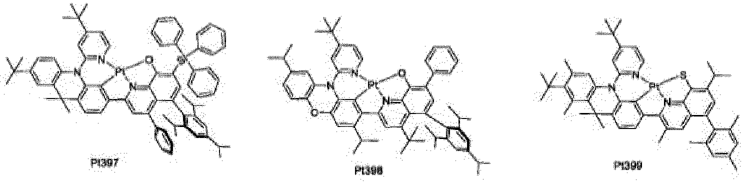

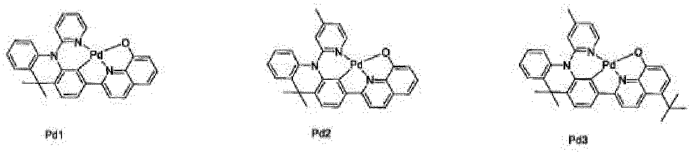

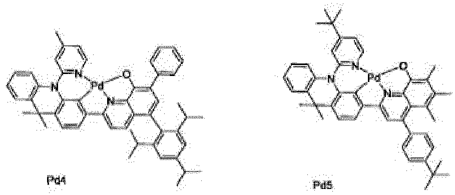

In the present disclosure, the metal platinum (II) and palladium (II) complex containing quinoline structural units is used as luminescent materials and can be used in electroluminescent devices. Further, it can be used in full-color displays, light-emitting display devices or organic light-emitting diodes. The device is characterized by including at least one cathode, at least one anode and at least one luminescent layer. At least one of at least one luminescent layer includes any one of the metal platinum (II) and palladium (II) complexes containing quinoline structural units described above.

The disclosure has beneficial effects that: compared with bidentate ligand platinum complexes, cyclometalated platinum (II) complex molecules based on tetradentate ligands has strong molecular rigidity, can effectively suppress non-radiative transition due to molecular vibration, and greatly improve the quantum efficiency. Meanwhile, the tetradentate ligands facilitates improving of chemical stability and thermal stability of the material molecules. According to the disclosure, photophysical properties of the metal platinum complex can be adjusted by changing a ligand structure around a metal center and regulating substituent structures on the ligands. It has broad application prospects in many fields such as OLED display and lighting.

### BRIEF DESCRIPTION OF THE DRAWINGS

In order to explain technical schemes in the embodiments of the present disclosure more clearly, the drawings required in the description of the embodiments will be briefly introduced below; obviously, the drawings in the following description are only some embodiments of the present disclosure, and other drawings can be obtained according to these drawings by those of ordinary skill in the art without paying creative labor, in which
FIG. 1 is a room-temperature emission spectrum of platinum complex Pt1 in a dichloromethane solution according to an embodiment;
FIG. 2 is a room-temperature emission spectrum of platinum complex Pt2 in a dichloromethane solution according to an embodiment;
FIG. 3 is a room-temperature emission spectrum of platinum complex Pt3 in a dichloromethane solution according to an embodiment;
FIG. 4 is a room-temperature emission spectrum of platinum complex Pt42 in a dichloromethane solution according to an embodiment;
FIG. 5 shows HOMO and LUMO orbital distributions of Pt1, Pt2, Pt3 and Pt4 calculated according to density functional theory (DFT) and their energy level comparison;
FIG. 6 shows HOMO and LUMO orbital distributions of Pt5, Pt6, Pt7 and Pt8 calculated according to density functional theory (DFT) and their energy level comparison;
FIG. 7 shows HOMO and LUMO orbital distributions of Pt9, Pt10, Pt11 and Pt12 calculated according to density functional theory (DFT) and their energy level comparison;
FIG. 8 shows HOMO and LUMO orbital distributions of Pt13, Pt14, Pt15 and Pt16 calculated according to density functional theory (DFT) and their energy level comparison;
FIG. 9 shows HOMO and LUMO orbital distributions of Pt17, Pt18, Pt19 and Pt20 calculated according to density functional theory (DFT) and their energy level comparison;
FIG. 10 shows HOMO and LUMO orbital distributions of Pt21, Pt22, Pt23 and Pt24 calculated according to density functional theory (DFT) and their energy level comparison.
FIG. 11 shows HOMO and LUMO orbital distributions of Pt397, Pt398, Pt399 and Pd1 calculated according to density functional theory (DFT) and their energy level comparison.
FIG. 12 shows HOMO and LUMO orbital distributions of Pd2, Pd3, Pd4 and Pd5 calculated according to density functional theory (DFT) and their energy level comparison.
FIG. 13 is a structural diagram of an organic light emitting element.

### DETAILED DESCRIPTION

Before compounds, devices, and/or methods of the present disclosure are disclosed and described, it should be understood that they are not limited to specific synthetic methods (unless otherwise specified) or specific reagents (unless otherwise specified), as these may vary. It should also be understood that terminologies used in the present disclosure is for a purpose of describing specific aspects only and is not intended to be limiting. While any method and material similar or equivalent to those described herein may be used in this practice or test, example methods and materials are described below.

A term "optional" or "optionally" as used herein means that a subsequently described event or condition may or may not occur, and that such description includes both instances in which the described event or condition occurs and instances in which it does not.

Components useful in preparing compositions of the present disclosure are disclosed, as well as the compositions themselves to be used in the methods disclosed in the present disclosure. These and other substances are disclosed in the present application, and it should be understood that when combinations, subsets, interactions, groups, etc. of these substances are disclosed, but specific reference to each and every individual and total combination and substitution of these compounds cannot be specifically disclosed, each is specifically contemplated and described in the present disclosure. For example, if a specific compound is disclosed and discussed, as well as many modifications that can be made to many molecules containing the compound, various and every combination and substitution of the compound are specifically contemplated and the modification may be made, otherwise it will be specifically indicated to the contrary. Thus, if examples of a type of molecules A, B, and C and a type of molecules D, E, and F, and combined molecules A-D are disclosed, it is contemplated that respective individual and general expected combination of meanings, A-E, A-F, B-D, B-E, B-F, C-D, C-E and C-F, are disclosed, even if each of them is not individually recited. Likewise, any subset or combination of these are also disclosed. Therefore, for example, disclosure of combinations A-E, B-F and C-E should be contemplated. These concept applies to all aspects of the present disclosure, including, but not limited to, steps in methods of making and using the composition. Thus, if there are various additional steps that can be performed, it should be understood that each of these additional steps can each be performed in a particular implementation of the method or a combination of implementations thereof.

A linking atom used in the present disclosure is capable of linking two groups, for example, N and C groups. The connecting atom can optionally (if available for the valence bond) have other attached chemical moieties. For example, on one hand, an oxygen atom will not have any other chemical group attachment since once two atoms (e. g., N or C) are bonded, the valence bonds are already fully used. In contrast, when the linking atom is carbon, two additional chemical moieties can be attached to the carbon atom. Suitable chemical moieties include, but are not limited to, hydrogen, hydroxyl, alkyl, alkoxy, =O, halogen, nitro, amine, amide, mercapto, aryl, heteroaryl, cycloalkyl, and heterocyclyl.

A term "cyclic structure" or the like as used herein refers to any cyclic chemical structure including, but not limited to, aryl, heteroaryl, cycloalkyl, cycloalkenyl, heterocyclyl, carbene and N- heterocyclic carbene.

A term "substituted" as used herein is intended to include all permissible substituents of an organic compound. In a broad aspect, permissible substituents include acyclic and cyclic, branched and unbranched, carbocyclic and heterocyclic, and aromatic and nonaromatic substituents of organic compounds. Illustrative substituents include, for example, those described below. For suitable organic compounds, the permissible substituents may be one or more, the same or different. For a purposes of the present disclosure, heteroatom (e. g., nitrogen) can have a hydrogen substituent and/or any permissible substituent of the organic compound of the present disclosure satisfying valence bonds of the heteroatom. The present disclosure is not intended to be limiting in any way with the permissible substituents of the organic compound. As such, a term "substituted" or "substituted with" encompasses an implicit condition that such a substitution conforms to permissible valence bonds of a substituted atom and the substituent, and that the substitution results in stable compounds (e. g., compounds that do not spontaneously undergo conversion (e. g., by rearrangement, cyclization, elimination, etc.)). It is also contemplated that in certain aspects, individual substituents can be further optionally substituted (i.e., further substituted or unsubstituted) unless explicitly indicated to the contrary.

A term "alkyl" as used herein is a branched or unbranched saturated hydrocarbon group with 1 to 24 carbon atoms, such as methyl, ethyl, n-propyl, isopropyl, n-butyl, isobutyl, sec-butyl, tert-butyl, n-pentyl, isopentyl, sec-pentyl, neopentyl, hexyl, heptyl, octyl, nonyl, decyl, dodecyl, tetradecyl, hexadecyl, eicosyl, tetracosyl and the like.

Throughout this specification, "alkyl" is generally used to refer to both unsubstituted and substituted alkyl. However, substituted alkyl is also specifically mentioned in this present disclosure by determining specific substituents on the alkyl. For example, a term "halogenated alkyl" or "haloalkyl" specifically refers to an alkyl substituted with one or more halogens (e.g., fluorine, chlorine, bromine, or iodine). A term "alkoxyalkyl" specifically refers to an alkyl substituted with one or more alkoxy, as described below. A term "alkylamino" specifically refers to an alkyl substituted with one or more amino, as described below or the like. When "alkyl" is used in one case and specific terms such as "alkyl alcohol" are used in another case, it is not intended to imply that the term "alkyl" does not simultaneously refer to specific terms such as "alkyl alcohol" or the like.

This practice is also applicable to other groups described herein. That is, when terms such as "cycloalkyl" refer to both unsubstituted and substituted cycloalkane moieties, the substituted moiety may additionally be specifically determined in the present disclosure. For example, a specifically substituted cycloalkyl may be referred to as, for example, "alkyl cycloalkyl". Similarly, a substituted alkoxy may be specifically referred to as, for example, "haloalkoxy" and a specific substituted alkenyl may be, for example, "enol" or the like. Similarly, practice of using a general term such as "cycloalkyl" and a specific term such as "alkylcycloalkyl" is not intended to imply that the general term does not also include the specific term.

The term "cycloalkyl" as used herein is a non-aromatic carbon-based ring composed of at least three carbon atoms. Examples of cycloalkyl include, but are not limited to, cyclopropyl, cyclobutyl, cyclopentyl, cyclohexyl, cyclononyl, and the like. A term "heterocycloalkyl" is a type of cycloalkyl as defined above and is included in meaning of the term "cycloalkyl" in which at least one ring carbon atom is substituted by a heteroatom such as, but not limited to, a nitrogen, oxygen, sulfur or phosphorus atom. The cycloalkyl and heterocycloalkyl may be substituted or unsubstituted. The cycloalkyl and heterocycloalkyl may be substituted with one or more groups including, but not limited to, alkyl, cycloalkyl, alkoxy, amino, halogen, hydroxy, nitro, silyl, sulfo-oxo and thiol as described herein.

A term "polyolefin group" is used in the present disclosure to refer to a group containing two or more CH₂ groups and connected with other identical parts. The "polyolefin group" may be expressed as -(CH₂)ₐ-, where "a" is an integer between 2 and 500.

Terms "alkoxy" and "alkoxy group" as used herein refer to an alkyl or cycloalkyl bonded by ether connecting groups. That is, "alkoxy" may be defined as -OR¹, where R¹ is an alkyl or cycloalkyl as defined above. "Alkoxy" further includes alkoxy polymers just described. That is, the alkoxy may be a polyether such as -OR¹-OR² or -OR¹- (OR²)ₐ-OR³, where "a" is an integer between 1 to 200, and R¹, R² and R³ are each independently an alkyl, a cycloalkyl, or a combination thereof.

A term "alkenyl" as used herein is a hydrocarbon of 2 to 24 carbon atoms with a structural formula containing at least one carbon-carbon double bond. An asymmetric structure such as (R¹R²)C=C(R³R⁴) is intended to contain E and Z isomers. This can be inferred in a structural formula of the present disclosure in which an asymmetric olefin is present, or it can be explicitly expressed by a bond symbol C=C. The alkenyl may be substituted with one or more groups including, but not limited to, alkyl, cycloalkyl, alkoxy, alkenyl, cycloalkenyl, alkynyl, cycloalkynyl, aryl, heteroaryl, aldehyde, amino, carboxyl, ester, ether, halogen, hydroxy, carbonyl, azido, nitro, silyl, sulfo-oxo or thiol as described herein.

A term "cycloalkenyl" as used herein is a non-aromatic carbon-based ring, which is composed of at least 3 carbon atoms and contains at least one carbon-carbon double bond, that is, C=C. Examples of cycloalkenyl include, but are not limited to, cyclopropenyl, cyclobutenyl, cyclopentenyl, cyclopentadienyl, cyclohexenoyl, cyclohexadienyl,norbornenyl, and the like. A term "heterocyclic alkenyl" is a type of cycloalkenyl as defined above and is included in meaning of the term "cycloalkenyl" in which at least one carbon atom of the ring is substituted with a heteroatom such as, but not limited to, a nitrogen, oxygen, sulfur or phosphorus atom. The cycloalkenyl and heterocyclic alkenyl may be substituted or unsubstituted. The cycloalkenyl and heterocyclic alkenyl may be substituted with one or more groups including, but not limited to, alkyl, cycloalkyl, alkoxy, alkenyl, cycloalkenyl, alkynyl, cycloalkynyl, aryl, heteroaryl, aldehyde, amino, carboxyl, ester, ether, halogen, hydroxy, carbonyl, azido, nitro, silyl, sulfo-oxo or thiol as described herein.

A term "alkynyl" as used herein is a hydrocarbon having 2 to 24 carbon atoms, which has a structural formula containing at least one carbon-carbon triple bond. The alkynyl may be substituted with one or more groups including, but not limited to, alkyl, cycloalkyl, alkoxy, alkenyl, cycloalkenyl, alkynyl, cycloalkynyl, aryl, heteroaryl, aldehyde, amino, carboxyl, ester, ether, halogen, hydroxy, carbonyl, azido, nitro, silyl, sulfo-oxo or thiol as described herein.

A term "cycloalkynyl" as used herein is a non-aromatic carbon-based ring containing at least seven carbon atoms and at least one carbon-carbon triple bond. Examples of cycloalkynyl include, but are not limited to, cycloheptynyl, cyclooctynyl, cyclononynyl, and the like. A term "heterocyclic alkynyl" is a cycloalkenyl as defined above and is included within meaning of the term "cycloalkynyl" in which at least one of the carbon atoms of the ring is replaced by a heteroatom such as, but not limited to, a nitrogen, oxygen, sulfur or phosphorus atom. The cycloalkynyl and heterocyclic alkynyl may be substituted or unsubstituted. The cycloalkynyl and heterocyclic alkynyl may be substituted with one or more groups including, but not limited to, alkyl, cycloalkyl, alkoxy, alkenyl, cycloalkenyl, alkynyl, cycloalkynyl, aryl, heteroaryl, aldehyde, amino, carboxyl, ester, ether, halogen, hydroxy, carbonyl, azido, nitro, silyl, sulfo-oxo or thiol as described herein.

A term "aryl" as used herein is a group containing any carbon-based aromatic group, including but not limited to benzene, naphthalene, phenyl, biphenyl, phenoxybenzene, and the like. A term "aryl" also includes "heteroaryl", which is defined as a group containing an aromatic group having at least one heteroatom introduced into a ring of the aromatic group. Examples of heteroatoms include, but are not limited to, a nitrogen, oxygen, sulfur, and phosphorus atom. Likewise, a term "non-heteroaryl" (which is also included in the term "aryl") defines an aromatic-containing group that is free of heteroatoms. The aryl may be substituted or unsubstituted. The aryl may be substituted with one or more groups including, but not limited to, alkyl, cycloalkyl, alkoxy, alkenyl, cycloalkenyl, alkynyl, cycloalkynyl, aryl, heteroaryl, aldehyde, amino, carboxyl, ester, ether, halogen, hydroxy, carbonyl, azido, nitro, silyl, sulfo-oxo or thiol as described herein. A term "biaryl" is a particular type of aryl and is included in definition of "aryl". The biaryl refers to two aryl joined together by a fused ring structure, as in naphthalene, or two aryl joined by one or more carbon-carbon bonds, as in biphenyl.

A term "aldehyde" as used herein is represented by a formula -C(O)H. Throughout this specification, "C(O)" is a short form of carbonyl (i.e., C=O).

A term "amine" or "amino" as used herein is represented by the formula -NR¹R², where R¹ and R² may independently be selected from hydrogen, alkyl, cycloalkyl, alkenyl, cycloalkenyl, alkynyl, cycloalkynyl, aryl or heteroaryl.

A term "alkylamino" as used herein is represented by a formula -NH(-alkyl), where the alkyl is as described in the present disclosure. Representative examples include, but are not limited to, methylamino, ethylamino, propylamino, isopropylamino, butylamino, isobutylamino, sec-butylamino, tert-butylamino, pentylamino, isopentylamino, tert-pentylamino, hexylamino and the like.

A term "alkylamino" as used herein is represented by a formula -N(-alkyl)₂, where the alkyl is as described in the present disclosure. Representative example include, but are not limited to, dimethylamino, diethylamino, dipropylamino, diisopropylamino, dibutylamino, diisobutylamino, di-sec-butylamino, di-tert-butylamino, dipentylamino, diisopentylamino, di-tert-pentylamino, dihexylamino, N-ethyl-N-methylamino, N-methyl-N-propylamino, N-ethyl-N-propylamino and the like.

A term "carboxylic acid" as used herein is represented by a formula -C(O)OH.

A term "ester" as used herein is represented by a formula -OC(O)R¹ or -C(O)OR¹, where R1 may be alkyl, cycloalkyl, alkenyl, cycloalkenyl, alkynyl, cycloalkynyl, aryl or heteroaryl as described herein. A term "polyester" as used herein is represented by a formula -(R¹O(O)C-R²-C(O)O)ₐ- or -(R¹O(O)C-R²-OC(O))ₐ-, where R¹ and R² can independently be alkyl, cycloalkyl, alkenyl, cycloalkenyl, alkynyl, cycloalkynyl, aryl or heteroaryl as described herein and "a" is an integer between 1 to 500. The term "polyester" is used to describe groups produced by reaction between a compound having at least two carboxyl and a compound having at least two hydroxy.

A term "ether" as used herein is represented by a formula R¹OR², where R¹ and R² may independently be alkyl, cycloalkyl, alkenyl, cycloalkenyl, alkynyl, cycloalkynyl, aryl or heteroaryl as described herein. A term "polyether" as used herein is represented by a formula -(R¹O-R²O)ₐ-, where R¹ and R² may independently be alkyl, cycloalkyl, alkenyl, cycloalkenyl, alkynyl, cycloalkynyl, aryl or heteroaryl as described herein and "a" is an integer between 1 to 500. Examples of polyether groups include polyethylene oxide, polypropylene oxide and polybutylene oxide.

A term "halogen" as used herein refers to halogen fluorine, chlorine, bromine and iodine.

A term "heterocyclyl" as used herein refers to monocyclic and polycyclic non-aromatic ring systems, and "heteroaryl" as used herein refers to monocyclic and polycyclic aromatic ring systems, where at least one of ring members is not carbon. This term includes azetidinyl, dioxane, furyl, imidazolyl, isothiazolyl, isoxazolyl, morpholinyl, oxazolyl (oxazolyl including 1,2,3- oxadiazole, 1,2,5- oxadiazole and 1,3,4- oxadiazole), piperazinyl, piperidyl, pyrazinyl, pyrazolyl, pyridazinyl, pyridinyl, pyrimidinyl, pyrrolyl, pyrrolidinyl, tetrahydrofuran, tetrahydropyranyl, tetrazinyl including 1,2,4, 5- tetrazinyl, tetrazolyl including 1,2,3,4- tetrazolyl and 1,2,4,5-tetrazolyl, thiadiazole including 1,2,3- thiadiazole, 1,2,5- thiadiazole and 1,3,4-thiadiazole, thiazolyl, thienyl, triazinyl including 1,3,5- triazinyl and 1,2,4- triazinyl, thiadiazolyl including 1,2,3- triazolinyl and 1,3,4- triazolinyl, and the like.

A term "hydroxyl" as used herein is represented by a formula -OH.

A term "ketone" as used herein is represented by a formula R¹C(O)R², where R¹ and R² may independently be alkyl, cycloalkyl, alkenyl, cycloalkenyl, alkynyl, cycloalkynyl, aryl or heteroaryl as described herein.

A term "azido" as used herein is represented by a formula -N₃.

A term "nitro" as used herein is represented by a formula -NO₂.

A term "nitrile" as used herein is represented by a formula -CN.

A term "silyl" as used herein is represented by a formula -SiR¹R²R³, where R¹, R² and R³ may independently be alkyl, cycloalkyl, alkoxy, alkenyl, cycloalkenyl, alkynyl, cycloalkynyl, aryl or heteroaryl as described herein.

A term "sulfo-oxo" as used herein is represented by a formula -S(O)R¹, -S(O)²R¹, -OS(O)²R¹ or -OS(O)²OR¹, where R1 may be alkyl, cycloalkyl, alkenyl, cycloalkenyl, alkynyl, cycloalkynyl, aryl or heteroaryl as described herein. Throughout this specification, "S(O)" is a short form of S=O. A term "sulfonyl" as used herein refers to a sulfo-oxo represented a formula -S(O)²R¹, where R¹ may be alkyl, cycloalkyl, alkenyl, cycloalkenyl, alkynyl, cycloalkynyl, aryl or heteroaryl. A term "sulfone" as used herein is represented by a formula R¹S(O)₂R², where R¹ and R² may independently be alkyl, cycloalkyl, alkenyl, cycloalkenyl, alkynyl, cycloalkynyl, aryl or heteroaryl as described herein. A term "sulfoxide" as used herein is represented by a formula R¹S(O)R², where R¹ and R² may independently be alkyl, cycloalkyl, alkenyl, cycloalkenyl, alkynyl, cycloalkynyl, aryl or heteroaryl as described herein.

A term "thiol" as used herein is represented by a formula -SH.

"R¹", "R²", "R³", "Rⁿ" (where n is an integer) as used herein may independently have one or more of groups listed above. For example, if R¹ is linear alkyl, one hydrogen atom of the alkyl may be optionally substituted with hydroxy, alkoxy, alkyl, halogen or the like. Depending on a selected group, a first group may be incorporated within a second group, or alternatively, the first group may be suspended, i.e., linked to the second group. For example, for a phrase "an alkyl including amino", the amino may be incorporated within a main chain of the alkyl. Alternatively, the amino may be linked to the main chain of the alkyl. Nature of the selected group may determine whether the first group is embedded in or linked to the second group.

The compound of that present disclosure may contain an "optionally substituted" moiety. In general, a term "substituted" (whether or not a term "optionally" exists before it) means that one or more hydrogen atoms of a given moiety are substituted with a suitable substituent. Unless otherwise stated, an "optionally substituted" group may have suitable substituents at each of substitutable positions of the group, and when more than one position in any given structure may be substituted with more than one substituent selected from groups designated, the substituents at each position may be the same or different. Combinations of substituents contemplated in the present disclosure are preferably those that form stable or chemically feasible compounds. It is also included that in certain aspects, respective substituents may be further optionally substituted (i. e., further substituted or unsubstituted) unless explicitly stated to the contrary.

A structure of the compound may be represented by a following formula: 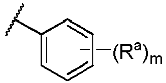 which is understood as equivalent to a following formula: 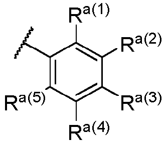 where n is typically an integer. That is, Rⁿ is understood to represent five individual substituents, namely, R^{a(1)}, R^{a(2)}, R^{a(3)}, R^{a(4)} and R^{a(5)}. "individual substituents" means that each R substituent may be defined independently. For example, if R^{a(m)} is halogen in one case, R^{a(n)} is not necessarily halogen in this case.

R¹, R², R³, R⁴, R⁵, R⁶ etc. are mentioned several times in chemical structures and moieties disclosed and described in this disclosure. Any description of R¹, R², R³, R⁴, R⁵, R⁶, etc. in the specification is applicable to any structure or moiety referring to R¹, R², R³, R⁴, R⁵, R⁶, etc. respectively, unless otherwise specified.

For various reasons, it is more and more urgent for optoelectronic devices to use organic materials. Many materials used to make such devices are relatively cheap, so organic photoelectric devices have potential of cost advantage over inorganic devices. In addition, inherent characteristics of organic materials, such as their flexibility, can make them very suitable for special applications such as manufacturing on flexible substrates. Examples of organic optoelectronic devices include organic light emitting devices (OLED), organic phototransistors, organic photovoltaic cells and organic photodetectors. For OLED, organic materials may have performance advantages over conventional materials. For example, a wavelength at which the organic light-emitting layer emits light can usually be tuned with an appropriate dopant.

If an exciton decays from a singlet excited state to a ground state to produce instantaneous luminescence, it is fluorescence. If the exciton decays from a triplet excited state to the ground state to produce luminescence, it is phosphorescence. Due to strong spin-orbit coupling of heavy metal atoms between singlet and triplet excited states, and intersystem crossing (ISC) is effectively enhanced, phosphorescent metal complexes (such as platinum complexes) have shown their potential to utilize singlet and triplet excitons at the same time and achieve 100% internal quantum efficiency. Therefore, the phosphorescent metal complexes are a good choice of dopants in am emission layer of an organic light-emitting device (OLED), and have attracted great attention in academic and industrial fields. In the past decade, many achievements have been made, which leads to profitable commercialization of this technology. For example, the OLED has been used in advanced displays of smart phones, televisions and digital cameras.

However, up to now, a blue electroluminescent device still lies in the most challenging field for this technology, and stability of blue devices is one of its big problems. It has been proved that selection of a main material is very important for the stability of the blue devices. However, minimum energy for a triplet excited state (T1) of a blue luminescent material is very high, which means that a minimum energy of a triplet excited state (T1) of the main material of the blue device should be higher. This makes it more difficult to develop the main material of the blue devices.

The metal complexes according to the present disclosure can be customized or tuned to specific applications where specific emission or absorption characteristics are desired. Optical properties of the metal complexes disclosed in this application can be adjusted by changing a structure of ligands around a metal center or changing a structure of fluorescent luminophores on the ligands. For example, in emission and absorption spectra, metal complexes of ligands with electron-donating substituents or electron-accepting substituents can usually show different optical properties. A luminescence color of the metal complex can be adjusted by modifying conjugated groups on the fluorescent luminophores and ligands.

Emission of the complex according to the present disclosure can be adjusted, for example, by changing a structure of the ligands or fluorescent luminophores, for example ranging from ultraviolet to near infrared. Fluorescent luminophores are a group of atoms in organic molecules, which can absorb energy to produce singlet excited states which decay rapidly to produce instant luminescence. On one hand, the complex according to the present disclosure can provide emission in most of visible spectrum. In specific examples, the complex according to the present disclosure can serve to emit light in a wavelength range of visible light or near infrared light. On the other hand, the complex according to the disclosure has improved stability and efficiency compared with traditional emission complexes. In addition, the complex according to the present disclosure can be used as a luminescent label for biological applications, anticancer agents, emitters in organic light emitting diodes (OLED) or combinations thereof. On the other hand, the complex according to the present disclosure can be used in light emitting devices, such as compact fluorescent lamps (CFL), light emitting diodes (LEDs), incandescent lamps and combinations thereof.

Disclosed herein is a compound or a composite complex containing platinum. A term compound or complex are used interchangeably in the present disclosure. In addition, the compound disclosed herein have neutral charges.

The compound disclosed herein can exhibit desirable properties and have emission and/or absorption spectra that can be adjusted by selecting appropriate ligands. On the other hand, any one or more compounds, structures or parts thereof specifically described herein can be excluded in the disclosure.

The compounds disclosed herein are suitable for various optical and electro-optical devices, including but not limited to light absorption devices such as solar energy and photosensitive devices, organic light emitting diodes (OLEDs), light emitting devices, or devices capable of both light absorption and emission, and used as markers for biological applications.

As described above, the disclosed compound is a platinum complex. Meanwhile, the compound disclosed herein can be used as a main material for OLED applications, such as full-color displays.

The compound disclosed herein can be used in various applications. As a luminescent material, the compound can be used in organic light-emitting diodes (OLED), light-emitting devices and displays, and other light-emitting devices.

In addition, compared with traditional materials, the compound in the disclosure can be used in light-emitting devices (such as OLEDs), which can improve luminous efficiency and operating time of the devices.

The compound according to the present disclosure can be prepared in various methods, including but not limited to those described in examples provided herein.

The compound disclosed herein may be a delayed fluorescent and/or phosphorescent emitter. In an aspect, the compound disclosed herein can be a delayed fluorescent emitter. In an aspect, the compound disclosed herein may be a phosphorescent emitter. In another aspect, the compound disclosed herein can be a delayed fluorescent emitter and a phosphorescent emitter.

The compound disclosed in an embodiment of the present disclosure is suitable for various optical and electro-optical devices, including but not limited to light-absorbing devices such as solar energy and light-sensitive devices, organic light-emitting diodes (OLEDs), light-emitting devices or devices with both light-absorbing and light-emitting capabilities, and can be used as markers for biological applications.

The compound according to the embodiment of the disclosure can be used in a light-emitting device such as an OLED, which includes at least one cathode, at least one anode and at least one light-emitting layer. At least one of the at least one light-emitting layers includes a tetradentate cyclometalated metal platinum complex based on phenyl carbazole. Specifically, the light emitting device may include an anode, a hole transport layer, a light-emitting layer, an electron transport layer, and a cathode which are sequentially deposited. The hole transport layer, the light-emitting layer and the electron transport layer are all organic layers, with which the anode and the cathode are electrically connected.

### Synthetic example

Following examples about compound synthesis, components, devices or methods are only to provide a general method for the industry, and are not intended to limit a protection scope of the disclosure. Data mentioned in the disclosure (number, temperature, etc.) are as accurate as possible, but there may be some errors. Unless otherwise specified, weighing is made separately, the temperature is in °C, or a normal temperature, and a pressure is close to a normal pressure.

A preparation method of a new compound is provided in following examples, but the preparation of such compounds is not limited thereto. In this technical field, because the claimed compound in this disclosure is easy to be modified and prepared, following methods or other methods can be adopted in its preparation. The following examples are only embodiments and are not intended to limit the protection scope of this disclosure. Temperature, catalyst, concentration, reactants and reaction processes can all be changed so as to select different conditions for different reactants for preparing the compound.

¹H NMR(500 MHz) and ¹³C NMR(126 MHz) spectra were measured on an ANANCE III (500 m) nuclear magnetic resonance spectrometer, which, unless otherwise specified, DMSO-*d₆* or CDCl₃ containing 0.1% TMS is used as a solvent, and when CDCl₃ is used as a solvent for a ¹H NMR spectrum, TMS (δ= 0.00 ppm) is used as an internal standard; and when DMSO-*d₆* is used as the solvent, TMS (δ = 0.00 ppm) or a residual DMSO peak (δ= 2.50 ppm) or a residual water peak (δ = 3.33 ppm) is used as the internal standard. In a ¹³C NMR spectrum, CDCl₃ (δ= 77.00 ppm) or DMSO-*d₆* (δ = 39.52 ppm) was used as the internal standard. It is measured on a HPLC-MS Agilent 6210 TOF LC/MS mass spectrometer; and a HRMS spectrum is measured on an Agilent 6210 TOF LC/MS liquid chromatography-time-of-flight mass spectrometer. In ¹H NMR spectrogram data, s means singlet, d means doublet, t means triplet, q means quart, p means quintet, m means multiplet, and br means broad.

### Synthetic route

### A general synthesis method is as follows:

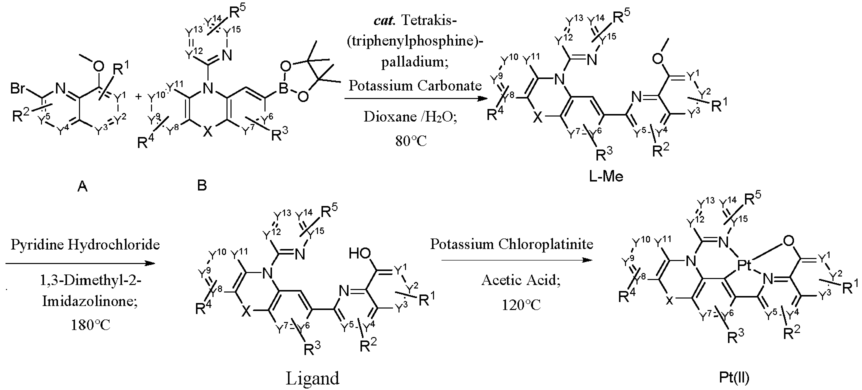

### Example 1: Platinum complex Pt1 can be synthesized as follows:

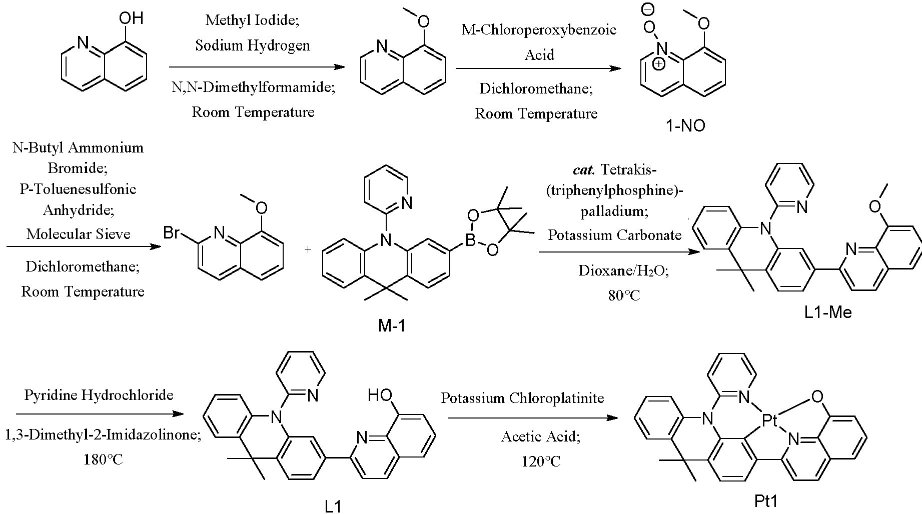

**Synthesis of intermecdiate 8- methoxyquinoline:** 8- hydroxyquinoline (5.00 g, 34.5 mmol, 1.00 equivalent) and N,N- dimethylformamide (40 ml) were added into a 100 ml three-necked flask with a magnetic stir bar, which was then put in a low-temperature bath and stirred; after the temperature was lowered, 60% by weight of sodium hydrogen (1.38 g, 34.5 mmol, 1.00 equivalent) was slowly added followed by standing at a room temperature, and then methyl iodide (5.14 g, 36.3 mmol, 1.05 equivalent) was added for reaction for 8 h. A reaction mixture was quenched by adding ethanol, and then the solvent was removed by vacuum distillation. After extracting with ethyl acetate for three times, organic phases were combined, dried with anhydrous sodium sulfate added, and a resultant crude product was separated and purified by a silica gel column chromatography, with an eluent of petroleum ether/acetone of 2: 1, and the solvent was removed by vacuum distillation, so as to obtain 4.76 g of black-red solid with a yield of 87%. ¹H NMR (400 MHz, CDCl₃): *δ* 4.10 (s, 3H), 7.06 (d, *J =* 8.0 Hz, 1H), 7.38-7.49 (m, 3H), 8.13 (d, *J =* 8.0 Hz, 1H), 8.94 (dd, *J =* 3.6Hz, 0.8 Hz, 1H).

**Synthesis of Intermediate 1-NO:** 8- methoxyquinoline (5.4 g, 34.2 mmol, 1.00 equivalent) and dichloromethane (170 ml) were added into a 500 ml three-necked flask with a magnetic stir bar, which is fixed in a low-temperature reaction bath followed by lowering of temperature, then m-chloroperoxybenzoic acid (8.84 g, 51.3 mmol, 1.50 equivalent) was slowly added, the three-necked flask was taken out for reaction at the room temperature for 24 hours. A reaction mixture was quenched with sodium thiosulfate added, diluted with water, extracted with dichloromethane for 4 to 5 times, and the solvent was removed by vacuum distillation, so as to obtain 4.15 g of crude black-red solid, with a yield of 69%. The solid was directly used in a next step.

**Synthesis of Intermediate 2- bromo -8- methoxyquinoline:** Intermediate 1-NO (6.90 g, 39.4 mmol, 1.00 equivalent), n-butyl ammonium bromide (19.05 g, 59 mmol, 1.50 equivalent) and molecular sieves were added to a 1000 ml three-necked flask with a magnetic stir bar, and dichloromethane (700 ml) was added, and stirred at the room temperature for 10 minutes, and p-toluenesulfonic anhydride (19.28 g, 59 mmol, 1.50 equivalent) was added and reacted at the room temperature for 24 h. A reaction mixture was filtered, and residue was washed with dichloromethane, a resultant crude product was separated and purified by silica gel column chromatography, with an eluent of petroleum ether/ethyl acetate of 10:1, and the solvent was removed by vacuum distillation, so as to obtain 4.3 g of white solid with a yield of 46%. ¹H NMR (400 MHz, CDCl₃): *δ* 4.06 (s, 3H), 7.08 (d, *J* = 8.0 Hz, 1H), 7.37 (d, *J* = 8.0 Hz, 1H),7.47-7.51 (m, 1H), 7.55 (d, *J =* 8.8 Hz, 1H), 7.96 (d, *J =* 8.8 Hz, 1H).

**Synthesis of intermediate L1-Me:** 2- bromo -8- methoxyquinoline (277 mg, 1.16 mmol, 1.10 equivalent), M-1 (420 mg, 1.06 mmol, 1.00 equivalent), Pd(PPh₃)₄ (35 mg, 0.03 mmol, 0.03 equivalent), and K₂CO₃ (365 mg, 2.65 mmol, 2.50 equivalent) were added in turn into a 50 ml three-necked flask with a magnetic stir bar, nitrogen was pumped and exchanged for three times, then dioxane /H₂O of 6:1 ml was injected and added, and heated up to 85 °C for reaction for 24 hours. A reaction mixture was quenched with water, diluted with ethyl acetate added, extracted with ethyl acetate for three times, dried with anhydrous sodium sulfate added, and a resultant crude product was separated and purified by a silica gel column chromatography, with an eluent of petroleum ether/ethyl acetate of 8: 1, and the solvent was removed by vacuum distillation, so as to obtain 348 mg of white foam solid with a yield of 75%. ¹H NMR (400 MHz, CDCl₃): *δ* 1.69 (s, 6H), 4.06 (s, 3H), 6.87 (dd, *J =* 8.0 Hz, 1.2 Hz, 1H), 7.02-7.12 (m, 3H), 7.28 (d, *J =* 0.8 Hz, 1H), 7.36-7.42 (m, 3H),7.48 (dd, *J =* 7.6 Hz, 1.2 Hz, 1H), 7.57-7.59 (m, 2H), 7.71 (d, *J =* 8.4 Hz, 1H), 7.82-7.88 (m, 2H), 8.10 (d, *J =* 8.4 Hz, 1H), 8.94 (ddd, *J =* 4.8 Hz, 2.0 Hz, 0.8 Hz 1H).

**Synthesis of intermediate L1:** L1-Me (280 mg, 0.63 mmol, 1.00 equivalent) and pyridine hydrochloride (912 mg, 6.3 mmol, 10.00 equivalent) were added in turn into a 50 ml three-necked flask with a magnetic stir bar, nitrogen was pumped and exchanged for three times, 3 ml of 1,3- dimethyl -2- imidazolinone was injected and added, and the temperature was raised to 180 °C, for reaction for 24 hours. A reaction mixture was quenched with 100 ml of water, extracted with ethyl acetate for three times, then organic phases were combined, dried with anhydrous sodium sulfate added, and the solvent was removed by vacuum distillation. A resultant crude product was separated and purified by silica gel column chromatography, with an eluent of petroleum ether/ethyl acetate of 15:1, and the solvent was removed by vacuum distillation, so as to obtain 175 mg of white foam solid, with a yield of 65%. ¹H NMR (400 MHz, DMSO-*d*₆): *δ* 1.63 (s, 6H), 6.75 (dd, *J =* 8.0 Hz, 1.2 Hz, 1H), 7.03-7.14 (m, 3H), 7.36-7.47 (m, 4H), 7.54 (dd, *J =* 7.6 Hz, 1.6 Hz, 1H), 7.61 (d, *J =* 2.0 Hz, 1H), 7.66 (d, *J =* 8.4 Hz, 1H), 7.84 (d, *J =* 8.8 Hz, 1H), 8.01 (td, *J =* 7.6 Hz, 2.0 Hz, 1H), 8.08 (dd, *J =* 8.0 Hz, 1.6 Hz, 1H), 8.32 (d, *J =* 8.4 Hz, 1H), 8.94 (dt, *J =* 4.8 Hz, 1.6 Hz, 1H), 9.40 (s, 1H).

**Synthesis of Pt1:** L1 (140 mg, 0.33 mmol, 1.00 equivalent) and potassium chloroplatinite (143 mg, 0.35 mmol, 1.05 equivalent) were added into a 50 ml three-necked flask with a magnetic stir bar, nitrogen was pumped and exchanged for three times, and acetic acid was injected and added. Nitrogen bubbling was made for 30 minutes, stirring was made at the room temperature for 8 hours, followed by heating up to 120 °C, for reaction for 24 hours. 30 ml of acetic acid was removed by vacuum distillation from a reaction mixture, and then the reaction mixture was separated and purified by silica gel column chromatography, with an eluent of dichloromethane/ethyl acetate of 15:1, and the solvent was removed by vacuum distillation so as to obtain 170 mg of deep red solid with a yield of 84%. ¹H NMR (400 MHz, DMSO-*d*₆): *δ* 1.34 (s, 3H), 1.84 (s, 3H), 5.76 (s, 1H), 6.52 (d, *J =* 8.0 Hz, 1H), 6.74 (d, *J =* 8.0 Hz, 1H), 7.07 (d, *J =* 8.0 Hz, 1H), 7.21-7.25 (m, 4H), 7.38 (d, *J* = 8.0 Hz, 1H), 7.53-7.56 (m, 2H), 7.78 (d, *J =* 8.8 Hz, 1H), 8.00-8.05 (m, 1H), 8.17 (d, *J* = 8.8 Hz, 1H), 8.88 (dd, *J =* 6.0 Hz, 2.0 Hz, 1H).

### Example 2: Platinum complex Pt2 can be synthesized as follows:

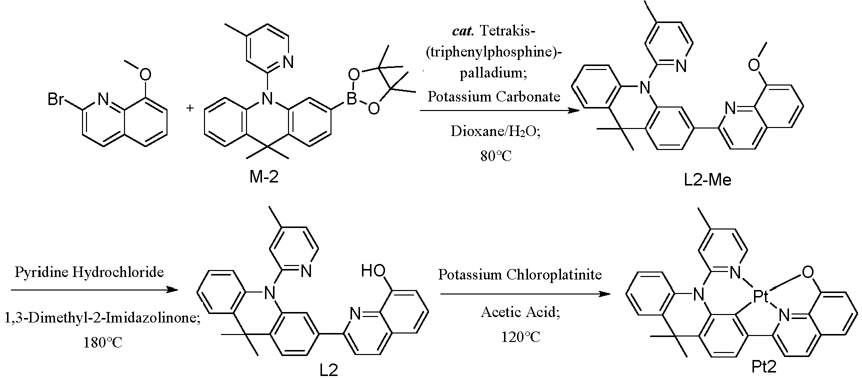

**Synthesis of L2-Me:** 2- bromo -8- methoxyquinoline (234 mg, 0.99 mmol, 1.05 equivalent), M-1 (400 mg, 0.94 mmol, 1.00 equivalent), Pd(PPh₃)₄ (32.5 mg, 0.03 mmol, 0.03 equivalent), and K₂CO₃ (324 mg, 2.34 mmol, 2.50 equivalent) were added in turn into a 50 ml three-necked flask with a magnetic stir bar, nitrogen was pumped and exchanged for three times, then dioxane /H₂O of 6:1 ml was injected and added, and heated up to 85 °C for reaction for 24 hours. A reaction mixture was quenched with water, diluted with ethyl acetate added, extracted with ethyl acetate for three times, dried with anhydrous sodium sulfate added, and a resultant crude product was separated and purified by a silica gel column chromatography, with an eluent of petroleum ether/ethyl acetate of 6: 1, and the solvent was removed by vacuum distillation, so as to obtain 333 mg of white foam solid with a yield of 78%. ¹H NMR (500 MHz, CDCl₃): *δ* 1.70 (s, 6H), 2.41 (s, 3H), 4.05 (s, 3H), 6.69 (dd, *J =* 8.0 Hz, 1.5Hz 1H), 7.00-7.08 (m, 3H), 7.14-7.15 (m, 1H), 7.22(t, *J* = 0.5 Hz, 1H), 7.34-7.43 (m, 3H), 7.47 (dd, *J* = 7.5 Hz, 1.5 Hz 1H), 7.58 (d, *J =* 8.0 Hz, 1H), 7.67 (d, *J =* 8.5 Hz, 1H), 7.82 (dd, *J =* 8.0 Hz, 1.5 Hz 1H), 8.09 (d, *J =* 8.5 Hz, 1H), 8.61 (d, *J =* 5.0 Hz, 1H).

**Synthesis of intermediate L2:** L2-Me (320 mg, 0.7 mmol, 1.00 equivalent) and pyridine hydrochloride (808 mg, 7 mmol, 10.00 equivalent) were added in turn into a 50 ml three-necked flask with a magnetic stir bar, nitrogen was pumped and exchanged for three times, 3 ml of 1,3- dimethyl -2- imidazolinone was injected and added, and the temperature was raised to 180 oC, for reaction for 24 hours. A reaction mixture was quenched with 100 ml of water, extracted with ethyl acetate for three times, then organic phases were combined, dried with anhydrous sodium sulfate added, and the solvent was removed by vacuum distillation. A resultant crude product was separated and purified by silica gel column chromatography, with an eluent of petroleum ether/ethyl acetate of 15:1, and the solvent was removed by vacuum distillation, so as to obtain 224 mg of white foam solid with a yield of 72%. ¹H NMR (500 MHz, CDCl₃): *δ* 1.73 (s, 6H), 2.44 (s, 3H), 6.63 (dd, *J* = 9.0 Hz, 1.5Hz 1H), 7.00-7.08 (m, 2H), 7.14 (dd, *J =* 7.5 Hz, 1.0Hz 1H), 7.21-7.22 (m, 1H), 7.24 (d, *J =* 0.5 Hz, 1H), 7.29 (dd, *J =* 8.0 Hz, 1.0 Hz 1H), 7.39 (t, *J =* 8.0 Hz, 1H), 7.47-7.49 (m, 2H), 7.69 (d, *J =* 8.0 Hz, 1H), 7.71 (dd, *J =* 8.5 Hz, 2.0 Hz 1H), 7.76 (d, *J =* 9.0 Hz, 1H), 8.61 (d, *J=* 5.0 Hz, 1H), 8.66 (d, *J =* 5.0 Hz, 1H).

**Synthesis of Pt2:** L2 (220 mg, 0.49 mmol, 1.00 equivalent) and potassium chloroplatinite (216 mg, 0.52 mmol, 1.05 equivalent) were added into a 50 ml three-necked flask with a magnetic stir bar, nitrogen was pumped and exchanged for three times, and 30 ml of acetic acid was injected and added. Nitrogen bubbling was made for 30 minutes, stirring was made at the room temperature for 8 hours, followed by heating up to 120 °C, for reaction for 24 hours. Acetic acid was removed by vacuum distillation from a reaction mixture, and then the reaction mixture was separated and purified by silica gel column chromatography, with an eluent of dichloromethane/ethyl acetate of 20:1, and the solvent was removed by vacuum distillation so as to obtain 170 mg of light red solid with a yield of 94%. ¹H NMR (500 MHz, DMSO-*d*₆): *δ* 1.35 (s, 3H), 1.84 (s, 3H), 2.24(s, 3H), 6.53 (dd, *J =* 7.5 Hz, 0.5Hz 1H), 6.75 (d, *J =* 8.0 Hz, 1H), 7.07 (d, *J =* 7.5 Hz, 1H), 7.10 (dd, *J =* 6.5 Hz, 1.5Hz 1H), 7.20-7.25 (m, 4H), 7.37 (t, *J =* 4.5 Hz, 2H), 7.52-7.54 (m, 1H), 7.76 (d, *J* = 8.5 Hz, 1H), 8.17 (d, *J =* 9.0 Hz, 1H), 8.73 (d, *J =* 6.5 Hz 1H).

### Example 3: Platinum complex Pt3 can be synthesized as follows:

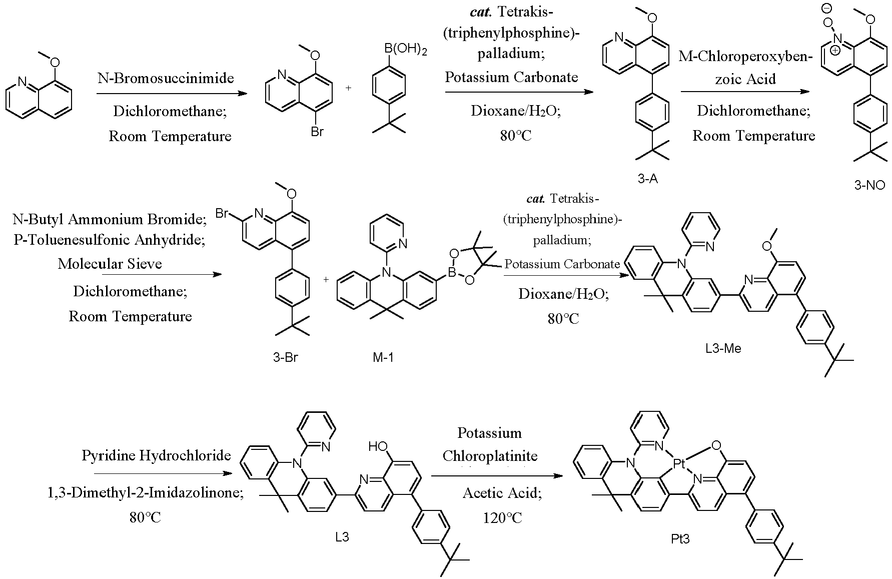

**Synthesis of 5- bromo -8- methoxyquinoline:** 8- methoxyquinoline (4.76 g, 29.9 mmol, 1.00 equivalent) and acetonitrile (230 ml) were added into a 500 ml three-necked flask with a magnetic stir bar, and then N-bromosuccinimide (5.32 g, 31.3 mmol, 1.05 equivalent) was slowly added for reaction for 8 hours. A reaction mixture was quenched by adding a sodium sulfite aqueous solution, diluted with dichloromethane added, extracted with dichloromethane for three times, organic phases were combined , and dried with anhydrous sodium sulfate added. A crude product was separated and purified by silica gel column chromatography, with an eluent of dichloromethane/ethyl acetate of 10:1, and the solvent was removed by vacuum distillation so as to obtain 5.8 g of light yellow solid with a yield of 82%. The solid was directly used in reaction in a next step.

**Synthesis of 3-A:** 5- bromo -8- methoxyquinoline (500 mg, 2.1 mmol, 1.00 equivalent), 4-tert-Butylphenylboronic acid (374 mg, 2.1 mmol, 1.00 equivalent), Pd(PPh₃)₄ (73 mg, 0.06 mmol, 0.03 equivalent), and K₂CO₃ (726 mg, 5.25 mmol, 2.5 equivalent) were added in turn into a reaction tube with a magnetic stir bar, nitrogen was pumped and exchanged for three times, then dioxane /H₂O of 8:2 ml was injected and added, and heated up to 85 °C for reaction for 24 hours. A reaction mixture was diluted with ethyl acetate, extracted with ethyl acetate for three times, organic phases were combined, dried with anhydrous sodium sulfate, a crude product was separated and purified by silica gel column chromatography, with an eluent of petroleum ether/ethyl acetate of 3:2 , and the solvent was removed by vacuum distillation, so as to obtain 580 mg of white solid with a yield of 94%. ¹H NMR (400 MHz, CDCl₃): *δ* 1.40 (s, 9H), 4.14 (s, 3H), 7.11 (d, *J =* 8.0 Hz 1H), 7.37-7.40 (m, 3H), 7.44 (d, *J =* 8.0 Hz, 1H), 7.49-7.52 (m, 2H), 8.28 (dd, *J =* 8.4, 1.6 Hz 1H), 8.94 (dd, *J =* 8.4 Hz, 2.0 Hz 1H).

**Synthesis of Intermediate 3-NO:** 3-A (5.00 g, 17.2 mmol, 1.00 equivalent) and dichloromethane (100 ml) were added into a 500 ml three-necked flask with a magnetic stir bar, which is fixed in a low-temperature reaction bath followed by lowering of temperature, then m-chloroperoxybenzoic acid (5.9 g, 34.4 mmol, 2.00 equivalent) was slowly added, the three-necked flask was taken out for reaction at the room temperature for 24 hours. A reaction mixture was quenched with sodium thiosulfate added, diluted with water, extracted with dichloromethane for 4 to 5 times, and the solvent was removed by vacuum distillation, a crude product was separated and purified with silica gel column chromatography to obtain 2.1 g of dark red solid, and the solvent was removed by vacuum distillation, with a yield of 40%. The solid was directly used in a next step.

**Synthesis of Intermediate 3-Br:** Intermediate 3-NO (2.1 g, 7.2 mmol, 1.00 equivalent), n-butyl ammonium bromide (3.48 g, 10.81 mmol, 1.50 equivalent) and molecular sieves were added to a 500 ml three-necked flask with a magnetic stir bar, and dichloromethane (180 ml) was added, and stirred at the room temperature for 10 minutes, and p-toluenesulfonic anhydride (3.53 g, 10.81 mmol, 1.50 equivalent) was added and stirred at the room temperature overnight for reaction for 24 h. A reaction mixture was filtered, and residue was washed with dichloromethane, the solvent was removed by vacuum distillation, and a resultant crude product was separated and purified by silica gel column chromatography, with an eluent of petroleum ether/ethyl acetate of 20:1, and the solvent was removed by vacuum distillation, so as to obtain 800 mg of light yellow solid with a yield of 30%. ¹H NMR (400 MHz, CDCl₃): *δ* 1.40 (s, 9H), 4.10 (s, 3H), 7.13 (d, *J =* 8.0 Hz 1H), 7.33-7.35 (m, 2H), 7.44-7.52 (m, 4H), 8.10 (d, *J =* 8.8 Hz 1H).

**Synthesis of Intermediate L3-Me:** 3-Br (150 mg, 0.4 mmol, 1.00 equivalent), M-1 (175 mg, 0.43 mmol, 1.05 equivalent), Pd(PPh₃)₄ (15 mg, 0.01 mmol, 0.03 equivalent), and K₂CO₃ (140 mg, 1.01 mmol, 2.50 equivalent) were added in turn into a reaction tube with a magnetic stir bar, nitrogen was pumped and exchanged for three times, then dioxane /H₂O of 6:1 ml was injected and added, and heated up to 85 °C for reaction for 24 hours. A reaction mixture was quenched with water, diluted with ethyl acetate added, extracted with ethyl acetate for three times, dried with anhydrous sodium sulfate added, and a resultant crude product was separated and purified by a silica gel column chromatography, with an eluent of petroleum ether/ethyl acetate of 5: 1, and the solvent was removed by vacuum distillation, so as to obtain 170 mg of white foam solid with a yield of 81%. ¹H NMR (500 MHz, CDCl₃): *δ* 1.40 (s, 9H), 1.69 (s, 6H), 4.09 (s, 3H), 6.83 (dd, *J =* 8.0, 1.5Hz, 1H), 7.02-7.10 (m, 3H), 7.25-7.28 (m, 1H), 7.36-7.40 (m, 4H), 7.47-7.51 (m, 3H), 7.54 (d, *J =* 2.0 Hz, 1H), 7.59 (t, *J =* 8.0 Hz, 1H), 7.63 (d, *J =* 9.0 Hz, 1H), 7.84 (ddd, *J =* 8.0, 7.5, 2.0Hz, 1H), 7.88 (dd, *J* = 8.0, 1.5 Hz, 1H), 8.23 (d, *J =* 9.0 Hz, 1H), 8.70 (ddd, *J =* 5.0, 2.0, 1.0Hz, 1H).

**Synthesis of intermediate L3:** L3-Me (170 mg, 0.30 mmol, 1.00 equivalent) and pyridine hydrochloride (341 mg, 3 mmol, 10.00 equivalent) were added in turn into a reaction tube with a magnetic stir bar, nitrogen was pumped and exchanged for three times, 3 ml of 1,3- dimethyl -2- imidazolinone was injected and added, and the temperature was raised to 180°C for reaction for 24 hours. A reaction mixture was quenched with 100 ml of water, extracted with ethyl acetate for three times, then organic phases were combined, dried with anhydrous sodium sulfate added, and the solvent was removed by vacuum distillation. A resultant crude product was separated and purified by silica gel column chromatography, with an eluent of petroleum ether/ethyl acetate of 30:1, and the solvent was removed by vacuum distillation, so as to obtain 80 mg of light yellow foam solid, with a yield of 47%. ¹H NMR (500 MHz, CDCl₃): *δ* 1.40 (s, 9H), 1.72 (s, 6H), 6.75 (dd, *J =* 8.0 Hz, 1.5Hz, 1H), 7.04(td, *J =* 7.5, 1.5 Hz, 1H), 7.09(td, *J =* 7.5, 2.0 Hz, 1H), 7.34 (ddd, *J =* 7.5, 5.0, 1.0Hz, 1H), 7.36-7.40 (m, 3H), 7.41(dt, *J =* 9.0, 1.0 Hz, 1H), 7.49-7.51 (m, 3H), 7.60 (d, *J =* 4.5 Hz, 1H), 7.61 (d, *J* = 9.0 Hz, 1H), 7.71 (d, *J =* 9.0 Hz, 1H), 7.76 (dd, *J =* 8.5, 2.0 Hz, 1H), 7.89 (ddd, *J =* 7.5, 5.5, 2.0Hz, 1H), 8.30 (d, *J =* 9.0 Hz, 1H), 8.77 (ddd, *J =* 7.0, 2.0, 0.5 Hz, 1H).

**Synthesis of Pt3:** L3 (80 mg, 0.14 mmol, 1.00 equivalent) and potassium chloroplatinite (61 mg, 0.14 mmol, 1.05 equivalent) were added into a 50 ml three-necked flask with a magnetic stir bar, nitrogen was pumped and exchanged for three times, and 20 ml of acetic acid was injected and added. Nitrogen bubbling was made for 30 minutes, stirring was made at the room temperature for 8 h, followed by heating up to 120 °C, for reaction for 24 hours. Acetic acid was removed by vacuum distillation from a reaction mixture, and then the reaction mixture was separated and purified by silica gel column chromatography, with an eluent of dichloromethane/ethyl acetate of 30:1, and the solvent was removed by vacuum distillation so as to obtain 95 mg of black-red solid with a yield of 90%. ¹H NMR (500 MHz, DMSO-*d*₆): *δ* 1.34 (s, 9H), 1.34 (s, 3H), 1.84 (s, 3H), 6.01 (d, *J =* 8.0 Hz, 1H), 7.05-7.13 (m, 2H), 7.21-7.26 (m, 4H), 7.35 (d, *J =* 8.0 Hz, 1H), 7.38-7.39 (m, 2H), 7.50-7.52 (m, 2H), 7.53-7.57 (m, 2H), 7.75 (d, *J =* 9.0 Hz, 1H), 8.02-8.05 (m, 1H), 8.13 (d, *J =* 9.0 Hz, 1H), 8.90 (dd, *J =* 6.0, 1.5 Hz, 1H).

### Example 4: Platinum complex Pt4 can be synthesized as follows:

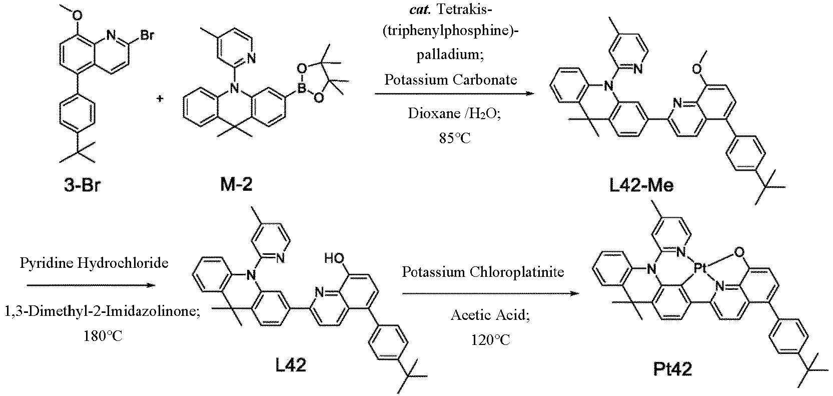

**Synthesis of Intermediate L42-Me:** 3-Br (130 mg, 0.35 mmol, 1.00 equivalent), M-2 (149 mg, 0.35 mmol, 1.00 equivalent), Pd(PPh₃)₄ (12.2 mg, 0.01 mmol, 0.03 equivalent), and K₂CO₃ (96.7 mg, 0.7 mmol, 2.0 equivalent) were added in turn into a reaction tube with a magnetic stir bar, nitrogen was pumped and exchanged for three times, then dioxane /H₂O of 5:1 ml was injected and added, and heated up to 85 °C for reaction for 24 hours. A reaction mixture was quenched with water, diluted with ethyl acetate added, extracted with ethyl acetate for three times, dried with anhydrous sodium sulfate added, and a resultant crude product was separated and purified by a silica gel column chromatography, with an eluent of petroleum ether/ethyl acetate of 10: 1, and the solvent was removed by vacuum distillation, so as to obtain 190 mg of white foam solid with a yield of 91%. ¹H NMR (500 MHz, CDCl₃): *δ* 1.40 (s, 9H), 1.70 (s, 6H), 2.40 (s, 3H), 4.09 (s, 3H), 6.67-6.72 (m, 1H), 7.02 (t, *J =* 6.0 Hz, 1H), 7.05-7.09 (m, 2H), 7.13 (d, *J*=5.0Hz, 1H), 7.22 (s, 1H), 7.36-7.40 (m, 4H), 7.47-7.51(m, 3H), 7.59(d, *J*=8.0Hz, 1H), 7.62(d, *J*=9.0Hz, 1H), 7.87(dd, *J* = 8.0, 1.0Hz, 1H), 8.24(d, *J*=9.0Hz, 1H), 8.61(d, *J*=5.0Hz, 1H).

**Synthesis of Intermediate L42:** L3-Me (180 mg, 0.30 mmol, 1.00 equivalent) and pyridine hydrochloride (352 mg, 3 mmol, 10.00 equivalent) were added in turn into a reaction tube with a magnetic stir bar, nitrogen was pumped and exchanged for three times, 3 ml of 1,3- dimethyl -2- imidazolinone was injected and added, and the temperature was raised to 180°C for reaction for 24 hours. A reaction mixture was quenched with saturated sodium bicarbonate solution, extracted with ethyl acetate for three times, then organic phases were combined, dried with anhydrous sodium sulfate added, and the solvent was removed by vacuum distillation. A resultant crude product was separated and purified by silica gel column chromatography, with an eluent of petroleum ether/ethyl acetate of 30:1, and the solvent was removed by vacuum distillation, so as to obtain 153 mg of light yellow solid, with a yield of 87%. ¹H NMR (500 MHz, CDCl₃): *δ* 1.40 (s, 9H), 1.73 (s, 6H), 2.44 (s, 3H), 6.66(d, *J =* 7.5 Hz, 1H), 7.02-7.10(m, 2H), 7.21-7.22(m, 2H), 7.24-7.27(m, 2H), 7.37-7.39(m, 3H), 7.48-7.51(m, 4H), 7.61(d, *J =* 8.0 Hz, 1H), 7.71-7.75(m, 2H), 8.33(d, *J =* 8.5 Hz, 1H), 8.66(d, *J =* 5.0 Hz, 1H).

### Photophysical test and theoretical calculation

Steady-state emission experiments and life measurement were carried out on a Horiba Jobin Yvon FluoroLog-3 spectrometer. The theoretical calculation of the Pt(II) complex was carried out using Titan software package. A geometric structure of molecules in a ground state (S₀) was optimized using density functional theory (DFT) . DFT calculation was performed by using B3LYP functional. 6-31G(d) basic sets were used for C, H, O and N atoms, while LANL2DZ basic sets were used for Pt atoms.

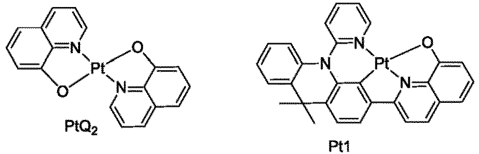

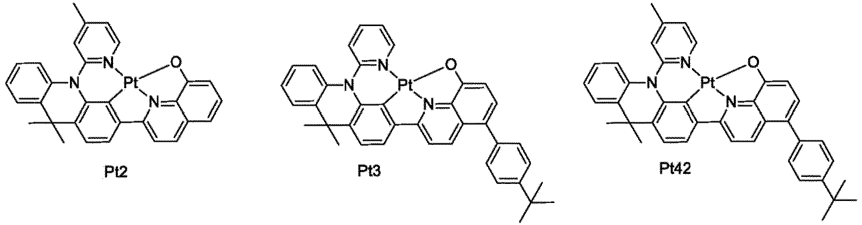

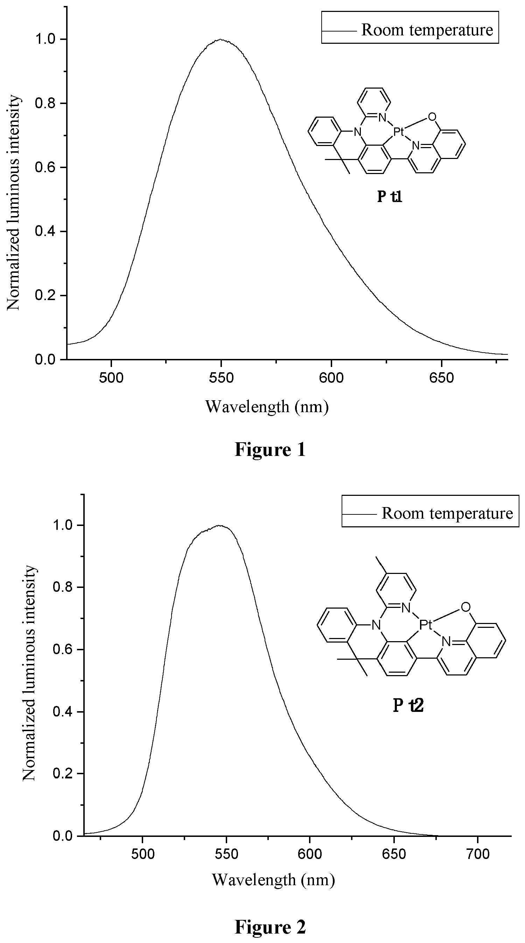

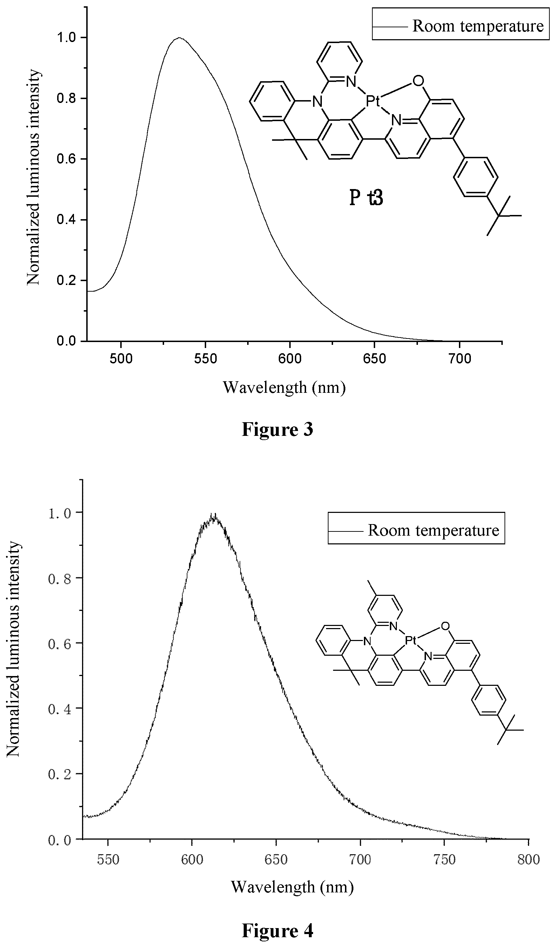

It can be seen from FIG.1 to FIG. 3 that platinum complexes Pt1, Pt2, Pt3 and Pt42 can strongly emit light in the dichloromethane solution, with maximum emission wavelengths of 549 nm, 547 nm and 534 nm, respectively, and solution quantum efficiencies all being greater than 40%. While solution quantum efficiency of a platinum complex PtQ2 based on bidentate ligands is only about 1.5% (I. Taydakov, K. Lyssenko, R. Saifutyarov, A. Akkuzina, R. Avetisov, A. Mozhevitina, I. Avetissov. Dyes and Pigments, 2016, 135, 80-85). It can be seen that the quantum efficiency of platinum complexes based on tetradentate ligands in this disclosure can be greatly improved compared with platinum complexes with bidentate ligands.

Table 1 shows the frontier orbital distribution energy level table of some platinum complexes obtained from the theoretical calculation experiment.

**Table 1: Frontier orbital energy levels of some metal complexes**

| Metal Complexes | HOMO / eV | LUMO / eV | Gap / eV |
|---|---|---|---|
| Pt1 | -4.5 | -1.79 | 2.71 |
| Pt2 | -4.46 | -1.73 | 2.73 |
| Pt3 | -4.44 | -1.8 | 2.64 |
| Pt4 | -4.49 | -1.81 | 2.68 |
| Pt5 | -4.43 | -1.77 | 2.66 |
| Pt6 | -4.88 | -2.02 | 2.41 |
| Pt7 | -4.49 | -1.84 | 2.65 |
| Pt8 | -4.46 | -1.97 | 2.49 |
| Pt9 | -4.63 | -2.11 | 2.52 |
| Pt10 | -4.66 | -2.17 | 2.49 |
| Pt11 | -4.7 | -2.12 | 2.58 |
| Pt12 | -4.7 | -1.95 | 2.75 |
| Pt13 | -4.37 | -1.67 | 2.7 |
| Pt14 | -4.46 | -1.8 | 2.66 |
| Pt15 | -4.39 | -1.79 | 2.6 |
| Pt16 | -4.42 | -1.74 | 2.68 |
| Pt17 | -4.33 | -1.83 | 2.5 |
| Pt18 | -4.03 | -1.68 | 2.35 |
| Pt19 | -4.34 | -1.89 | 2.45 |
| Pt20 | -3.42 | -1.71 | 1.71 |
| Pt21 | -4.57 | -1.79 | 2.78 |
| Pt22 | -4.61 | -1.89 | 2.72 |
| Pt23 | -4.45 | -1.63 | 2.82 |
| Pt24 | -4.41 | -1.59 | 2.82 |
| Pt397 | -4.57 | -1.83 | 2.74 |
| Pt398 | -4.41 | -1.74 | 2.67 |
| Pt399 | -4.26 | -1.8 | 2.46 |
| Pd1 | -4.49 | -1.69 | 2.8 |
| Pd2 | -4.45 | -4.63 | 2.82 |
| Pd3 | -4.36 | -1.59 | 2.77 |
| Pd4 | -4.43 | -1.72 | 2.71 |
| Pd5 | -4.16 | -1.55 | 2.61 |

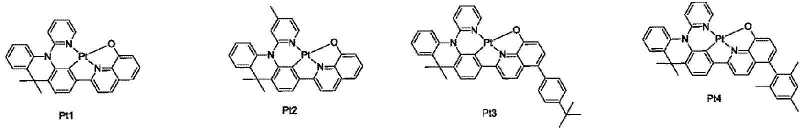

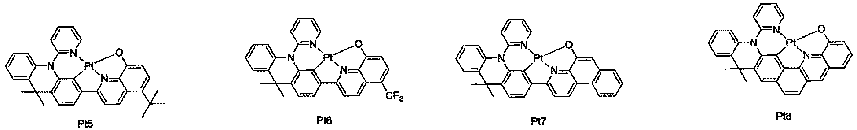

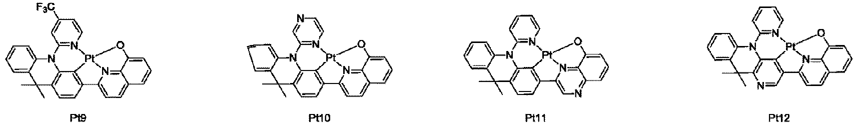

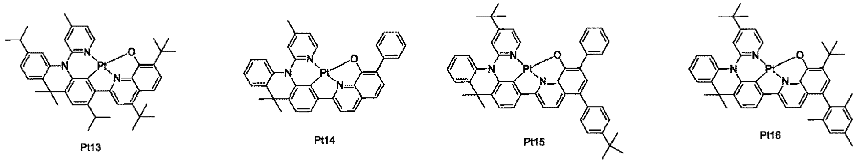

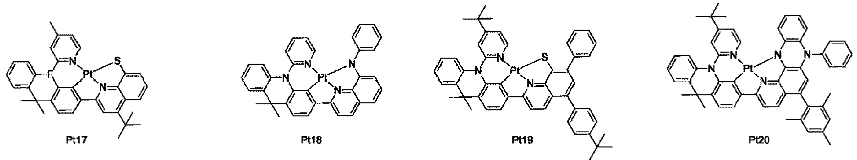

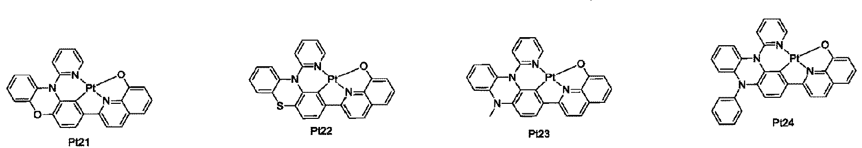

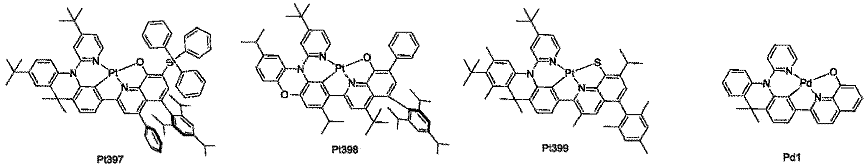

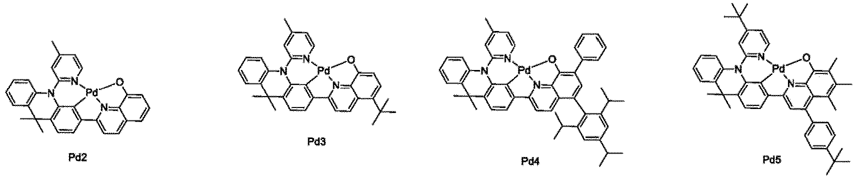

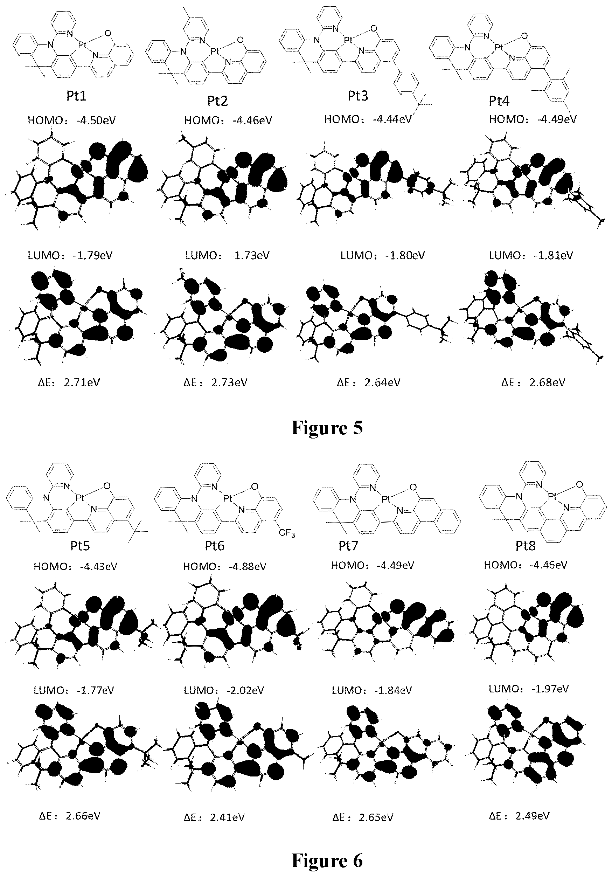

As can be seen from FIG. 5, for Pt2, compared with Pt1, with methyl group being introduced into pyridine, there is little influence on a highest occupied molecular orbital (HOMO) and a lowest unoccupied molecular orbital (LUMO), with small change in ΔE and close maximum emission wavelength, which can increase molecular stability. For Pt3, compared with Pt1, with p-tert-butylphenyl being introduced into quinoline, HOMO distribution delocalization is obvious, but with little influence on LUMO distribution, ΔE decreases, but the spectrum blue shifts, which may be due to increase of a triplet energy level, which results in blue shift of its molecular emission spectrum. Meanwhile, introduction of p-tert-butylbenzene can effectively suppress π -π stacking between molecules, which facilitates sublimation and purification. For Pt4, compared with Pt1, introduction of trimethylphenyl into quinoline has little influence on HOMO and LUMO, and ΔE changes little, and thus the spectrum is expected to change little. It may be because its dihedral angle is large, which breaks its conjugated system.

As can be seen from FIG. 6, for Pt5, compared with Pt1, with a tert-butyl group being introduced into quinoline and an electron-donating group being introduced, but it has little influence on LUMO, and therefore, it can be seen that introduction of an electron-accepting group into quinoline has little influence on distribution of molecular electron cloud, and π -π stacking between molecules can be suppressed by introducing the electron-donating group with large steric hindrance in this region. For Pt6, compared with Pt1, LUMO is significantly reduced, because its LUMO is mainly distributed in quinoline, and the LUMO distribution can be significantly reduced by introducing the electron-accepting group, trifluoromethyl, into quinoline, and thus its LUMO can be effectively regulated by introducing an electron-accepting group at this position, and thus photophysical properties of the molecule can be regulated. For Pt7, compared with Pt1, with phenyl being introduced to right of quinoline, which enlarges the conjugated system, it can be clearly seen that part of its HOMO is dispersed on phenyl, reducing ΔE, which is expected to red shift the emission spectrum. For Pt8, compared with Pt1, an acridine group is linked to quinoline group through a phenyl group, so that HOMO is mainly distributed in a quinoline part, LUMO delocalizes to a phenylquinoline part, which reduces △E and is expected to red shift the emission spectrum.

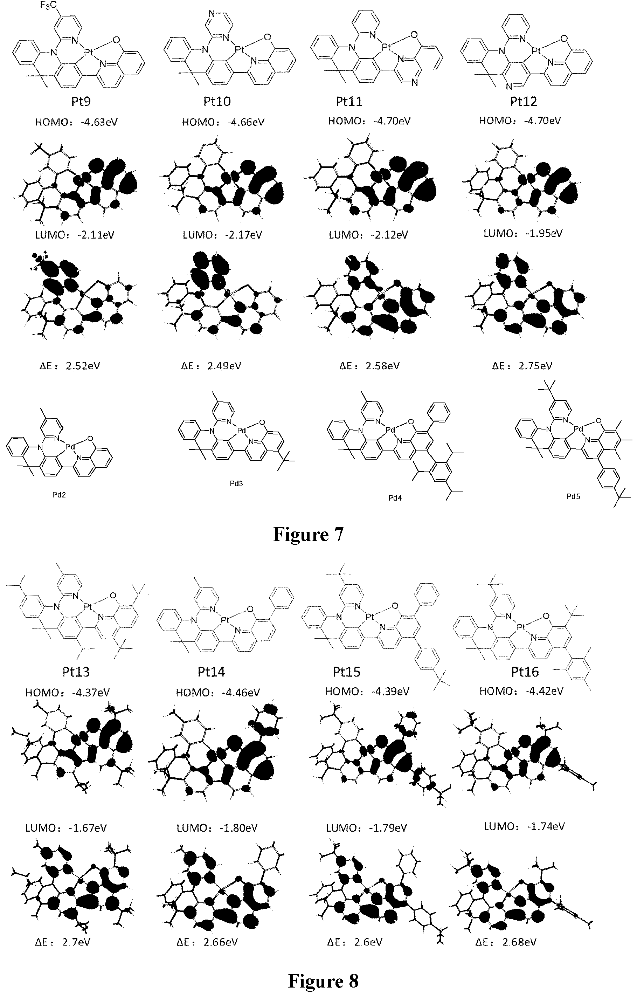

As can be seen from FIG. 7, for Pt9, compared with Pt1, its LUMO is partially distributed on a pyridine ring, and the LUMO distribution is delocalized in a direction toward the pyridine ring with the electron-accepting group, trifluoromethyl, being introduced on pyridine, so that its LUMO is mainly distributed in a pyridine part and there is little influence on distribution of HOMO, which reduces △E and is expected to red shift the emission spectrum. For Pt10, compared with Pt1, because an nitrogen atom is similar to the electron-accepting group, trifluoromethyl, it can be seen that its LUMO is partially delocalized to pyridine similar to Pt9, which reduces △E and is expected to red shift the emission spectrum. For Pt11, compared with Pt1, with the nitrogen atom being introduced into the quinoline part, it can be seen that its LUMO is partially delocalized to pyridine, which reduces LUMO and is expected to red shift the emission spectrum. Pt12 showed similar results.

As can be seen from FIG. 8, for Pt13, compared with Pt1, LUMO and HOMO energy levels are increased at the same time without changing its energy band, which can serve to match more suitable main materials in the device. For Pt14, compared with Pt2, the conjugated system is enlarged due to introduction of phenyl, which causes its delocalized HOMO and little change in ΔE. For Pt15, compared with Pt3, with a phenyl group being introduced into quinoline and a tert-butyl group into pyridine. It can be seen that apart from certain conjugated delocalization effect of the phenyl group on its HOMO, it has little influence on HOMO and LUMO distribution, and thus, introduction of the tert-butyl group into the pyridine ring can effectively increase its steric hindrance, and has little influence on electron cloud distribution density, which can improve its exciton utilization. However, introduction of the conjugated system into a quinoline region can enhance molecular rigidity but have little influence on electron cloud distribution. For Pt16, compared with Pt4, with a tert-butyl group being introduced into quinoline and pyridine respectively, the tert-butyl group on the pyridine ring has little influence on the electron cloud distribution density and increases steric hindrance, which facilitates improving of exciton utilization. By observing the electron cloud density distribution, the tertiary butyl group on a quinoline side has little influence on its distribution, thus with little change in the spectral properties, and a structure with two tertiary butyl groups can be more conducive to improving exciton utilization.

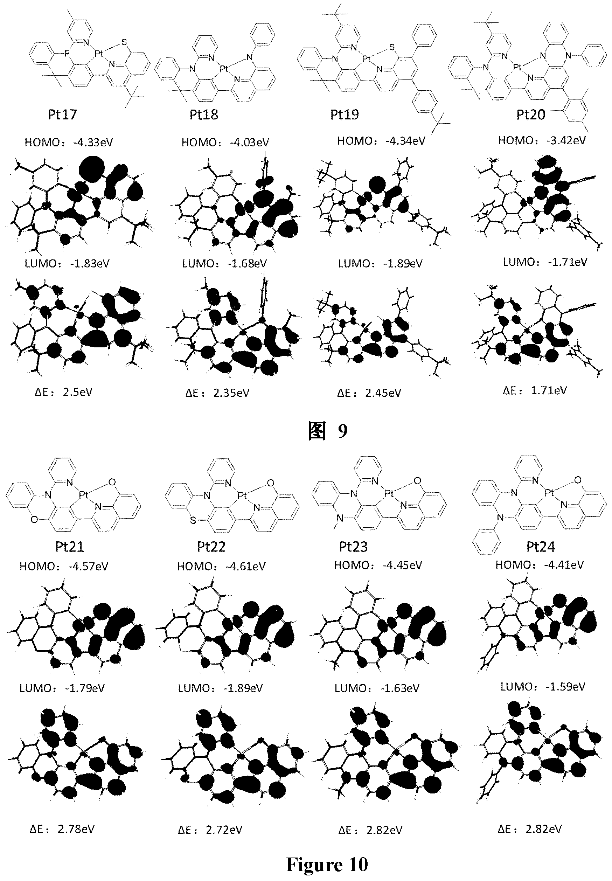

As can be seen from FIG. 9, for Pt17, compared with Pt5, it can be seen from comparison between Pt2 and Pt1 that introduction of methyl in the pyridine ring has little influence on the electron cloud distribution. If an oxygen atom is replaced with a sulfur atom, its HOMO is delocalized to the sulfur atom because of stronger electron absorption ability of the sulfur atom than the oxygen atom, which reduces △E and is expected to red shift the emission spectrum. For Pt18, compared with Pt1, the oxygen atom is replaced with aniline, and a dihedral angle of a conjugated plane between phenyl and quinoline is large, which breaks the conjugated system and enhances molecular rigidity. Its electron-donating ability significantly increases HOMO, which greatly reduces △E and is expected to red shift the emission spectrum. For Pt19, compared with Pt17, a dihedral angle between phenyl and p-tert-butylphenyl with the conjugate plane is large, which breaks the conjugate system, and thus there is little influence on distribution of HOMO and LUMO, while it is proved above that the tertiary butyl on the pyridine ring has no obvious influence on the electron cloud distribution, and thus its steric hindrance effect can be increased by introducing tertiary butyl on the pyridine ring and p-tert-butylphenyl on the quinoline ring, which facilitates suppressing of π -π stacking between molecules. For Pt20, compared with Pt16, with a strong electron donating group, diphenylamine, being introduced, the HOMO energy level is greatly increased, which reduces ΔE, causes the emission spectrum to red shift, and thus can substantially function in adjusting its luminescent color.

As can be seen from FIG. 10, Pt21, Pt22, Pt23 and Pt24 are compared with Pt1. According to the electron cloud distribution density of Pt1, its HOMO is mainly distributed in the quinoline conjugated system near the metal Pt atom, while LUMO is mainly distributed in the pyridine ring and quinoline conjugated system, while a bridging position of a six-membered ring has little influence on the HOMO and LUMO, so Pt21 to Pt24 has little influence on regulation of electron cloud distribution of molecules compared with Pt1.

To sum up, its LUMO level can be significantly reduced by introducing a strong electron-accepting structure at an oxygen para-position in the quinoline ring. Its LUMO energy level can reduced by introducing a strong electron-accepting structure into the pyridine ring. Introduction of p-tert-butylphenyl or trimethylphenyl at the oxygen para-position in quinoline ring can suppress π -π stacking of molecules with little influence on HOMO and LUMO, which facilitates purification of materials. Proper addition of a conjugated structure in the quinoline ring can enhance its molecular rigidity and extend the conjugated system, and thus its photophysical properties can be adjusted.

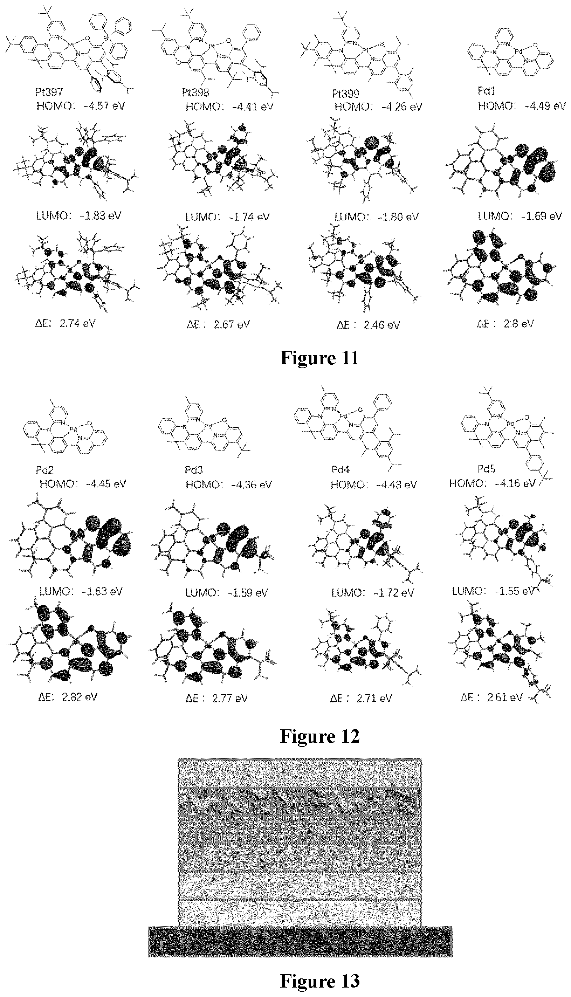

Application of the metal platinum (II) and palladium (II) complex luminescent material containing quinoline structural units as a light-emitting layer of an organic electroluminescent device. In an organic light emitting element, carriers are injected into a luminescent material from positive and negative electrodes to generate a luminescent material at an excited state and cause it to illuminate. The compound of the present disclosure represented by the general formula (1) can be applied as a luminescent material to an organic light emitting element such as an organic photoluminescent element or an organic electroluminescent element. The organic photoluminescence element has a structure in which at least a light-emitting layer is formed on a substrate. In addition, the organic electroluminescent element has a structure in which at least an anode, a cathode, and an organic layer between the anode and the cathode are formed. The organic layer includes at least a light-emitting layer and may be composed of only the light-emitting layer or may have more than one organic layer in addition to the light-emitting layer. Example of such other organic layers include a hole transport layer, a hole injection layer, an electron blocking layer, a hole blocking layer, an electron injection layer, an electron transport layer, an exciton blocking layer, and the like. The hole transport layer may be a hole injection and transport layer having a hole injection function, and the electron transport layer may be an electron injection and transport layer having an electron injection function. Specifically, a structure of the organic light emitting element is schematically shown in FIG. 13. In FIG. 13, there are seven layers in total from bottom to top, in which the substrate, the anode, the hole injection layer, the hole transport layer, the light-emitting layer, the electron transport layer, and the cathode are sequentially shown, where the light-emitting layer is a mixed layer in which a guest material is doped into a main material.

The complexes expressed in examples 1 to 3 were applied to an OLED device as a phosphorescent luminescent material, with a structure being expressed as:
ITO /HATCN (10 nm)/NPD (65 nm)/mCBP: complexes expressed in Examples 1 to 3 (5 to 20 wt.%, 20 nm)/BAlq (10 nm)/BPyTP (30 nm)/LiF (1 nm)/Al (100 nm) , in which ITO is a transparent anode; HATCN is a hole injection layer, NPD is a hole transport layer, mCBP is a main material, and the complex expressed in Examples 1 to 3 (5 to 20 wt.% refers to a doping concentration and 20 nm is a thickness of the light-emitting layer) is a guest material, BAlq is a hole blocking layer, BPyTP is an electron transport layer, LiF is an electron injection layer, and Al is a cathode. Numbers in nanometer (nm) in parentheses refer to thicknesses of films.

It should be noted that the structure is an example of application of the luminescent material of the present disclosure and does not limit a specific structure of the OLED device of the luminescent material of the present disclosure, and the luminescent material is not limited to the compounds expressed in the examples 1 to 3.

A molecular formula of the material used in the device is as follows:

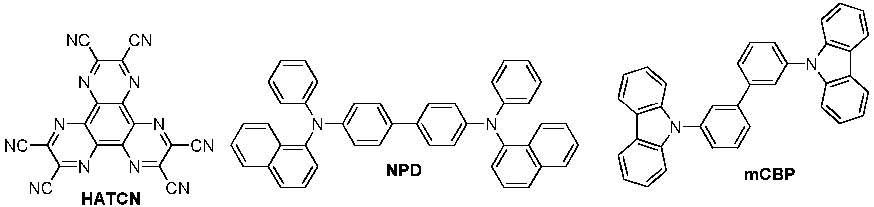

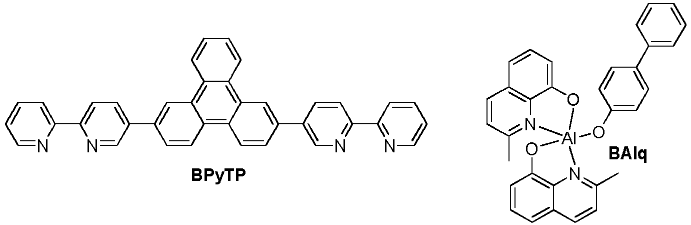

According to above device embodiments, external quantum efficiency (EQE), an operating voltage, lifetime and other characteristics of the OLED device are shown in Table 2 below.

**Table 2**

| Example | Compound | Operating voltage (Volt) | EQE | LT90 (hour) |
|---|---|---|---|---|
| Control device 1 | PtQ₂ | 3.8 | 6.5% | 10 |
| Device example 1 | Pt1 | 3.7 | 15.6% | 25 |
| Device example 2 | Pt2 | 3.7 | 16.0% | 32 |
| Device example 3 | Pt3 | 3.6 | 17.3% | 53 |

## Claims

1. A platinum (II) and palladium (II) complex containing quinoline structural units, wherein the platinum (II) and palladium (II) complex containing quinoline structural units has a structure shown in following general formula (I): 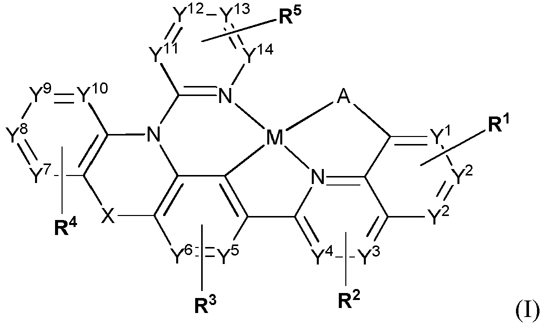 where
M is selected from Pt or Pd;
A is selected from O, S or NR⁶;
X is O, S, CH₂, CHD, CD₂, CR⁷R⁸, C=O, SiR⁹R¹⁰, NH, ND, NR¹¹, PH, PD, PR¹², R¹³P=O, S=O, or SO₂;
Y¹, Y², Y³, Y⁴, Y⁵, Y⁶, Y⁷, Y⁸, Y⁹, Y¹⁰, Y¹¹, Y¹², Y¹³ and Y¹⁴ are each independently N or CH;
substitution modes of R¹, R², R³, R⁴, R⁵, R⁶, R⁷, R⁸, R⁹, R¹⁰, R¹¹, R¹² and R¹³ are each independently expressed as single substitution, double substitution, triple substitution, four substitution or no substitution; R¹, R², R³, R⁴, R⁵, R⁶, R⁷, R⁸, R⁹, R¹⁰, R¹¹, R¹² and R¹³ each independently represent hydrogen, deuterium, alkyl, haloalkyl, cycloalkyl, alkoxy, aryl, heteroaryl, aryloxy, halogen, cycloalkenyl, heterocyclyl, alkenyl, alkynyl, hydroxyl, mercapto, nitro, cyano, amino, mono-or dialkylamino, mono-or diarylamino, ester, nitrile, isonitrile, heteroaryl, alkoxycarbonyl, amido, alkoxycarbonylamino, aryloxycarbonylamino, sulfonylamino, sulfamoyl, carbamoyl, alkylthio, sulfinyl, ureido, phosphoramido, imino, sulfo, carboxyl, hydrazino, silyl, substituted silyl, polymeric groups, or combinations thereof, and two or more adjacent R¹, R², R³, R⁴, R⁵, R⁶, R⁷, R⁸, R⁹, R¹⁰, R¹¹, R¹², and R¹³ is selectively linked to form a condensed ring.

2. The metal platinum (II) and palladium (II) complex containing quinoline structural units according to claim 1, wherein the platinum complex has structures shown in following general formula (II), (III), (IV) or (V): 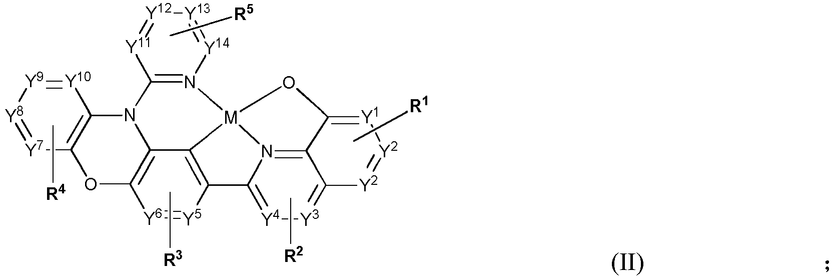 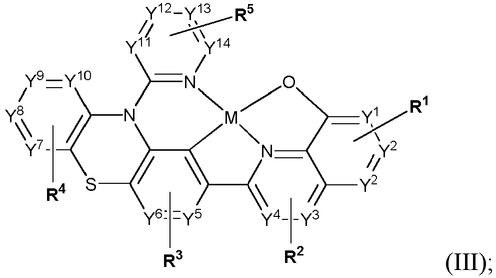 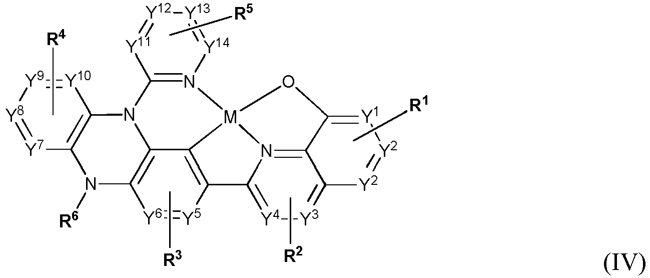 or

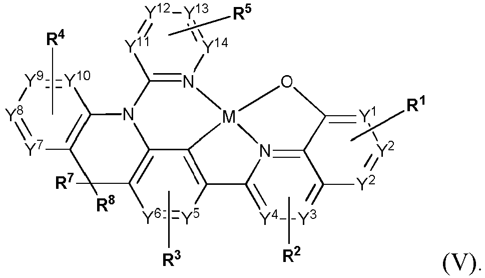

3. The metal platinum (II) and palladium (II) complex containing quinoline structural units according to claim 1, 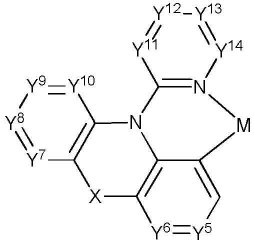 on the left is preferably of a structure selected from one of following structures:

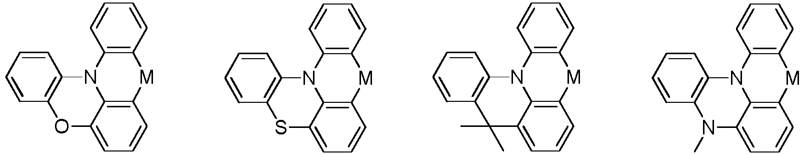

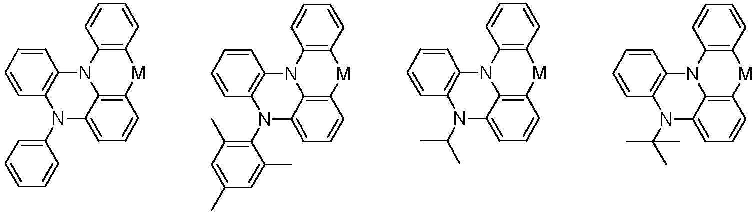

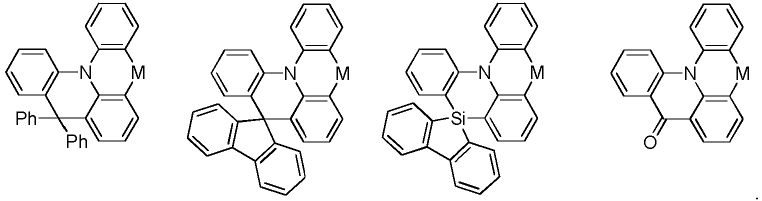

4. The metal platinum (II) and palladium (II) complex containing quinoline structural units according to claim 1, wherein the platinum (II) and palladium (II) complex has one of following structures:

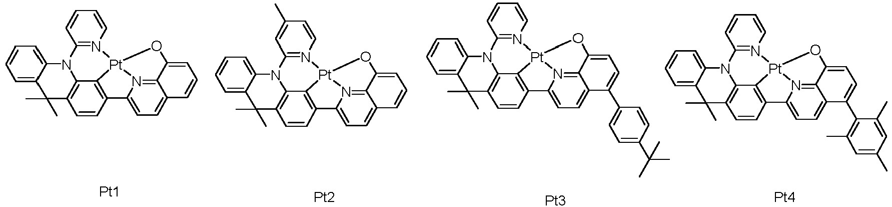

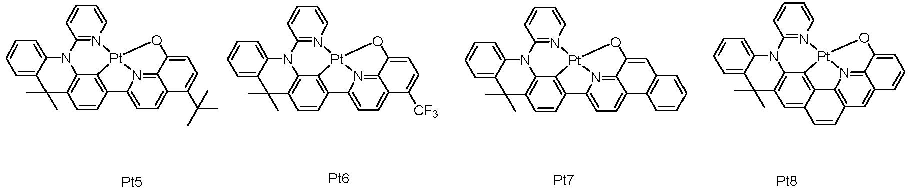

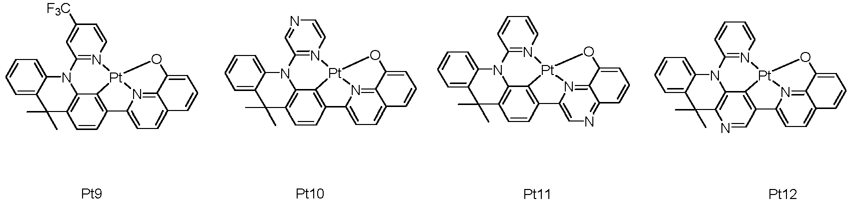

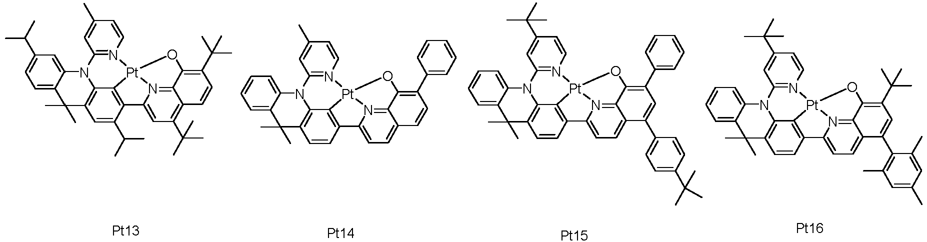

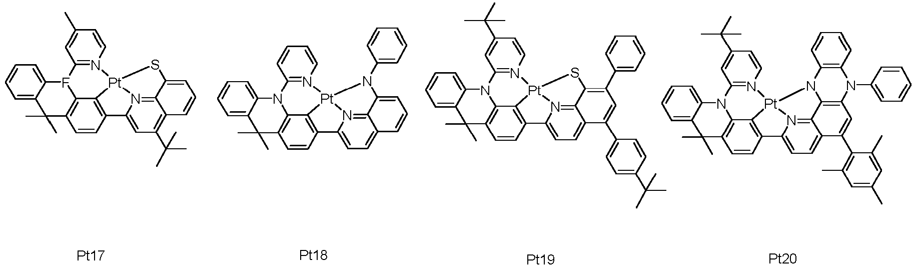

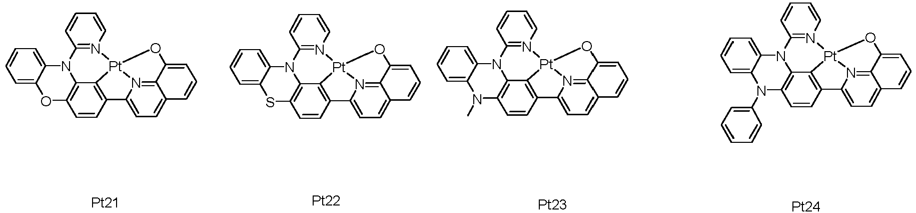

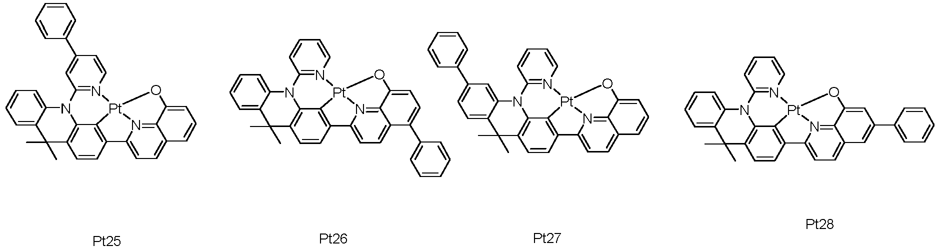

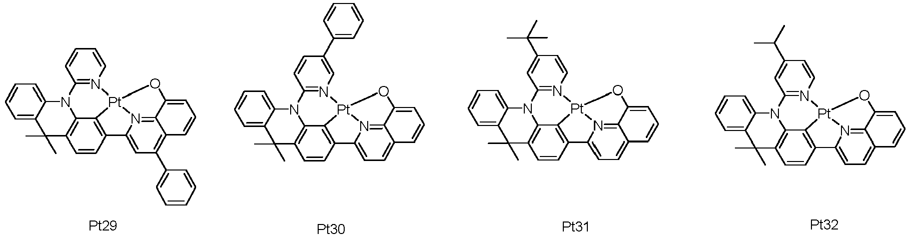

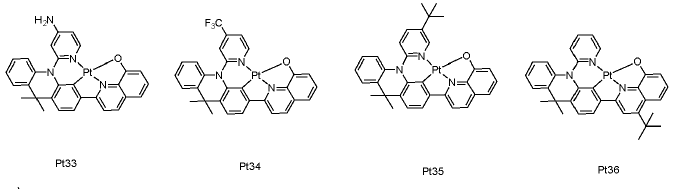

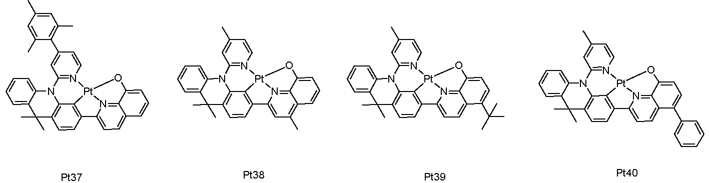

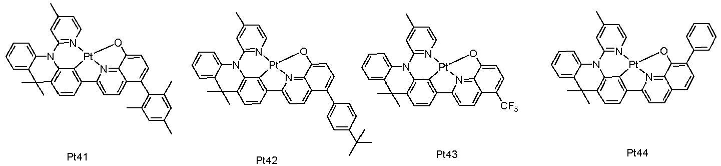

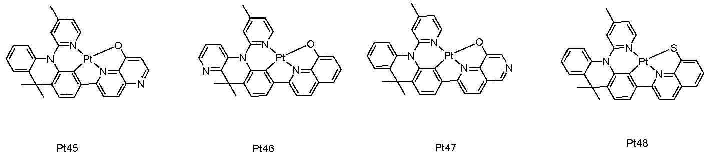

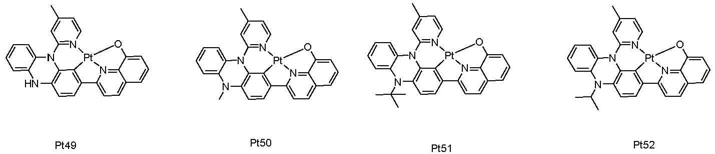

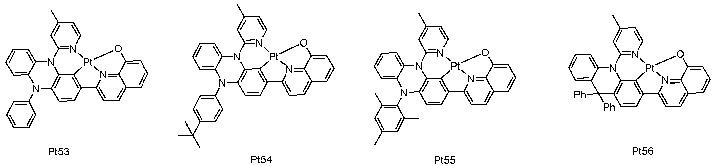

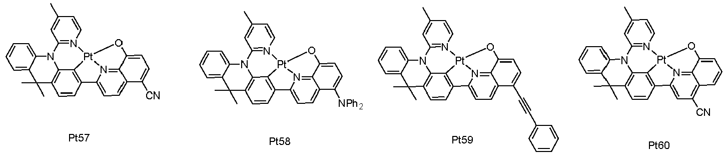

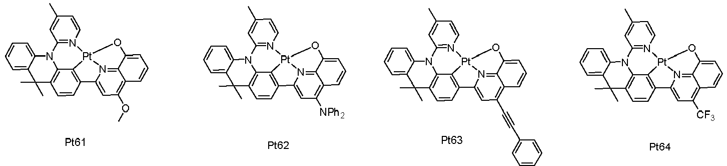

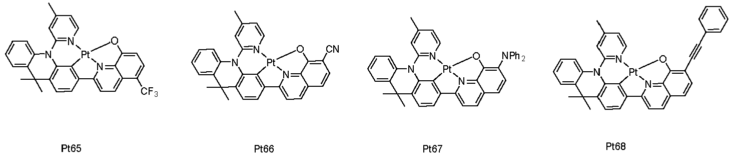

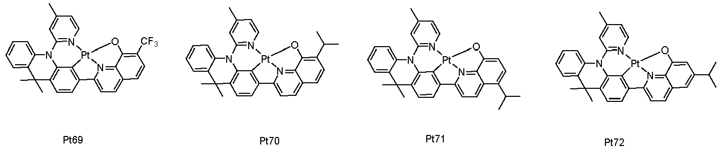

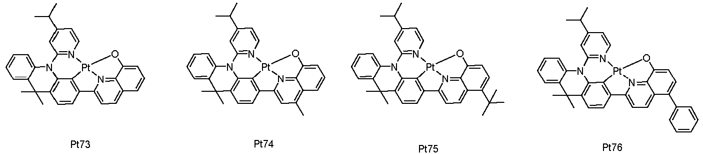

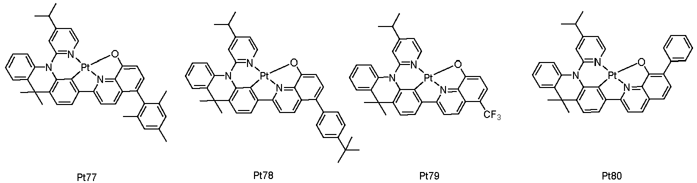

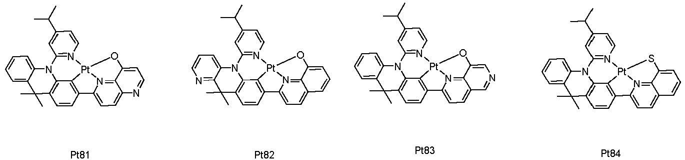

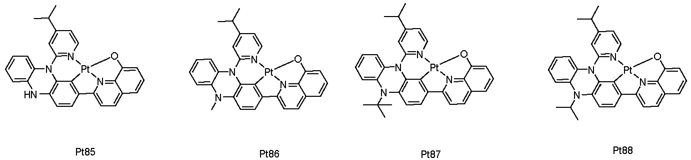

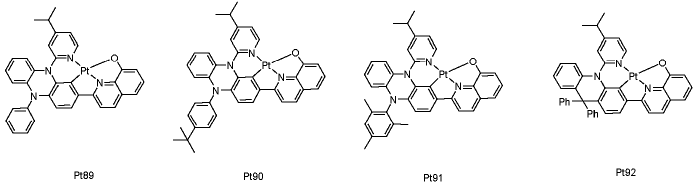

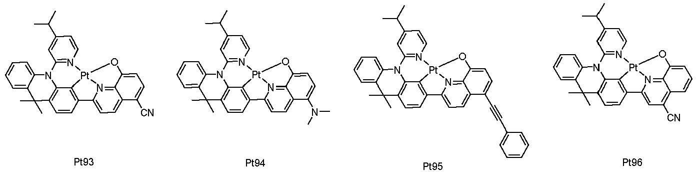

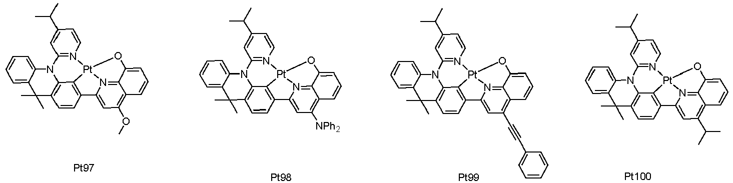

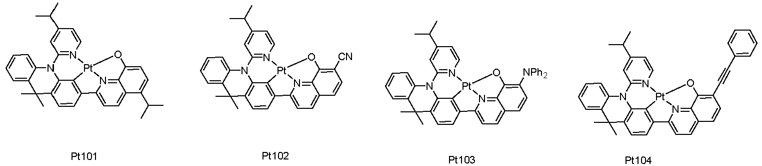

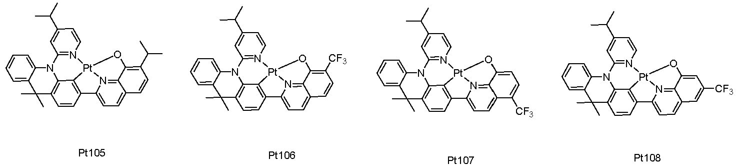

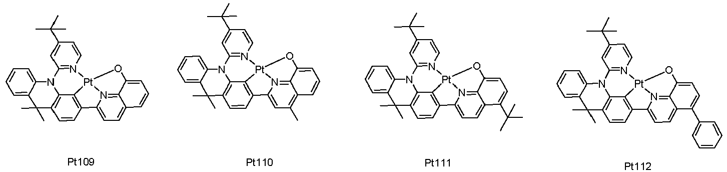

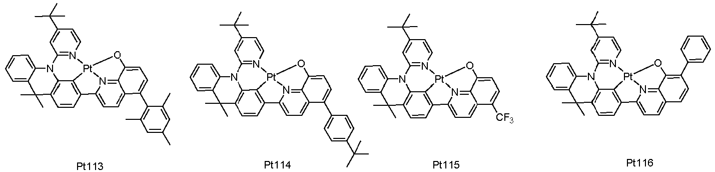

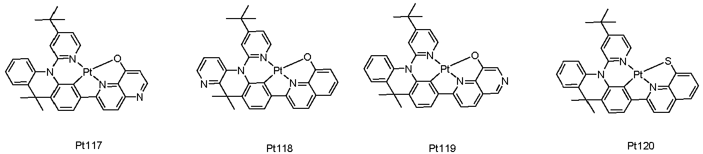

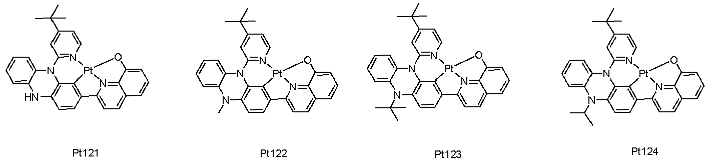

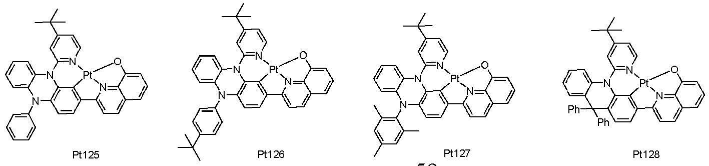

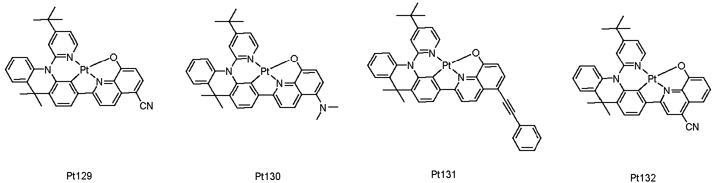

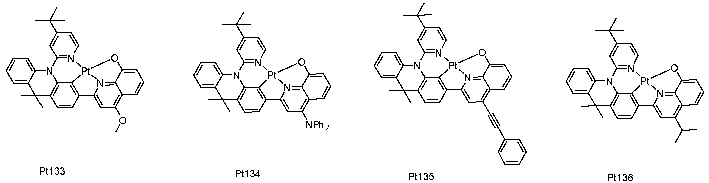

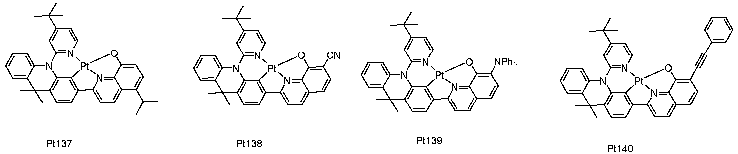

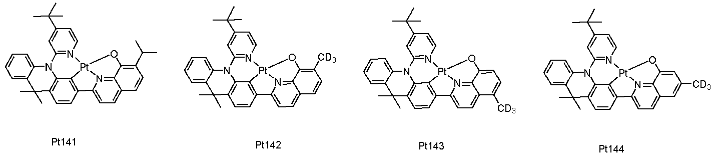

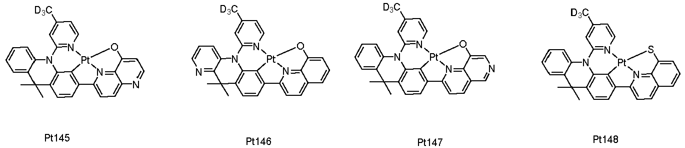

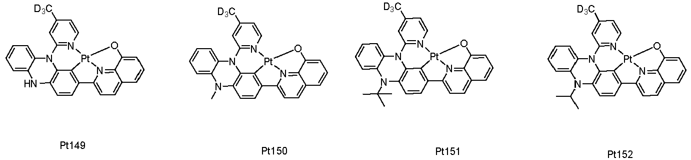

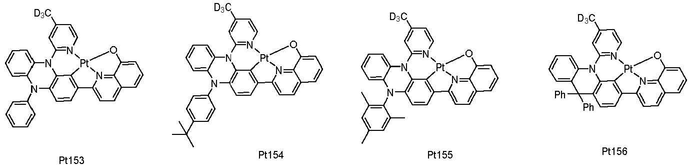

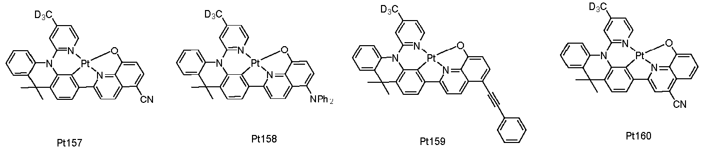

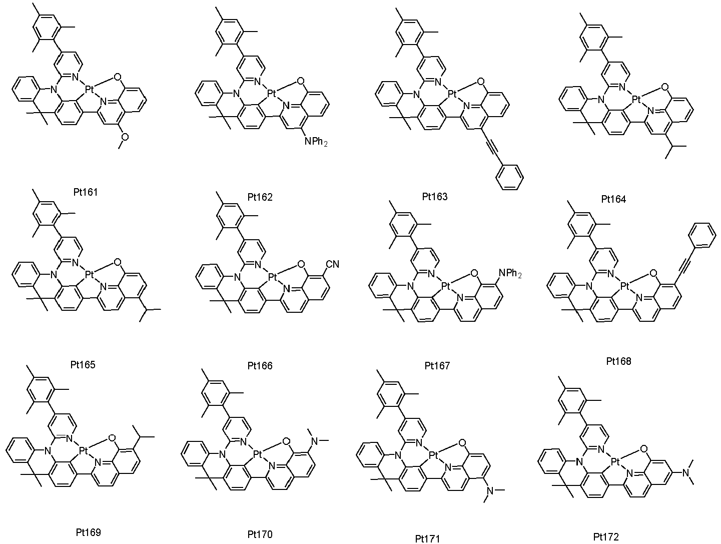

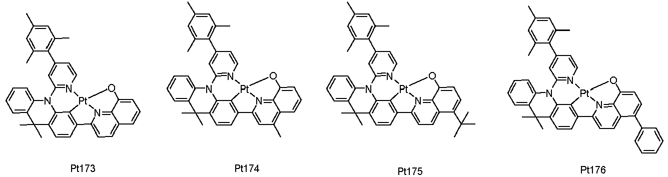

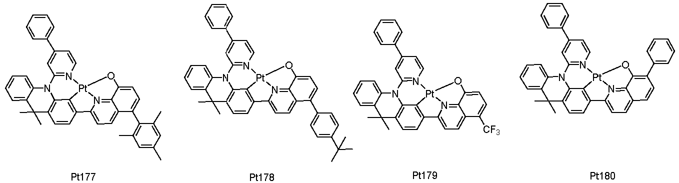

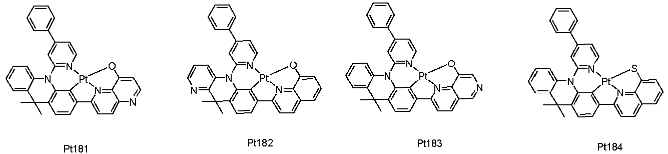

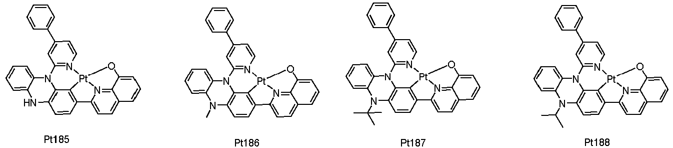

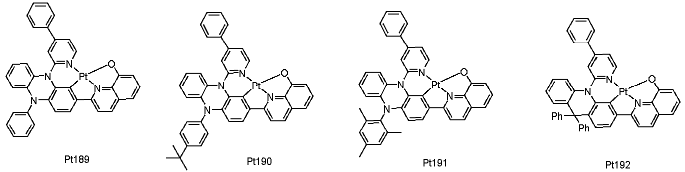

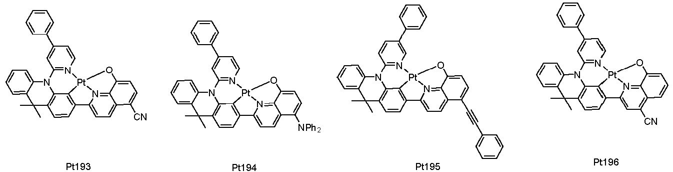

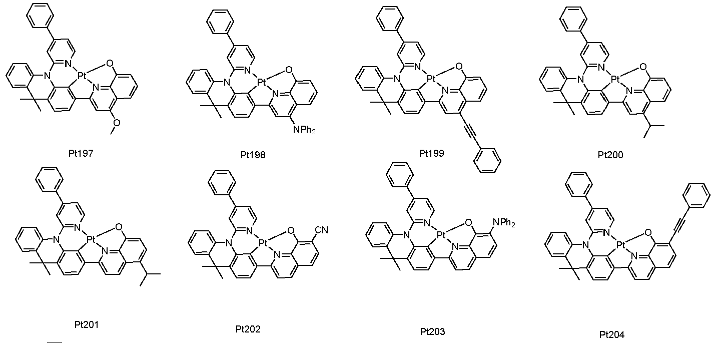

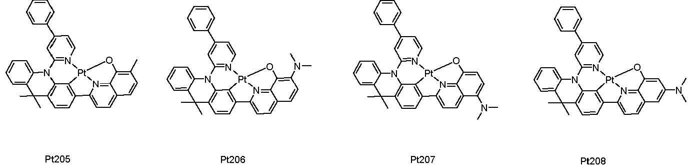

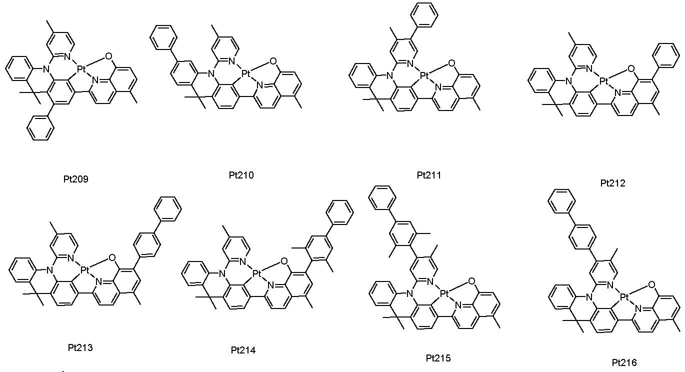

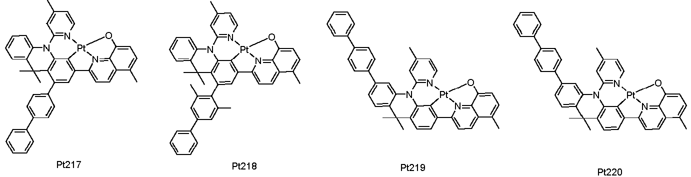

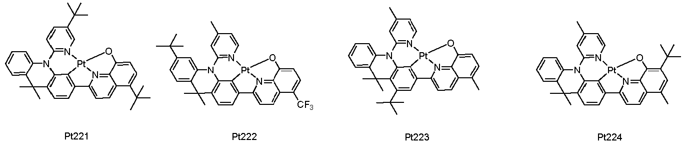

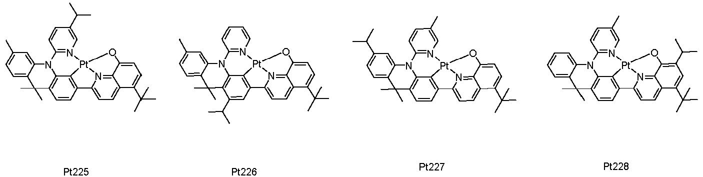

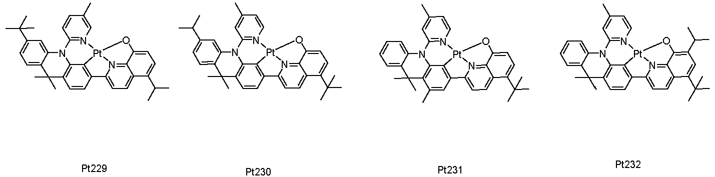

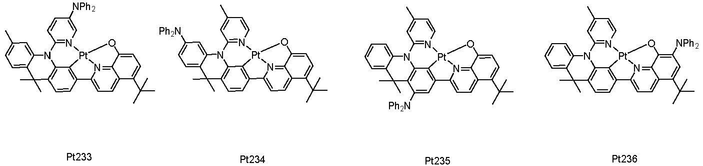

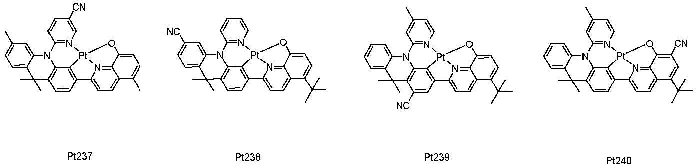

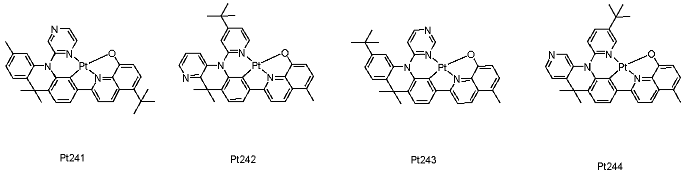

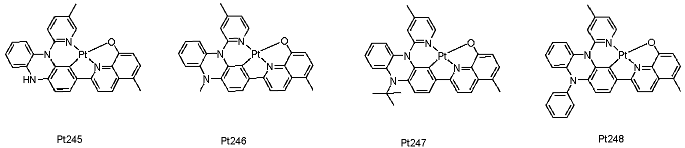

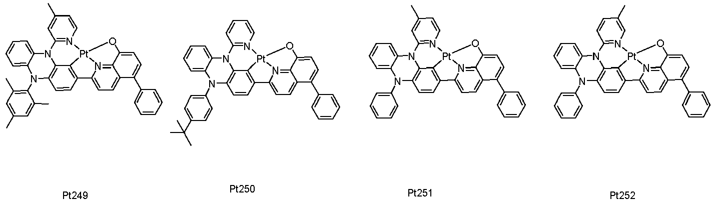

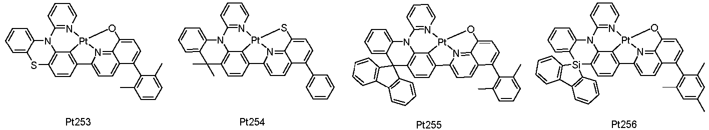

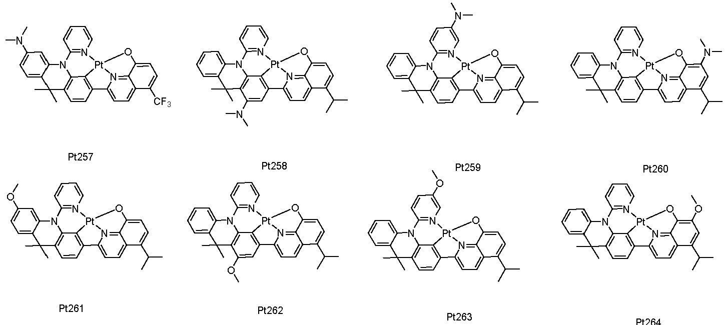

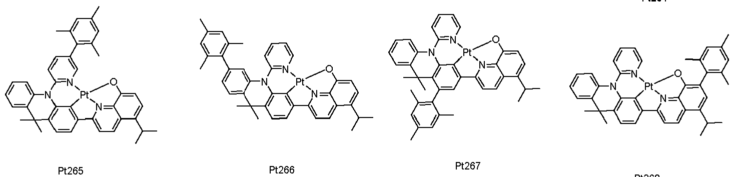

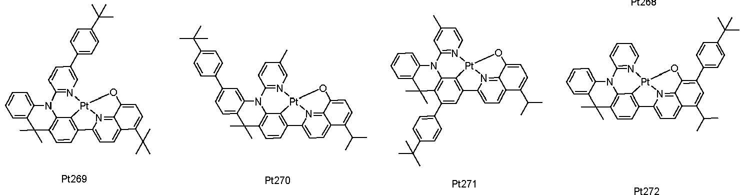

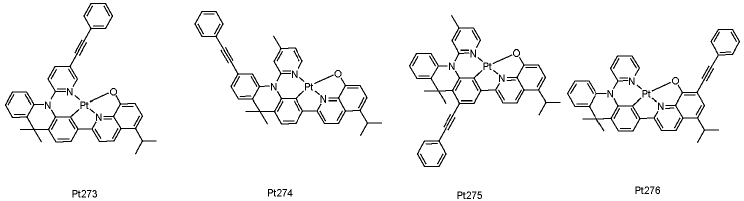

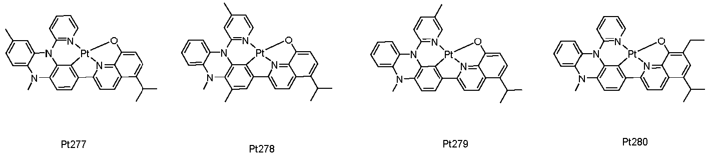

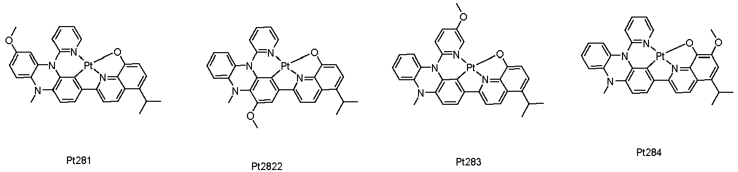

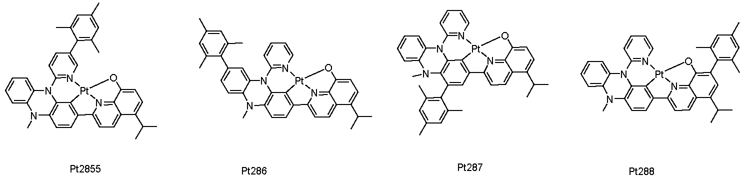

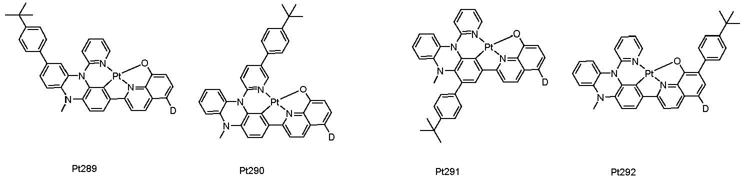

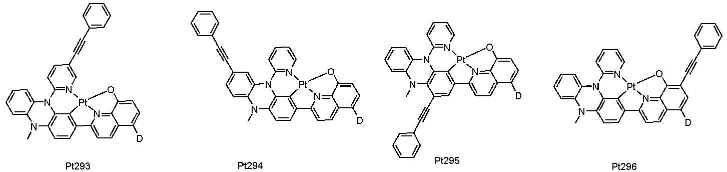

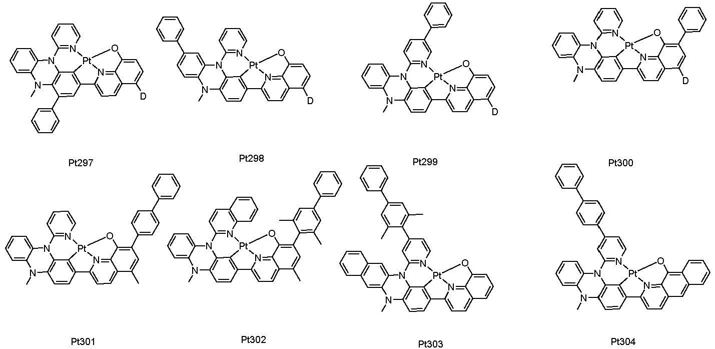

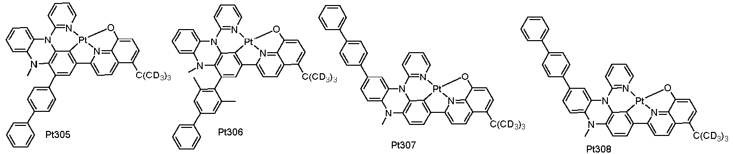

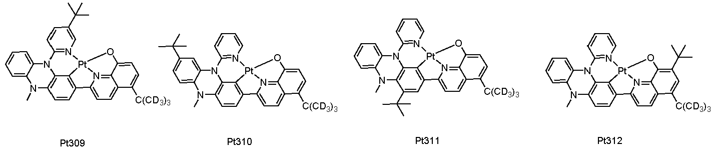

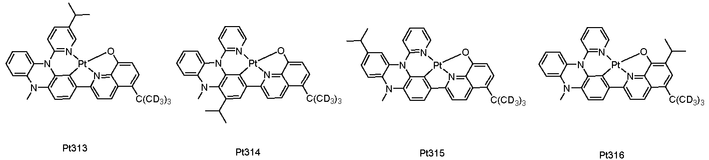

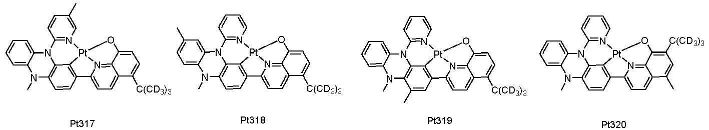

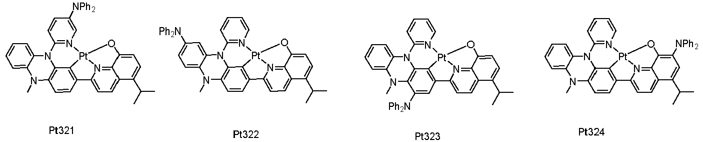

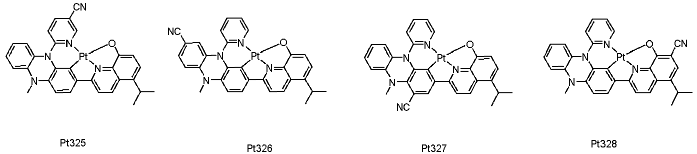

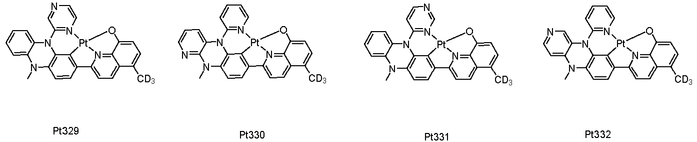

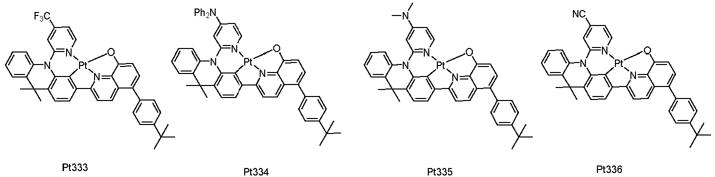

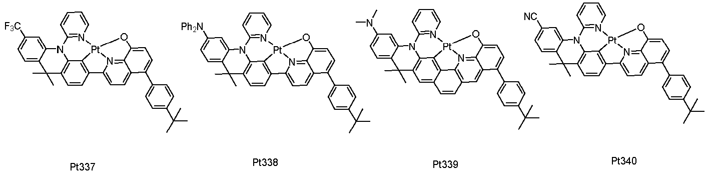

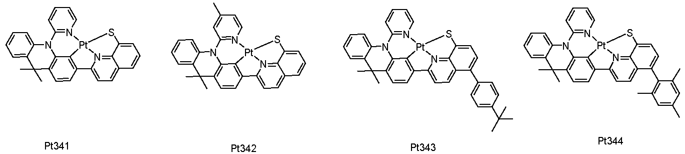

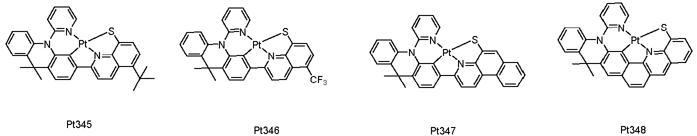

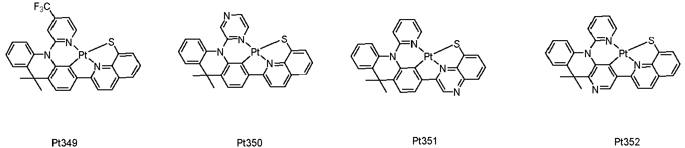

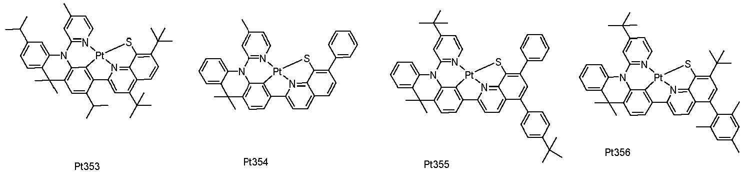

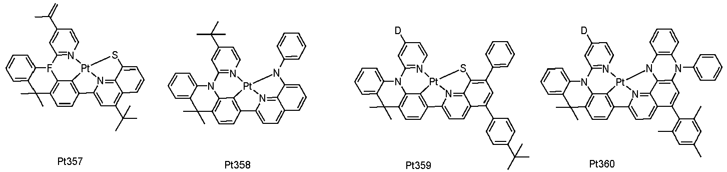

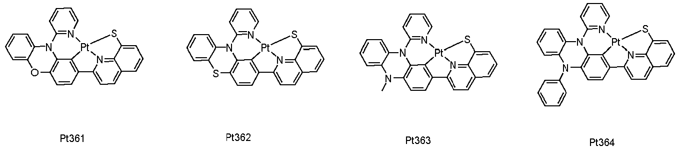

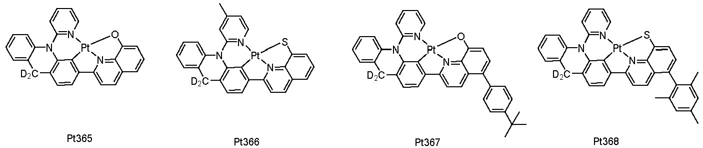

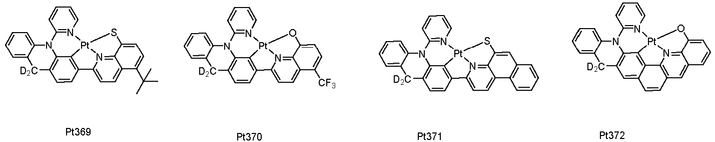

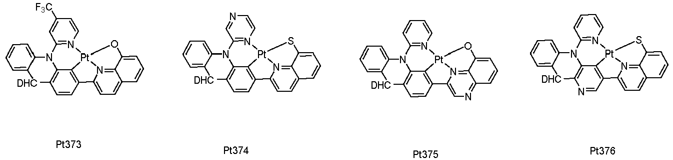

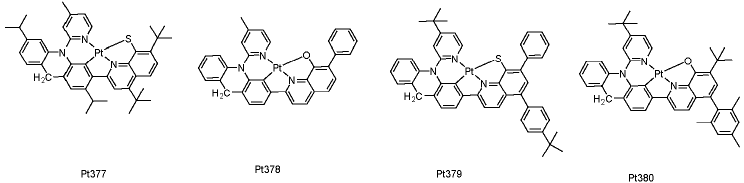

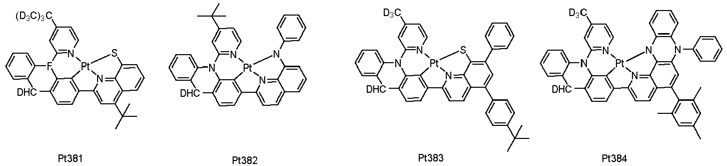

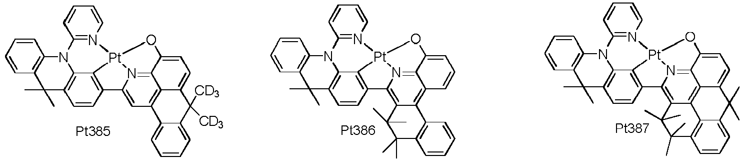

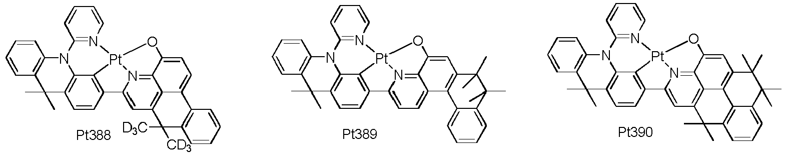

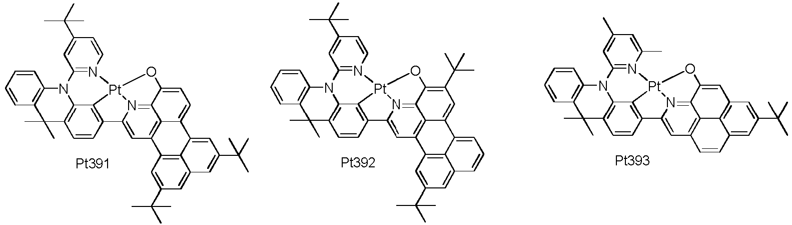

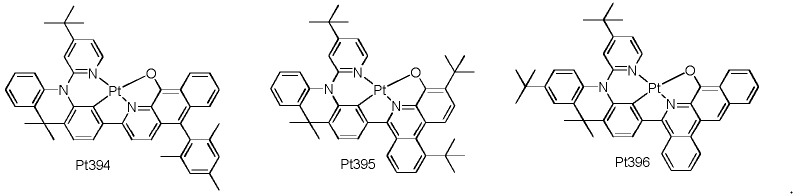

5. Application of the metal platinum (II) or palladium (II) complex containing quinoline structural units according to any one of claims 1 to 4 in electroluminescent devices.

6. A device, comprising the metal platinum (II) or palladium (II) complex containing quinoline structural units according to any one of claims 1 to 4.

7. The device according to claim 6, wherein the device is a full-color display, a photovoltaic device, a light-emitting display device or an organic light-emitting diode.

8. The device according to claim 6, comprising at least one cathode, at least one anode and at least one luminescent layer, wherein at least one of the at least one luminescent layer comprises any one of the metal platinum (II) and palladium (II) complexes containing quinoline structural units according to any one of claims 1 to 4.

9. The device according to claim 6, wherein the device is an organic light-emitting diode, with a light-emitting layer containing the metal platinum (II) or palladium (II) complex containing quinoline structural units and a corresponding main material, wherein a mass percentage of the metal platinum (II) or palladium (II) complex is 1% to 50%, and a mass percentage of the main material is not limited.

10. A display or lighting device, comprising the device according to any one of claims 6 to 9.

## Patentansprüche

1. Platin(II)-und-Palladium(II)-Komplex, enthaltend Chinolin-Struktureinheiten, wobei der Platin(II)-und-Palladium(II)-Komplex, enthaltend Chinolin-Struktureinheiten, eine Struktur aufweist, die in der folgenden allgemeinen Formel (I) gezeigt ist: 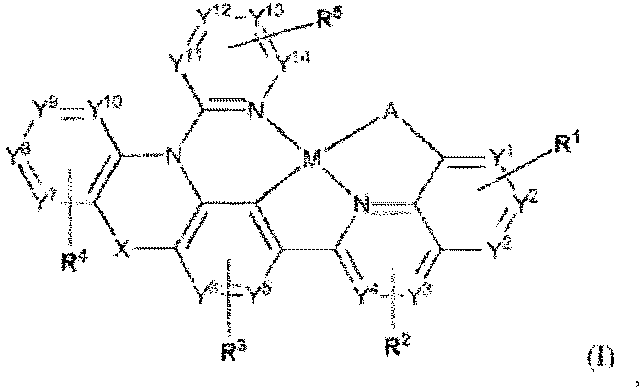 in der
M aus Pt oder Pd ausgewählt ist;
A aus O, S oder NR⁶ ausgewählt ist;
X O, S, CH₂, CHD, CD₂, CR⁷R⁸, C=O, SiR⁹R¹⁰, NH, ND, NR¹¹, PH, PD, PR¹², R¹³P=O, S=O oder SO₂ ist;
Y¹, Y², Y³, Y⁴, Y⁵, Y⁶, Y⁷, Y⁸, Y⁹, Y¹⁰, Y¹¹, Y¹², Y¹³ und Y¹⁴ jeweils unabhängig N oder CH sind;
Substitutionsmodi von R¹, R², R³, R⁴, R⁵, R⁶, R⁷, R⁸, R⁹, R¹⁰, R¹¹, R¹² und R¹³ jeweils unabhängig als Einzelsubstitution, Doppelsubstitution, Dreifachsubstitution, Vier-Substitution oder keine Substitution ausgedrückt sind; R¹, R², R³, R⁴, R⁵, R⁶, R⁷, R⁸, R⁹, R¹⁰, R¹¹, R¹² und R¹³ jeweils unabhängig für Wasserstoff, Deuterium, Alkyl, Haloalkyl, Cycloalkyl, Alkoxy, Aryl, Heteroaryl, Aryloxy, Halogen, Cycloalkenyl, Heterocyclyl, Alkenyl, Alkinyl, Hydroxyl, Mercapto, Nitro, Cyano, Amino, Mono- oder Dialkylamino, Mono -oder Diarylamino, Ester, Nitril, Isonitril, Heteroaryl, Alkoxycarbonyl, Amido, Alkoxycarbonylamino, Aryloxycarbonylamino, Sulfonylamino, Sulfamoyl, Carbamoyl, Alkylthio, Sulfinyl, Ureido, Phosphoramido, Imino, Sulfo, Carboxyl, Hydrazino, Silyl, substituiertes Silyl, polymere Gruppen oder Kombinationen davon stehen, und zwei oder mehr benachbarte R¹, R², R³, R⁴, R⁵, R⁶, R⁷, R⁸, R⁹, R¹⁰, R¹¹, R¹² und R¹³ selektiv verknüpft sind, um einen kondensierten Ring zu bilden.

2. Metall-Platin(II)-und-Palladium(II)-Komplex, enthaltend Chinolin-Struktureinheiten, nach Anspruch 1, wobei der Platinkomplex Strukturen aufweist, die in der folgenden allgemeinen Formel (II), (III), (IV) oder (V) gezeigt sind: 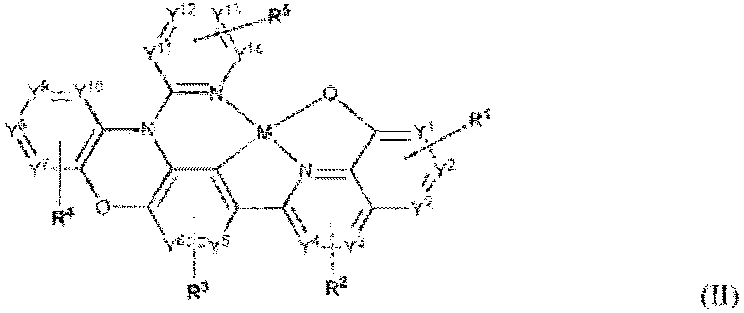 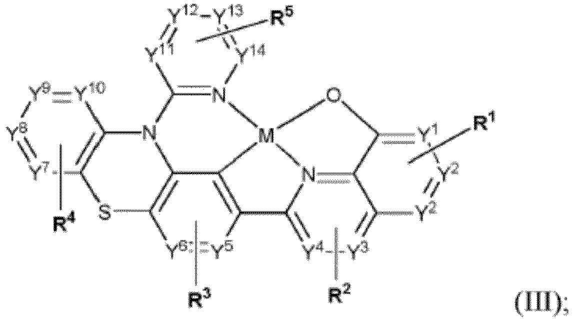 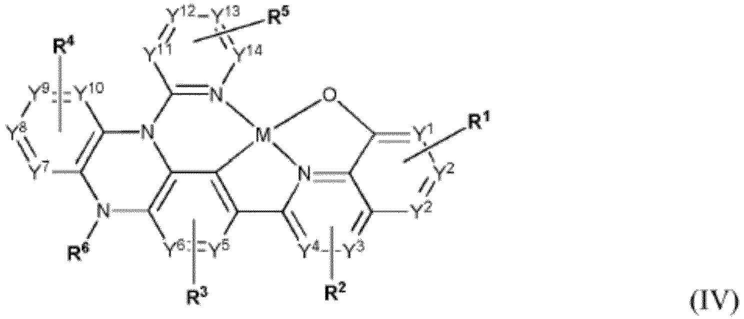 oder

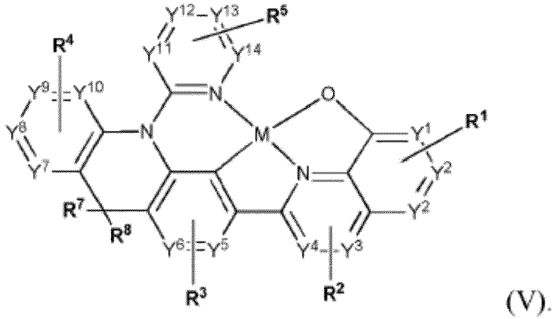

3. Metall-Platin(II)-und-Palladium(II)-Komplex, enthaltend Chinolin-Struktureinheiten, nach Anspruch 1, wobei 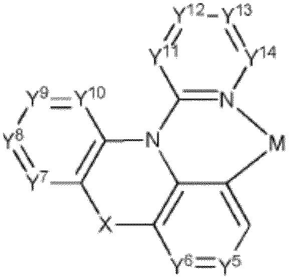 auf der linken Seite vorzugsweise eine Struktur ist, die aus einer der folgenden Strukturen ausgewählt ist:

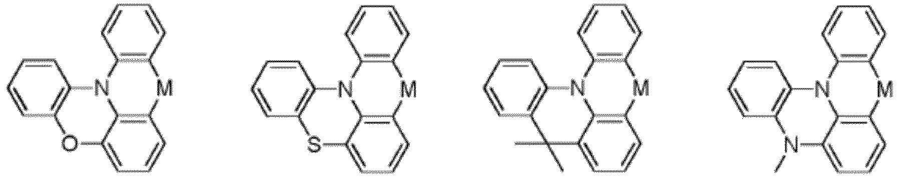

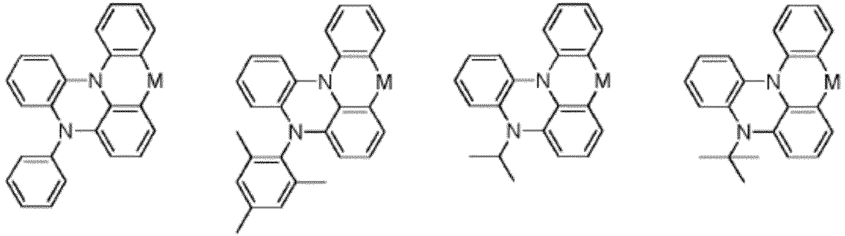

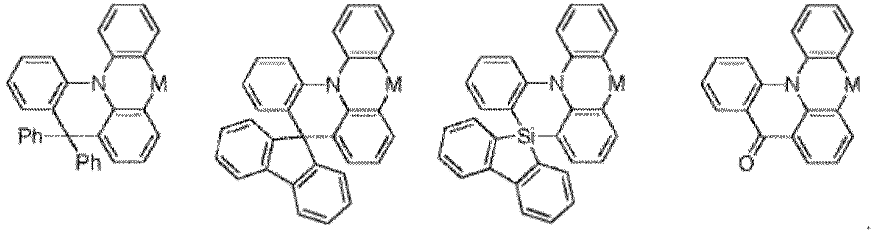

4. Metall-Platin(II)-und-Palladium(II)-Komplex, enthaltend Chinolin-Struktureinheiten, nach Anspruch 1, wobei der Platin(II)-und-Palladium(II)-Komplex eine der folgenden Strukturen aufweist:

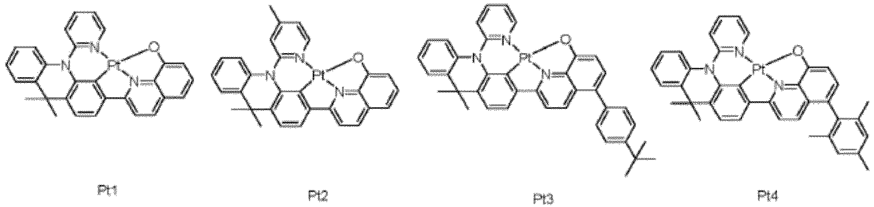

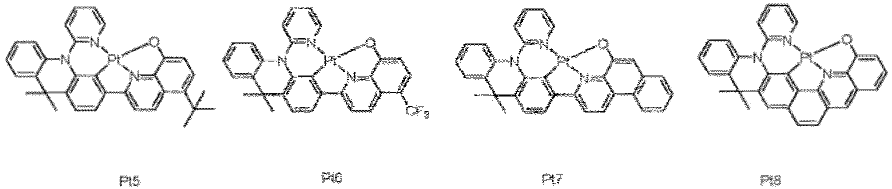

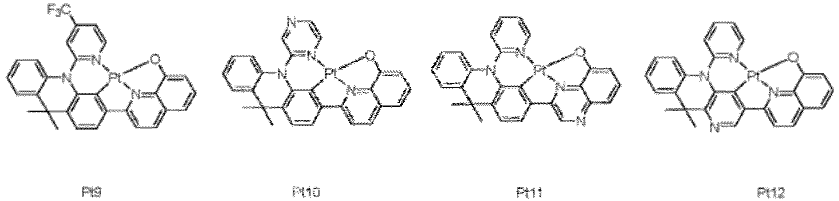

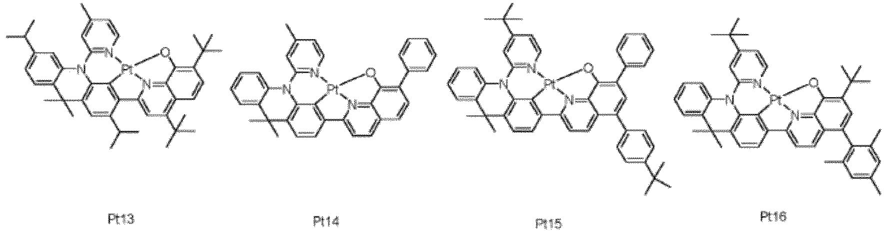

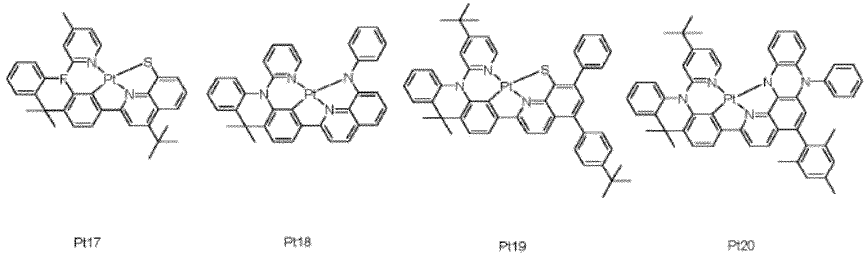

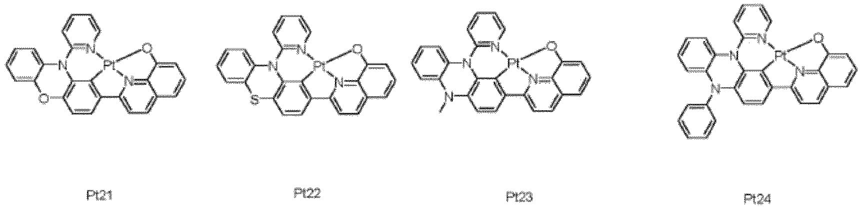

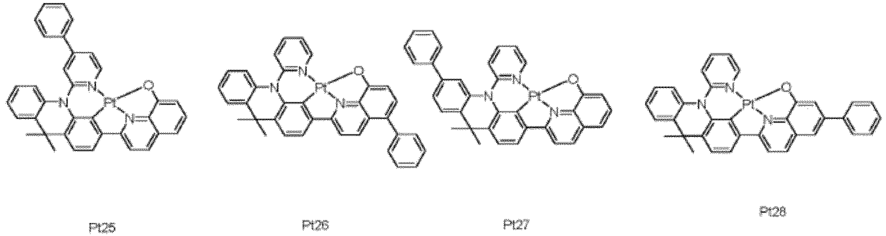

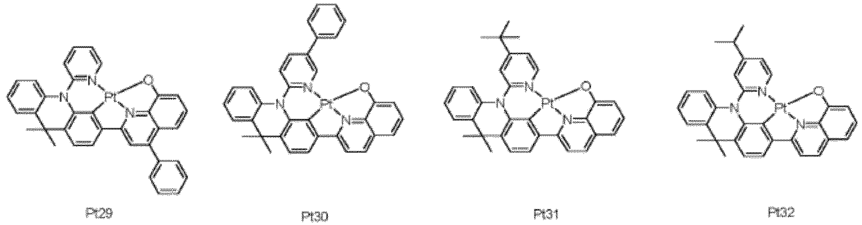

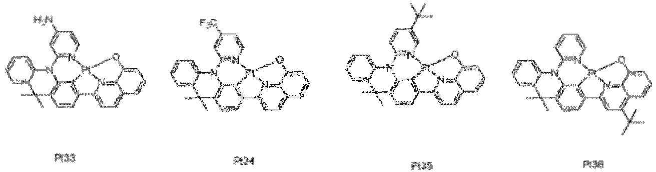

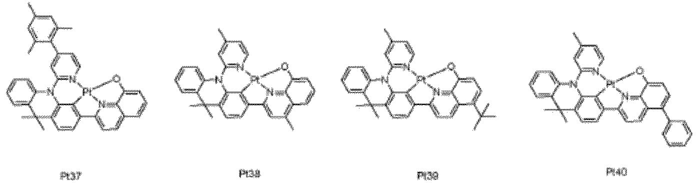

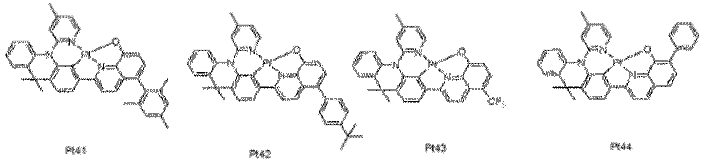

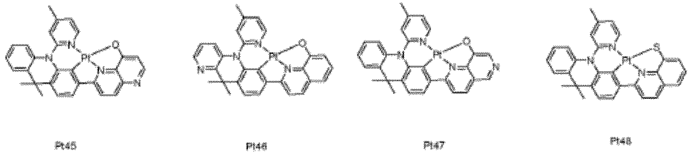

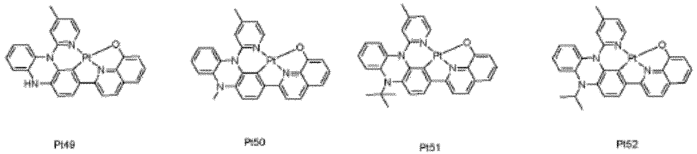

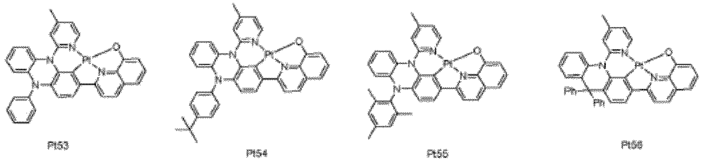

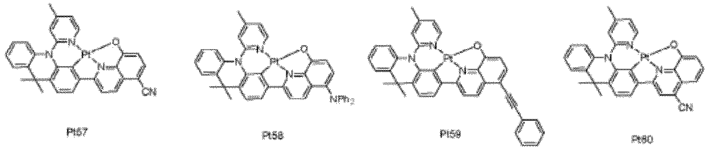

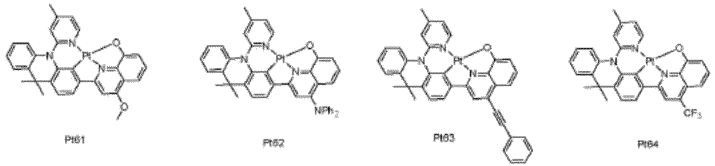

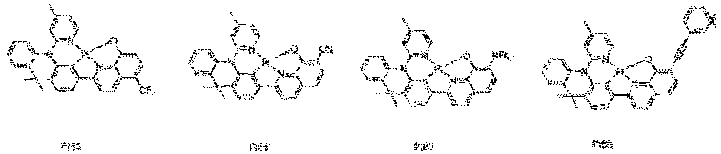

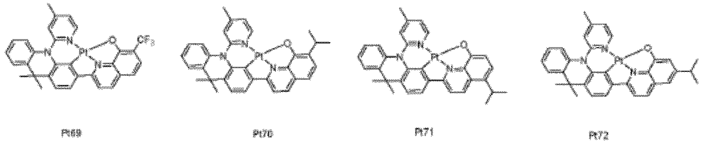

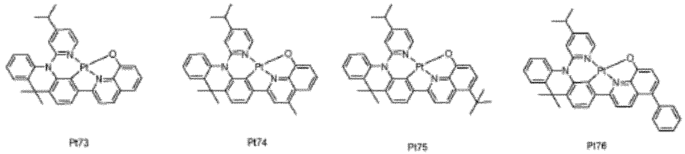

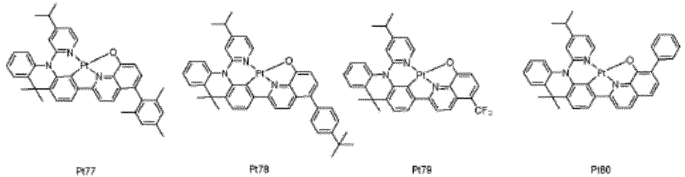

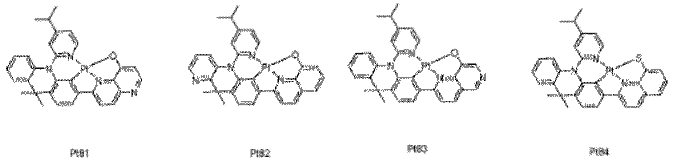

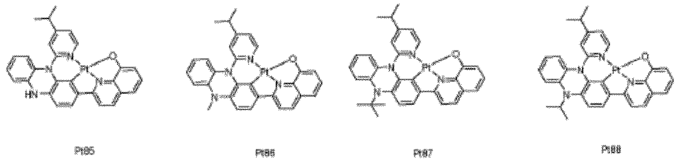

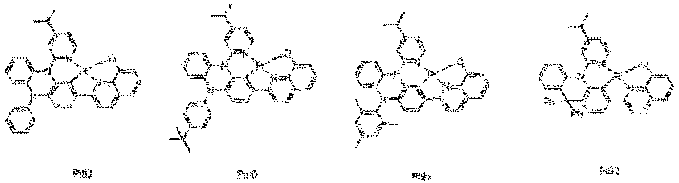

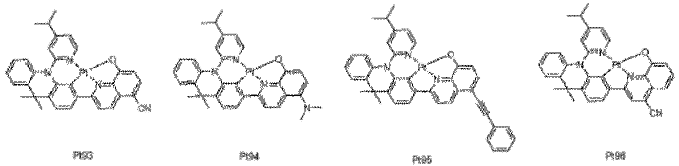

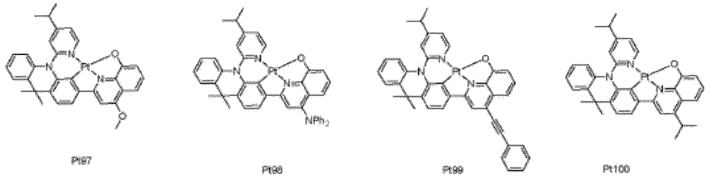

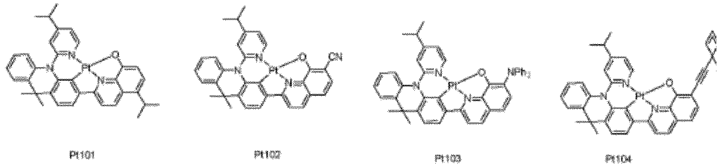

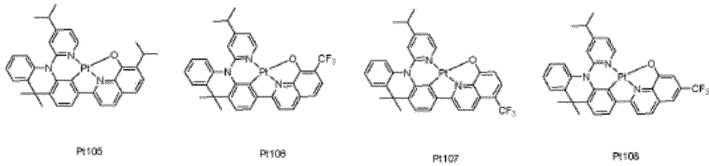

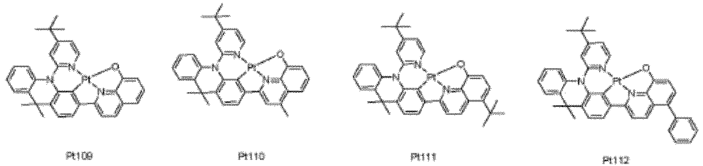

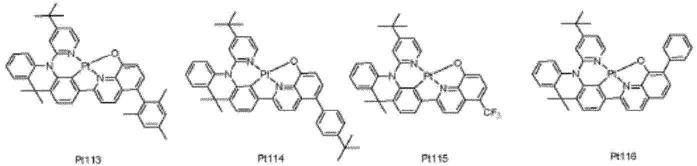

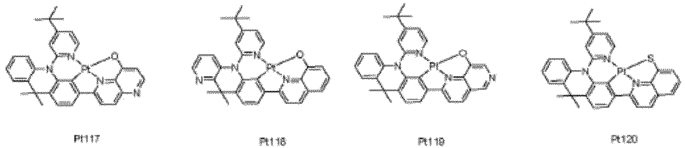

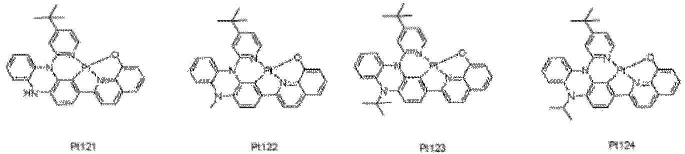

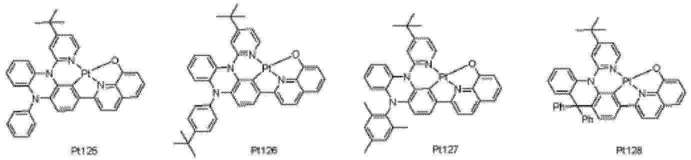

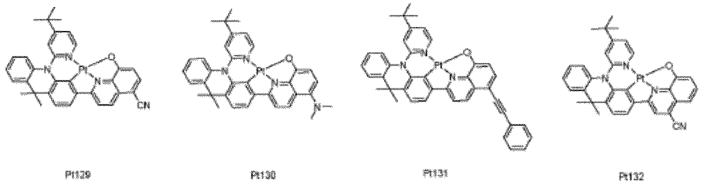

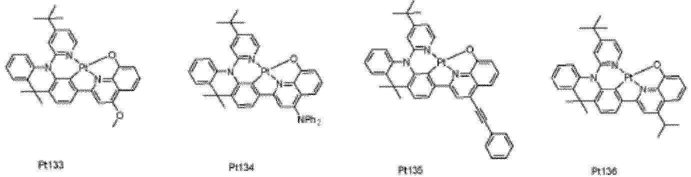

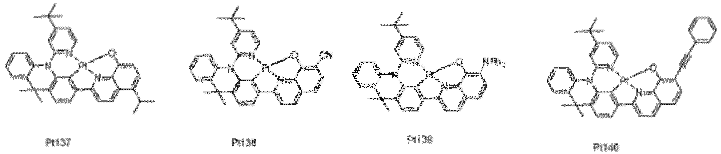

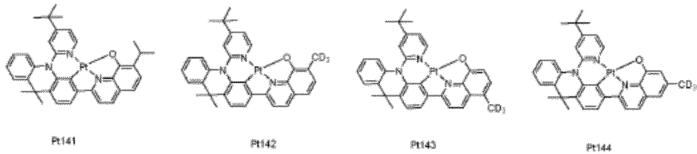

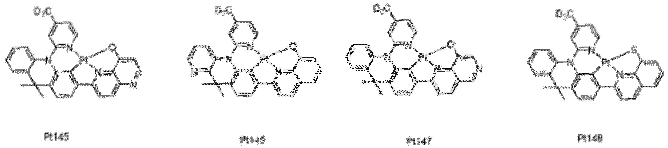

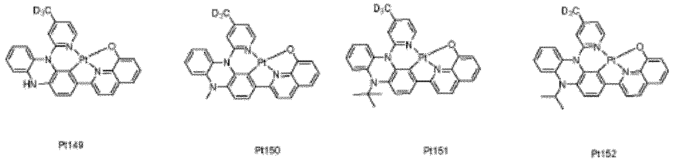

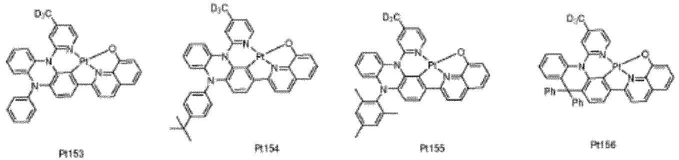

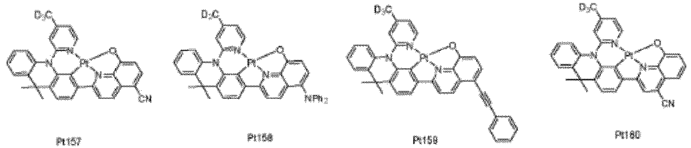

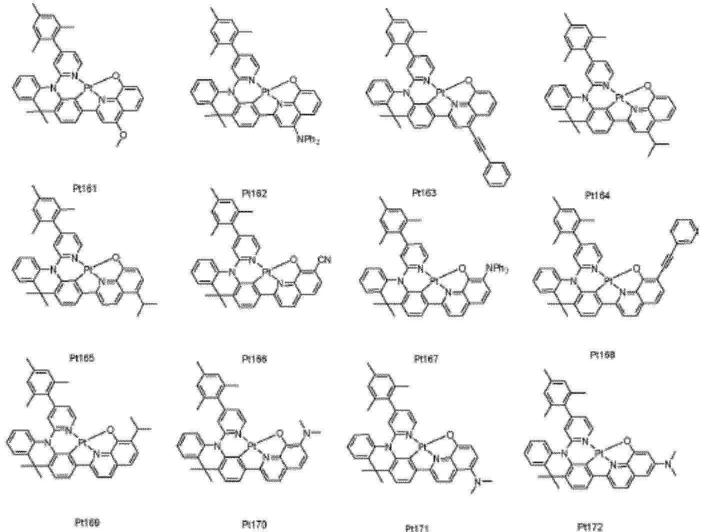

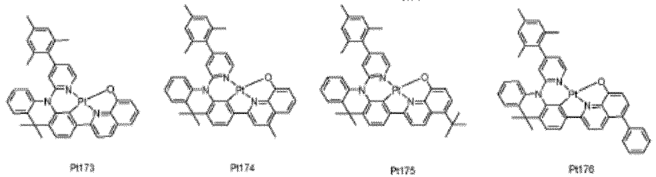

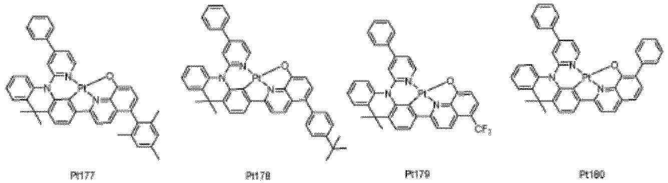

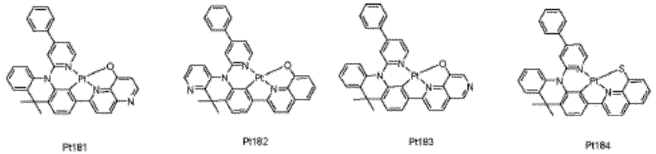

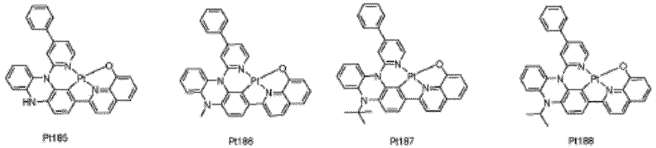

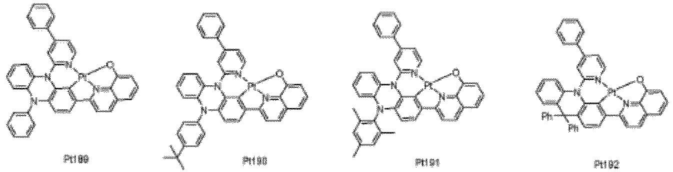

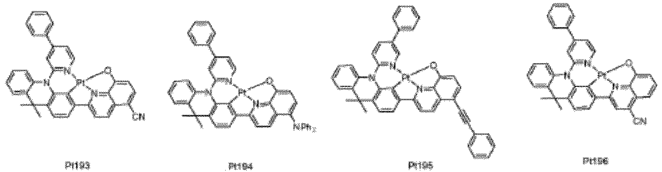

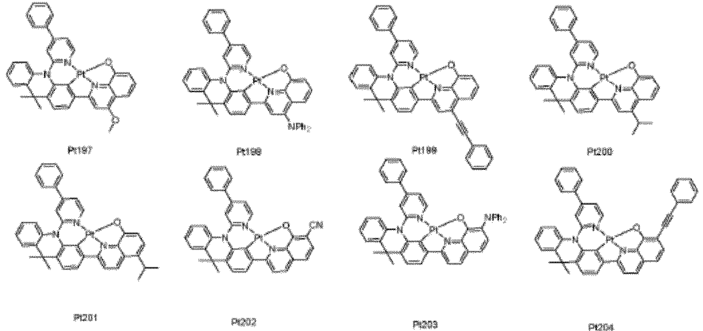

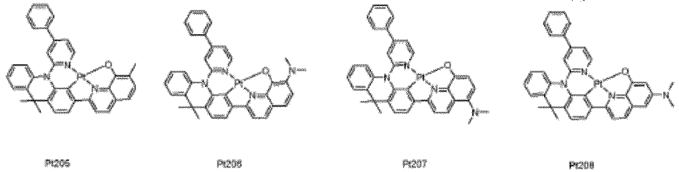

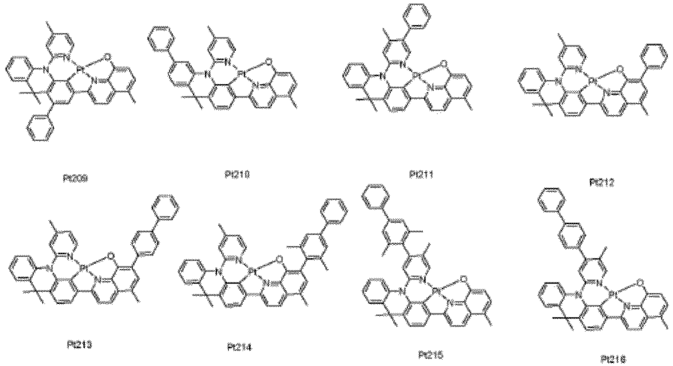

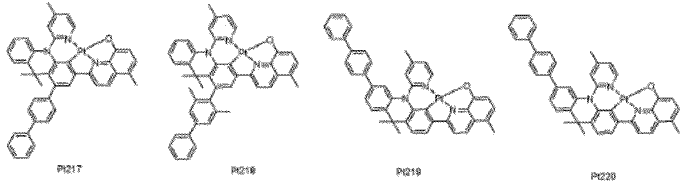

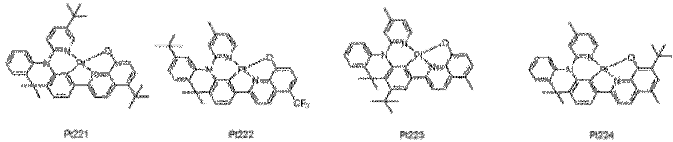

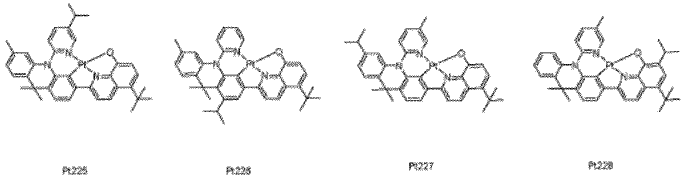

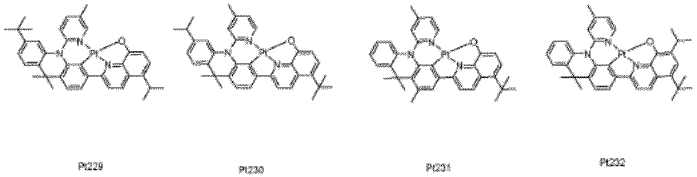

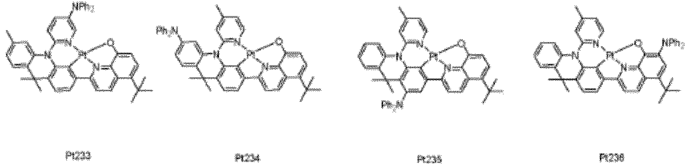

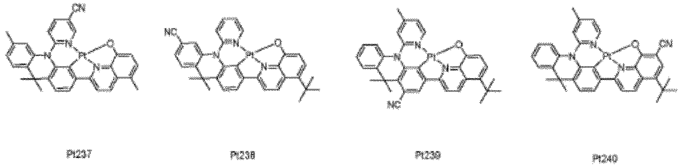

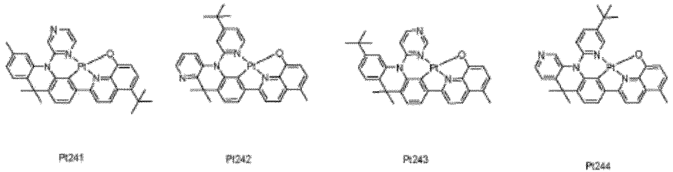

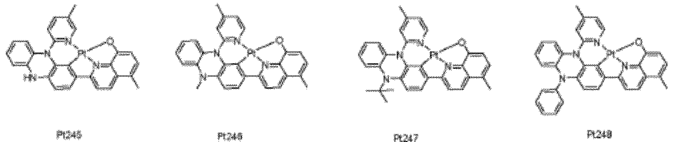

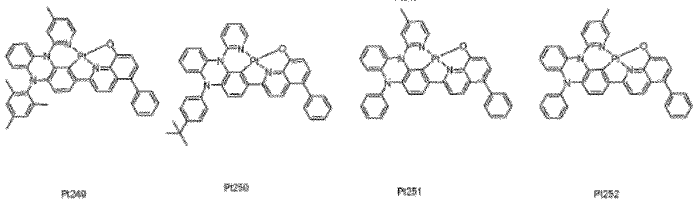

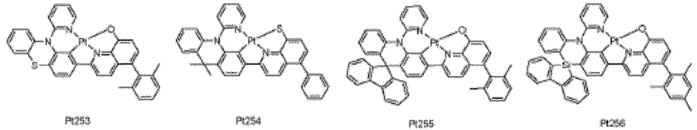

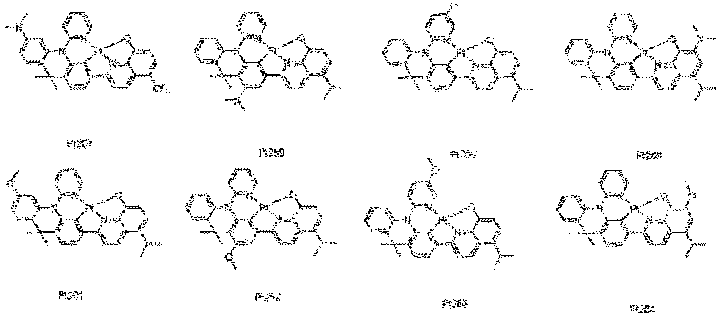

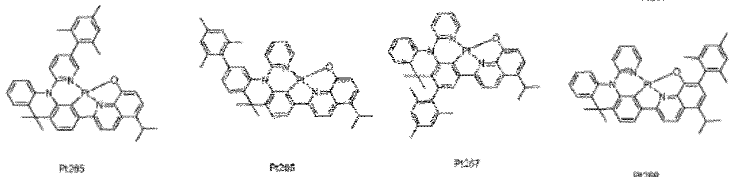

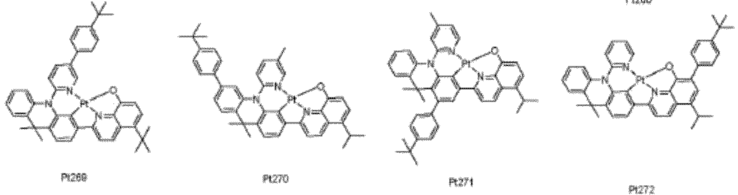

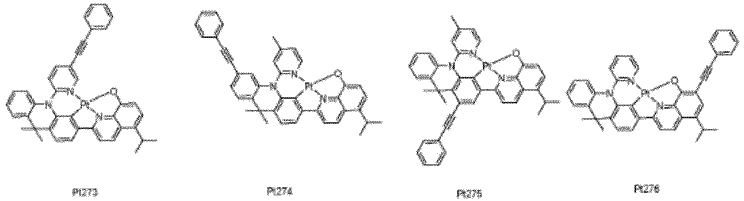

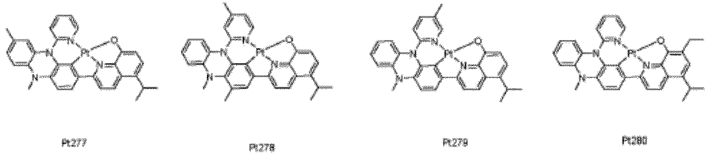

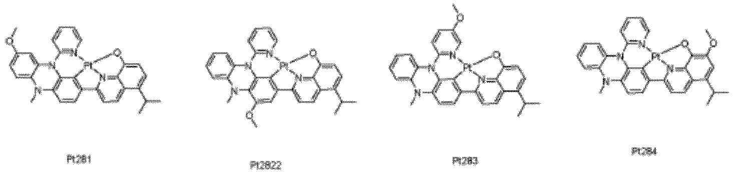

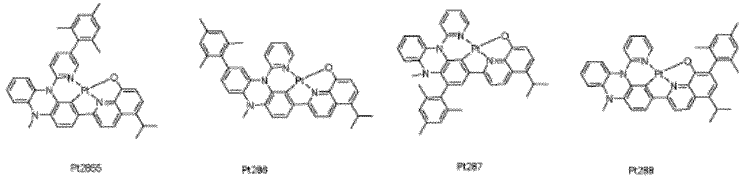

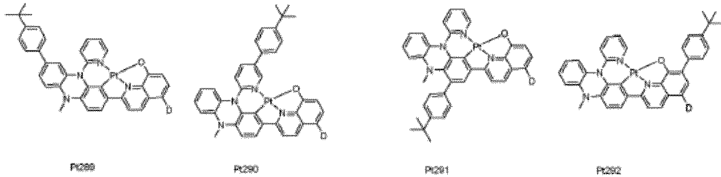

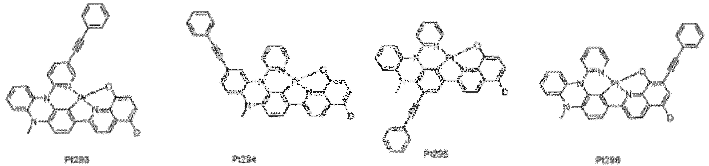

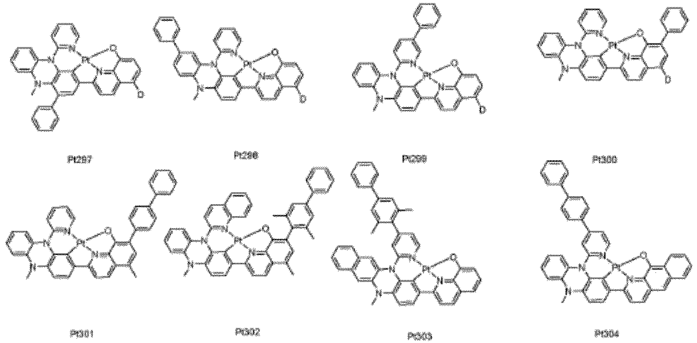

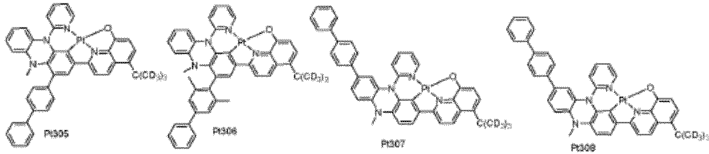

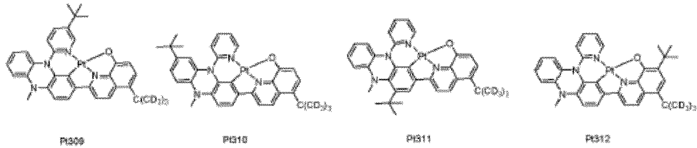

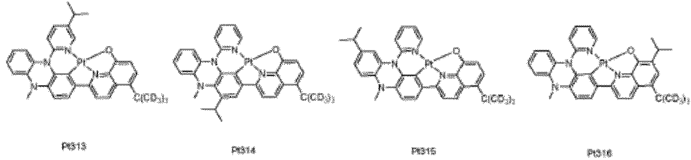

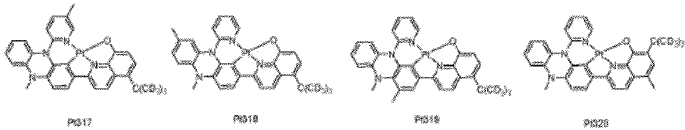

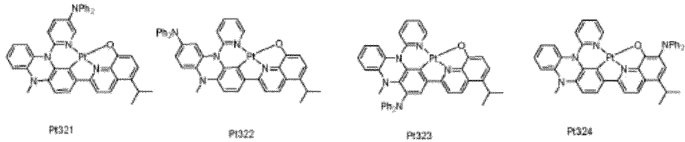

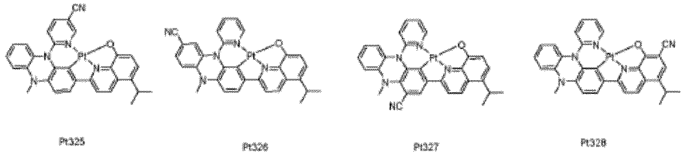

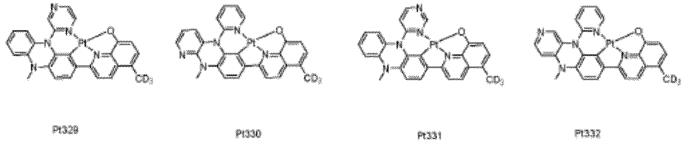

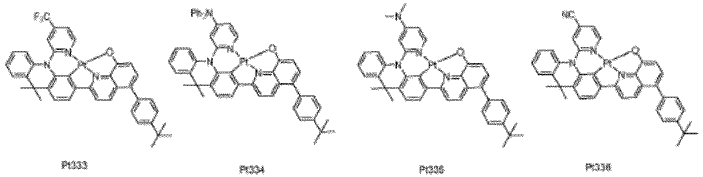

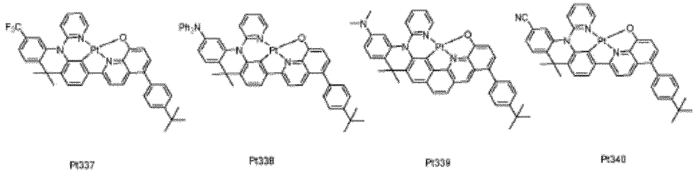

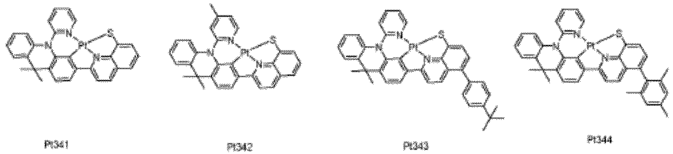

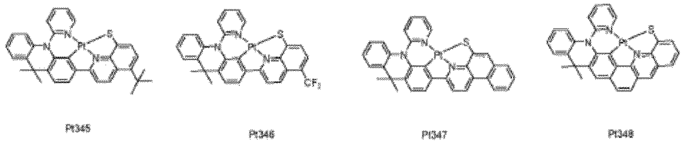

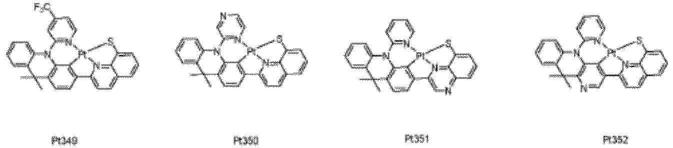

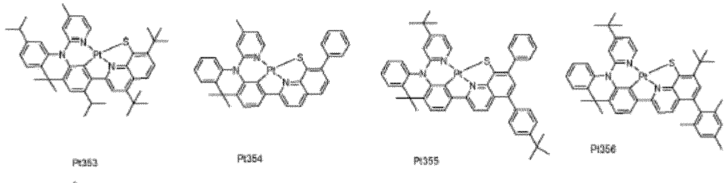

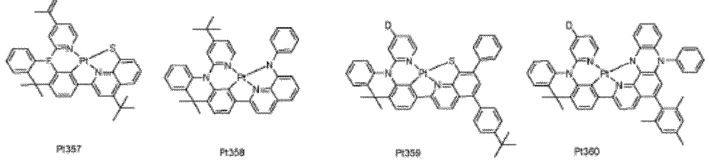

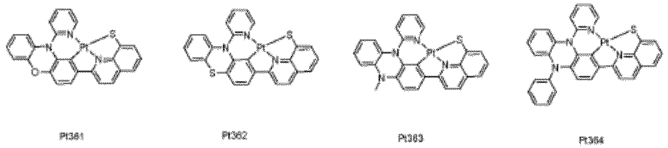

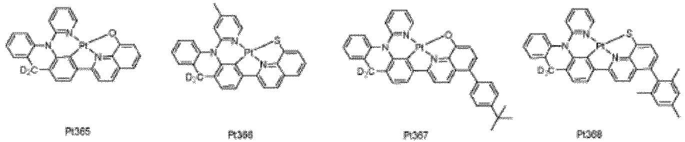

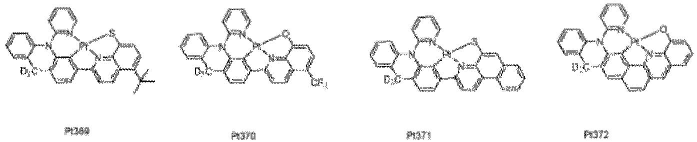

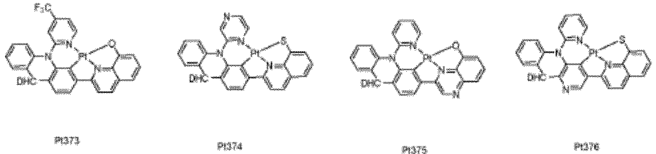

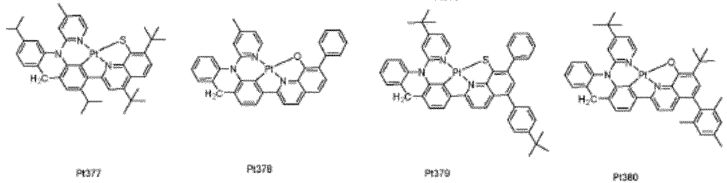

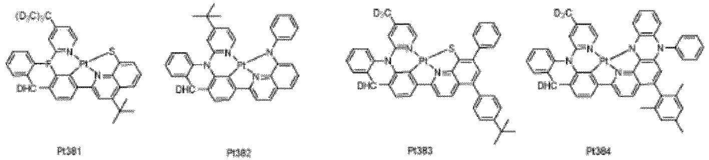

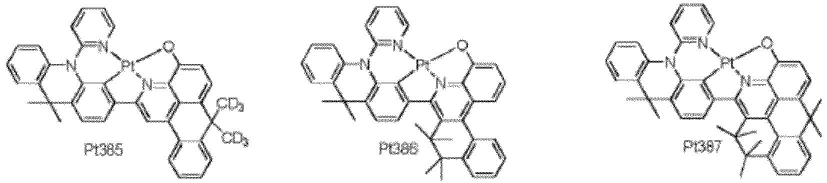

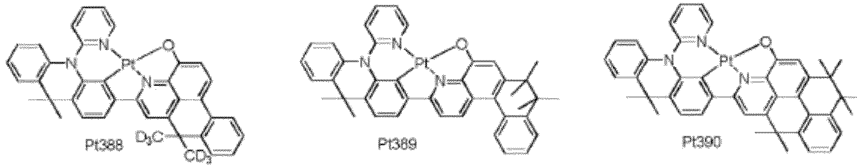

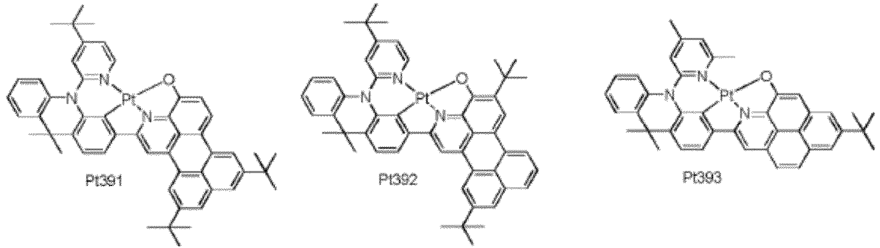

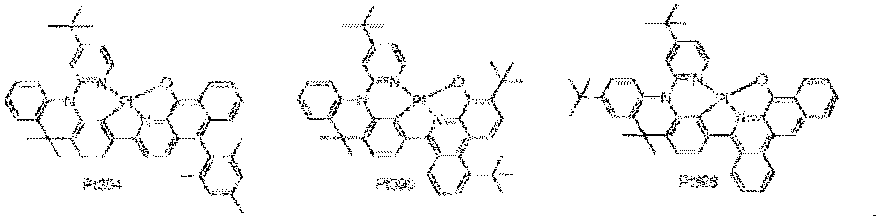

5. Anwendung des Metall-Platin(II)-und-Palladium(II)-Komplexes, enthaltend Chinolin-Struktureinheiten, nach einem der Ansprüche 1 bis 4 in Elektrolumineszenzvorrichtungen.

6. Vorrichtung, umfassend den Metall-Platin(II)-und-Palladium(II)-Komplex, enthaltend Chinolin-Struktureinheiten, nach einem der Ansprüche 1 bis 4.

7. Vorrichtung nach Anspruch 6, wobei die Vorrichtung eine Vollfarbanzeige, eine Photovoltaikvorrichtung, eine lichtemittierende Anzeigevorrichtung oder eine organische Leuchtdiode ist.

8. Vorrichtung nach Anspruch 6, umfassend mindestens eine Kathode, mindestens eine Anode und mindestens eine Lumineszenzschicht, wobei mindestens eine von der mindestens einen Lumineszenzschicht einen beliebigen von den Metall-Platin(II)-und-Palladium(II)-Komplexen, enthaltend Chinolin-Struktureinheiten, nach einem der Ansprüche 1 bis 4 umfasst.

9. Vorrichtung nach Anspruch 6, wobei die Vorrichtung eine organische Leuchtdiode mit einer lichtemittierenden Schicht ist, enthaltend den Metall-Platin(II)-und-Palladium(II)-Komplex, enthaltend Chinolin-Struktureinheiten, und ein entsprechendes Hauptmaterial, wobei ein Massenprozentanteil des Metall-Platin(II)-und-Palladium(II)-Komplexes 1 % bis 50 % beträgt und ein Massenprozentanteil des Hauptmaterials nicht beschränkt ist.

10. Anzeige- oder Beleuchtungsvorrichtung, umfassend die Vorrichtung nach einem der Ansprüche 6 bis 9.

## Revendications

1. Complexe de platine (II) et de palladium (II) contenant des unités structurales de quinoléine, **caractérisé en ce que** le complexe de platine (II) et de palladium (II) contenant des unités structurales de quinoléine possède une structure représentée dans la formule générale (I) suivante 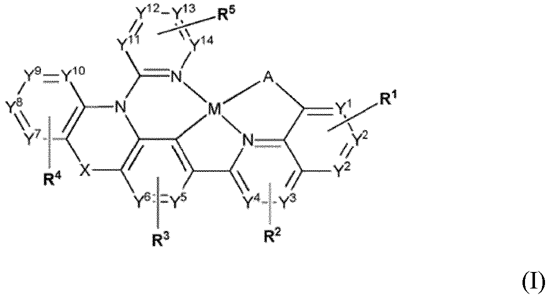 où
M est sélectionné parmi Pt ou Pd ;
A est sélectionné parmi 0, S ou NR⁶ ;
X représente O, S, CH₂, CHD, CD₂, CR ⁷R⁸, C=O, SiR⁹R¹⁰, NH, ND, NR¹¹, PH, PD, PR¹², R¹³P=O, S=O, ou SO₂;
Y¹, Y², Y³, Y⁴, Y⁵, Y⁶, Y⁷, Y⁸, Y⁹, Y¹⁰, Y¹¹, Y¹², Y¹³ et Y¹⁴ sont chacun indépendamment N ou CH ;
modes de substitution de R¹, R², R³, R⁴, R⁵, R⁶, R⁷, R⁸, R⁹, R¹⁰, R¹¹, R¹² et R¹³ sont chacun exprimés indépendamment en substitution simple, double substitution, triple substitution, quatre substitutions ou aucune substitution ; R¹, R², R³, R⁴, R⁵, R⁶, R⁷, R⁸, R⁹, R¹⁰, R¹¹, R¹² et R¹³ représentent chacun indépendamment hydrogène, deutérium, alkyle, haloalkyle, cycloalkyle, alcoxy, aryle, hétéroaryle, aryloxy, halogène, cycloalcényle, hétérocyclyle, alcényle, hydroxyle, mercapto, nitro, cyano, amino, mono ou dialkylamino, mono ou diarylamino, ester, nitrile, isonitrile, hétéroaryle, alcoxycarbonyle, amido, alcoxycarbonylamino, aryloxycarbonylamino, sulfonylamino, sulfamoyle, carbamoyle, des groupes alkylthio, sulfinyle, uréido, phosphoramido, imino, sulfo, carboxyle, hydrazino, silyle, silyle substitué, des groupes polymères ou des combinaisons de ceux-ci, et au moins deux groupes adjacents, R¹, R², R³, R⁴, R⁵, R⁶, R⁷, R⁸, R⁹, R¹⁰, R¹¹, R¹² et R¹³ sont liés sélectivement pour former un cycle condensé.

2. Complexe métallique de platine (II) et de palladium (II) contenant des unités structurales de quinoléine selon la revendication 1, le complexe de platine ayant des structures représentées dans les formules générales (II), (III), (IV) ou (V) suivantes : 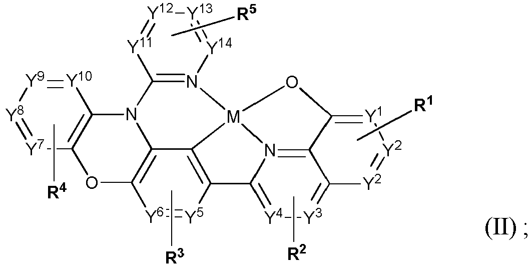 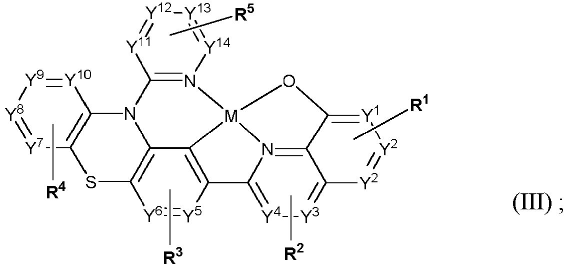 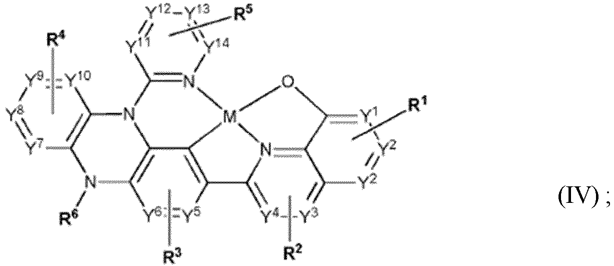 ou

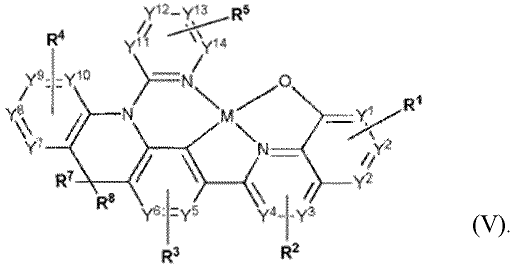

3. Le complexe métallique de platine (II) et de palladium (II) contenant de la quinoléine structurel unités selon la revendication 1, 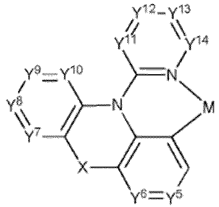 sur la gauche est de préférence d'une structure choisie parmi l'une des structures suivantes :

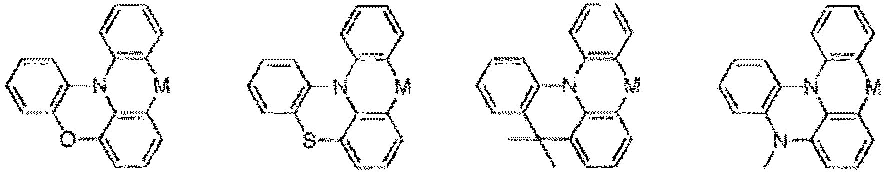

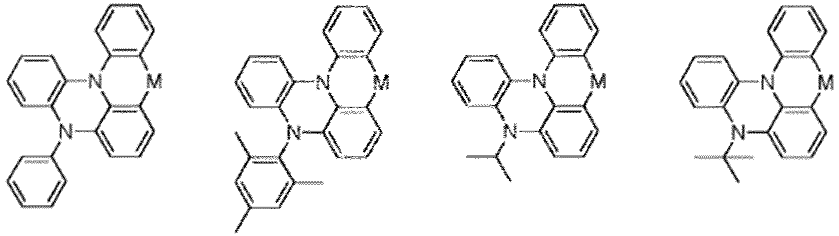

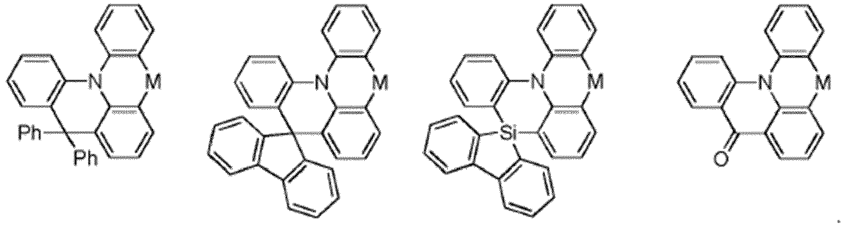

4. Complexe métallique de platine (II) et de palladium (II) contenant des unités structurales de quinoléine selon la revendication 1, le complexe de platine (II) et de palladium (II) présentant l'une des structures suivantes :

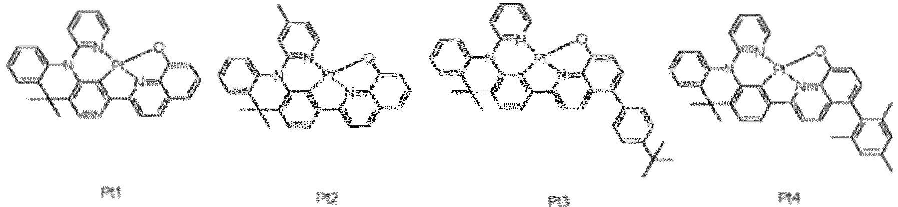

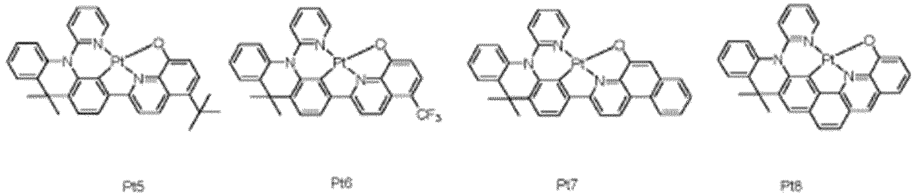

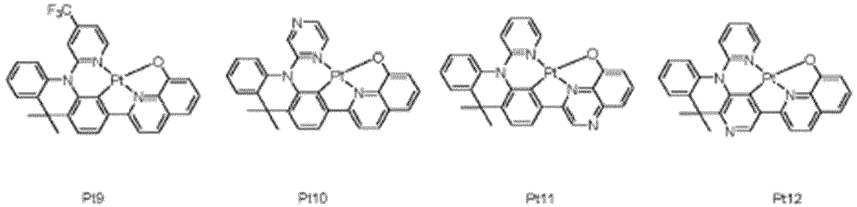

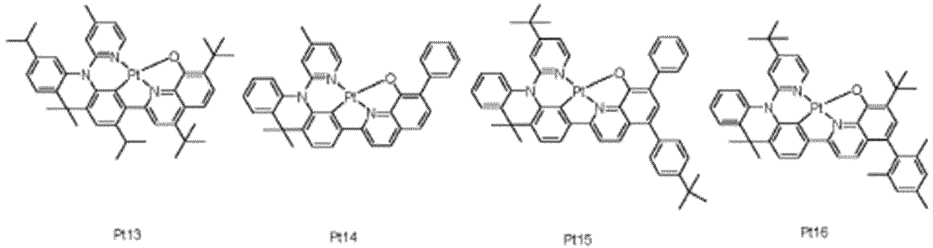

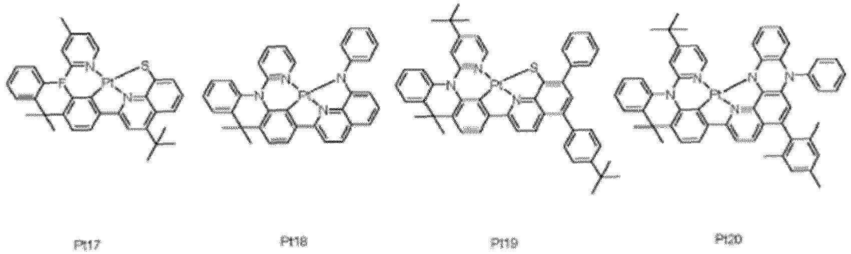

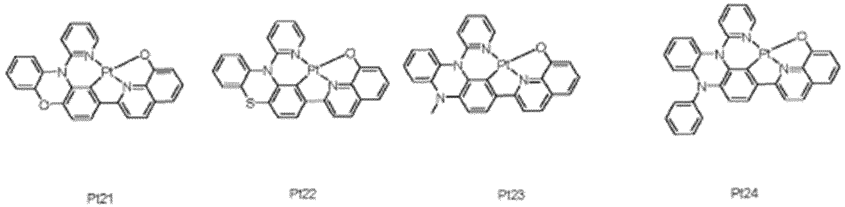

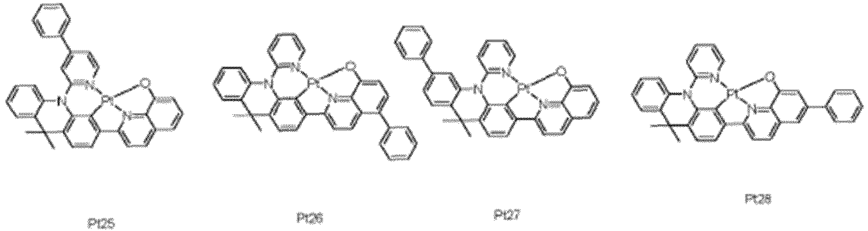

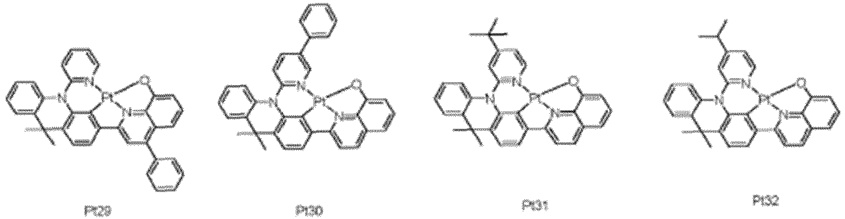

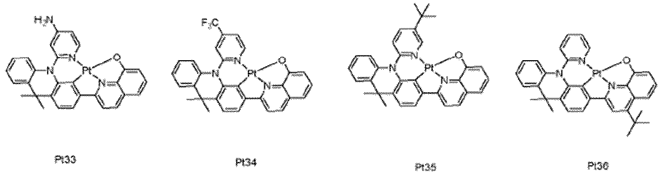

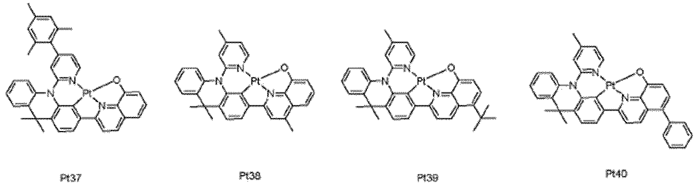

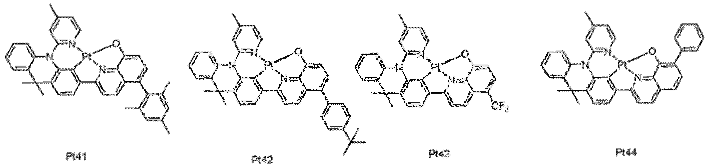

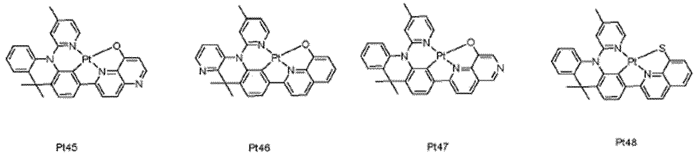

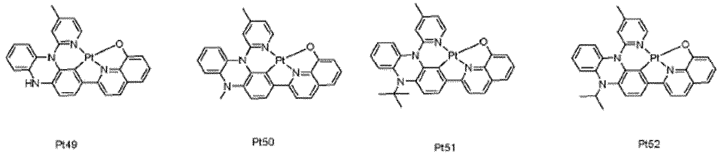

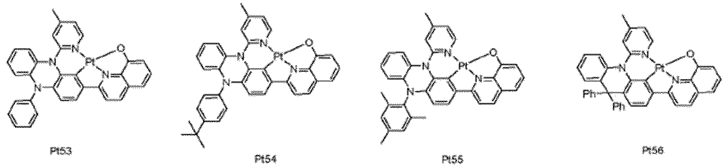

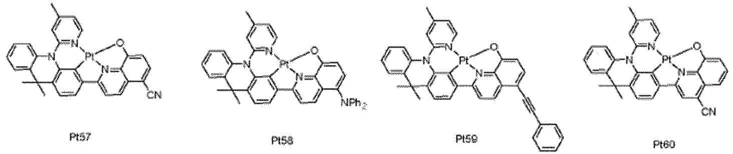

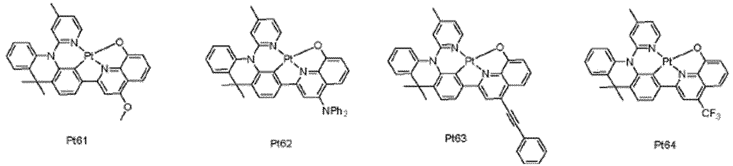

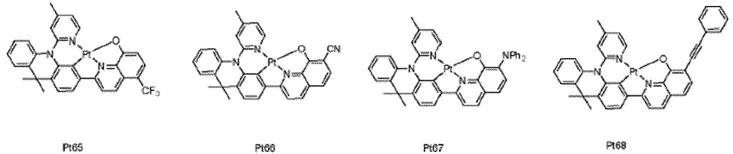

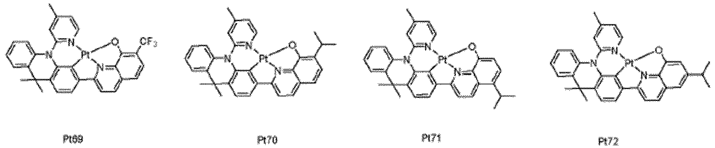

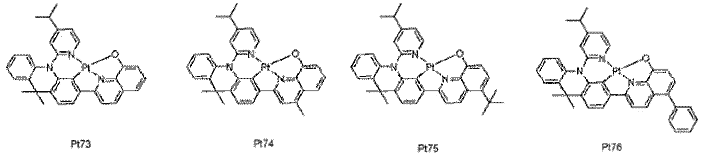

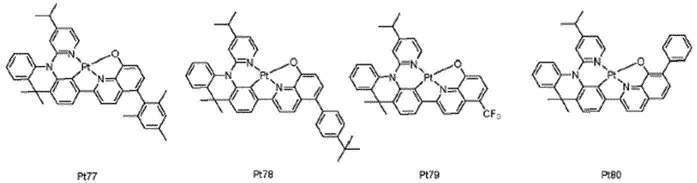

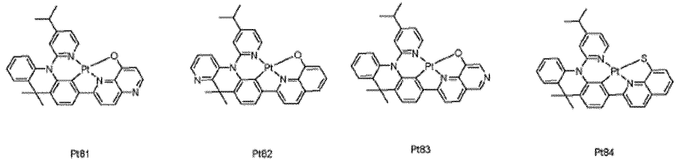

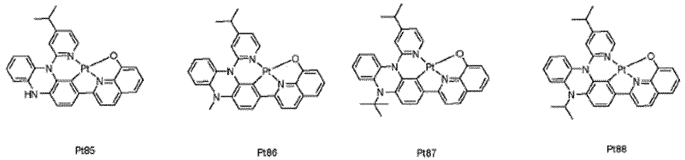

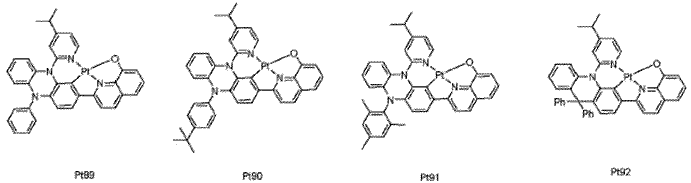

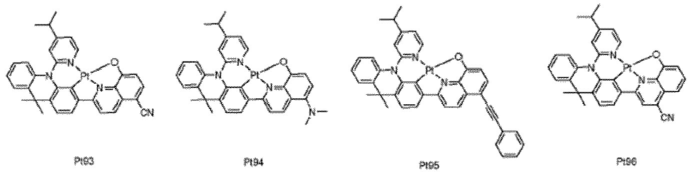

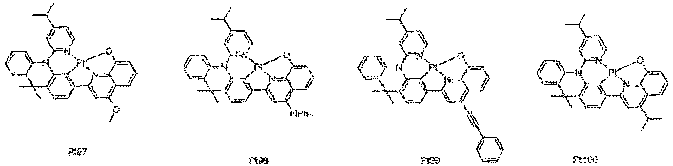

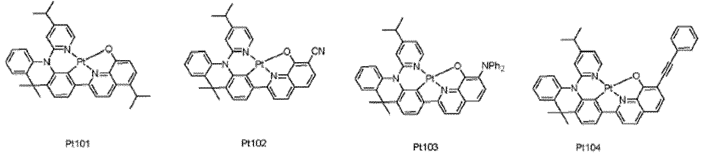

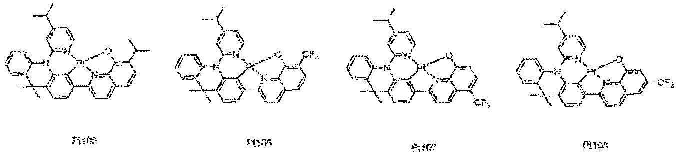

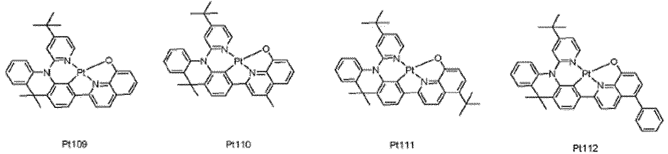

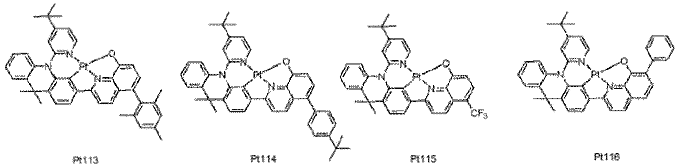

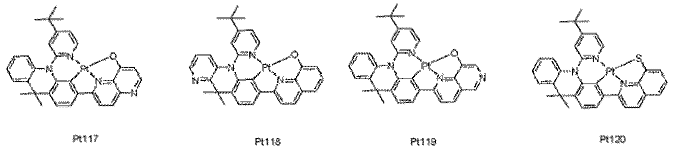

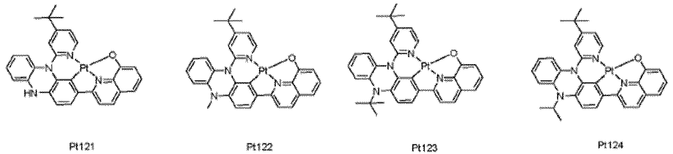

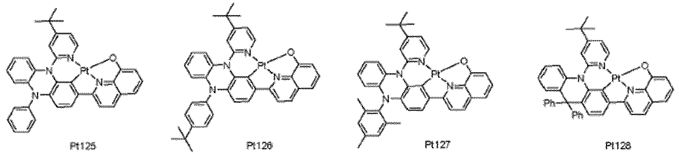

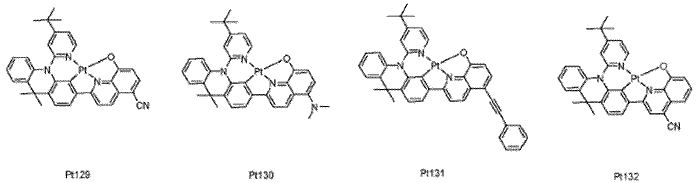

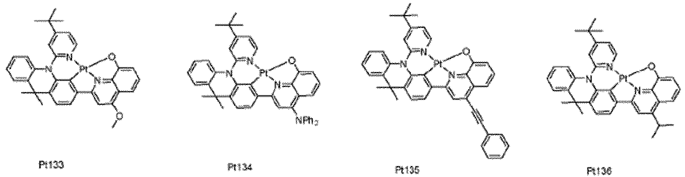

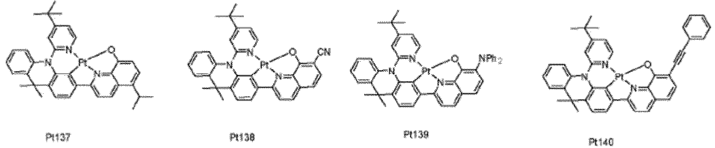

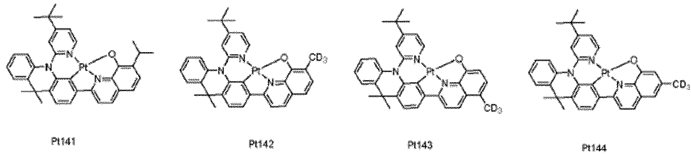

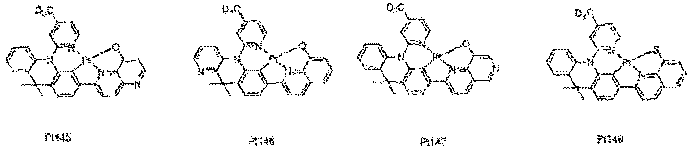

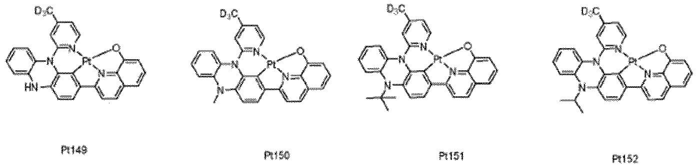

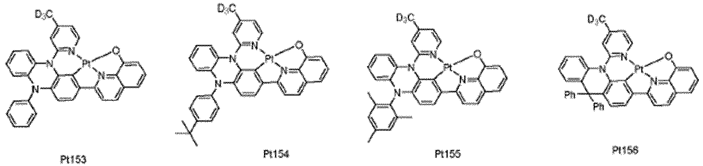

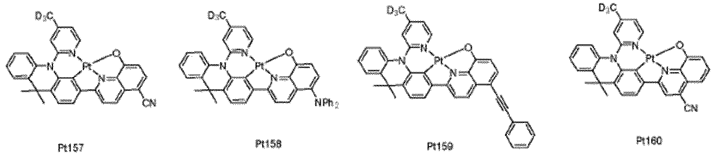

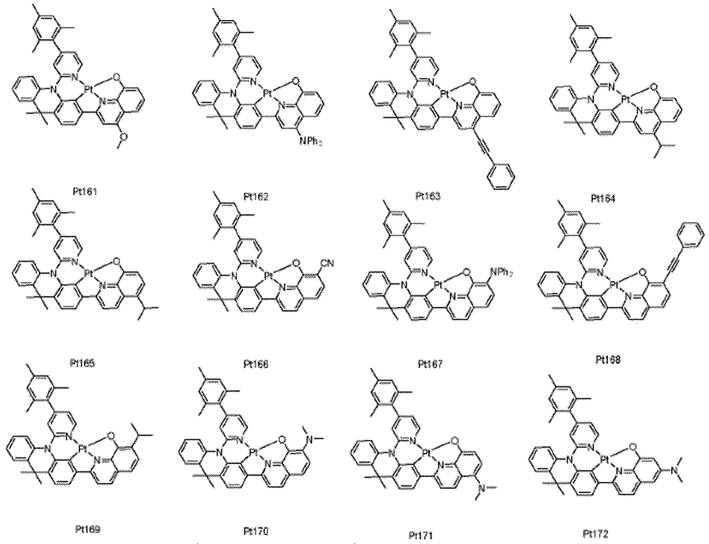

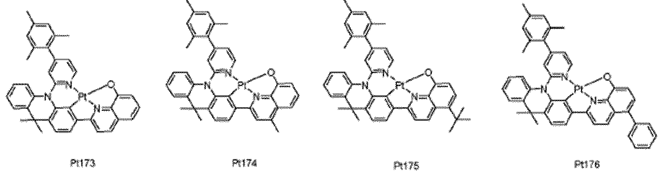

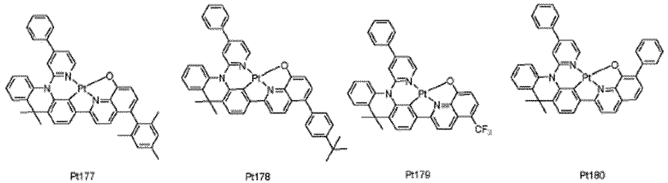

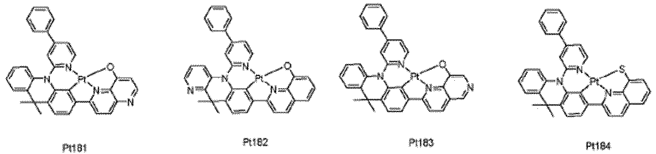

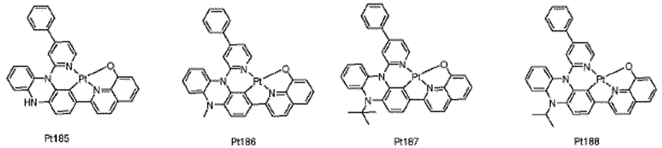

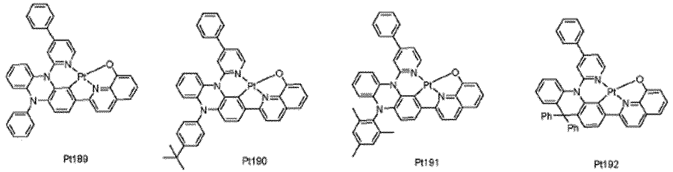

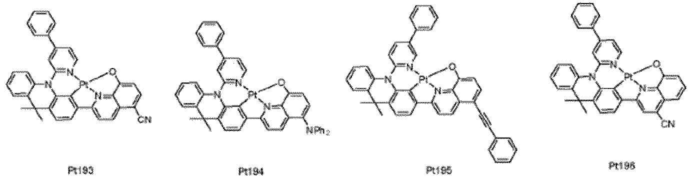

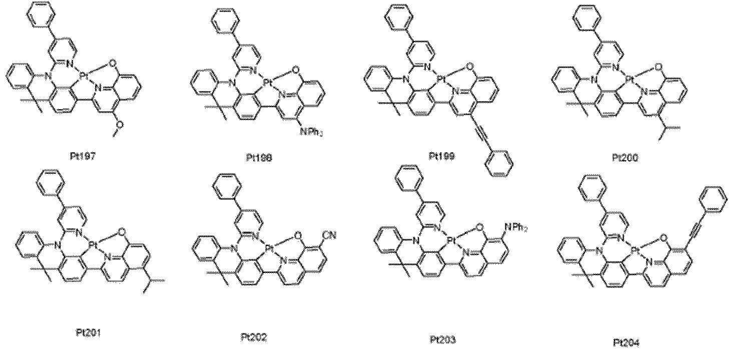

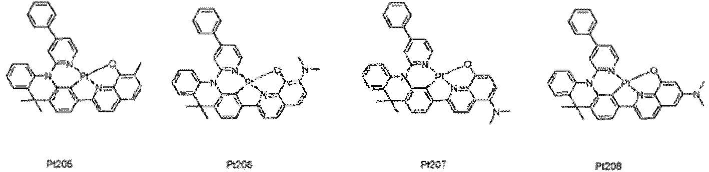

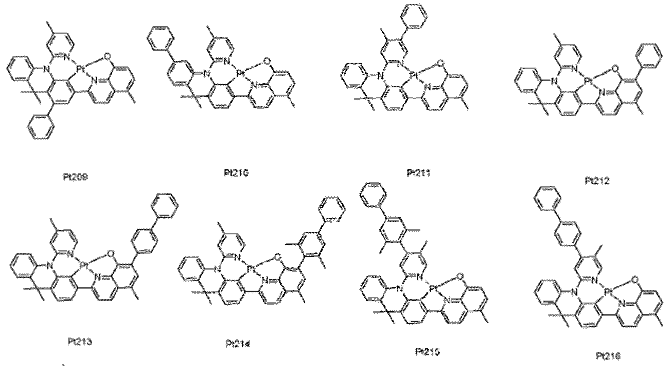

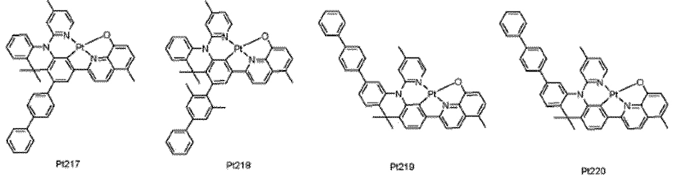

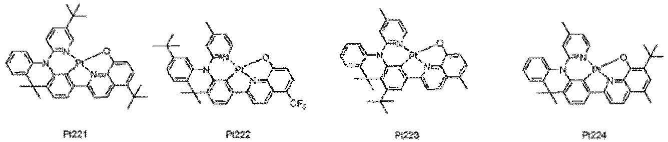

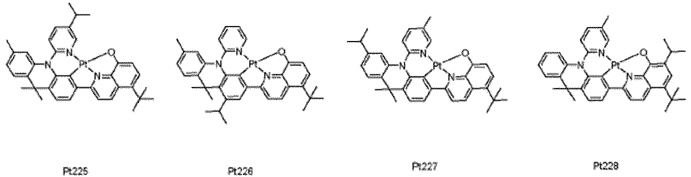

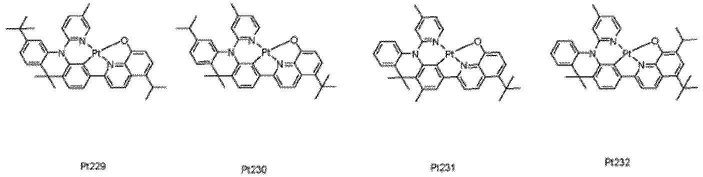

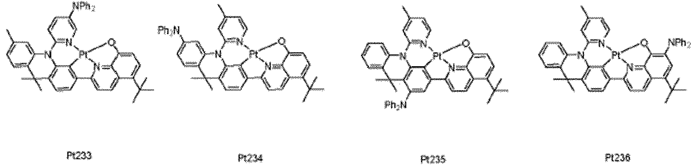

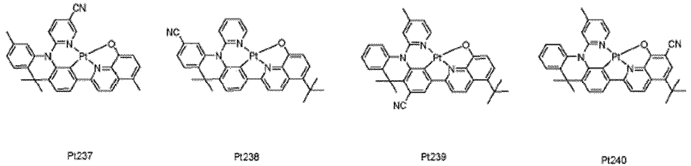

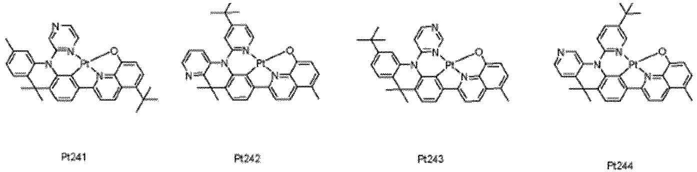

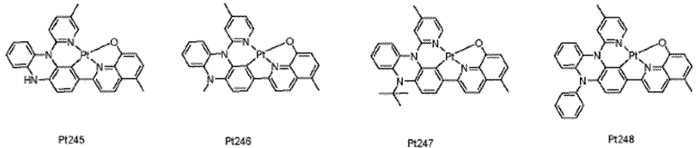

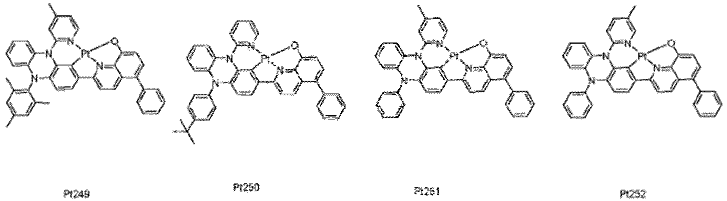

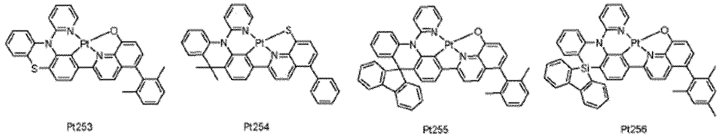

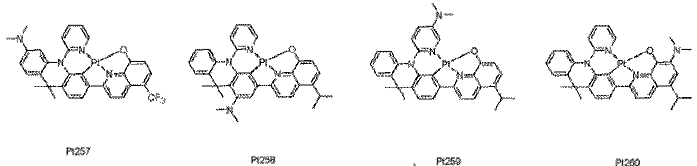

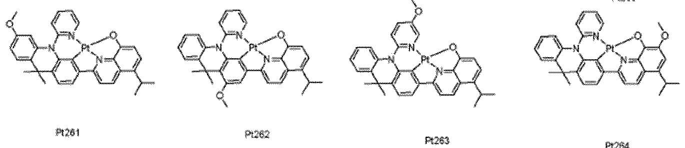

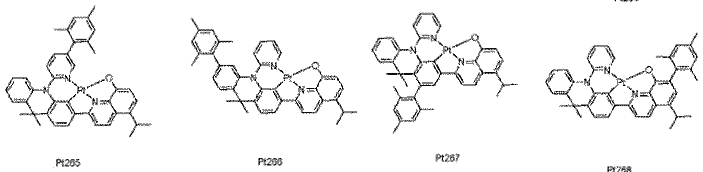

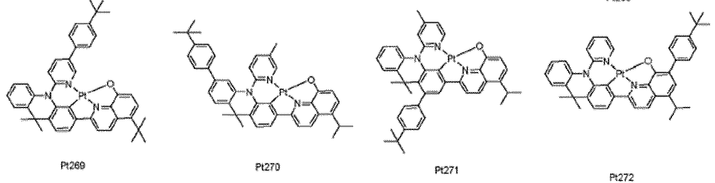

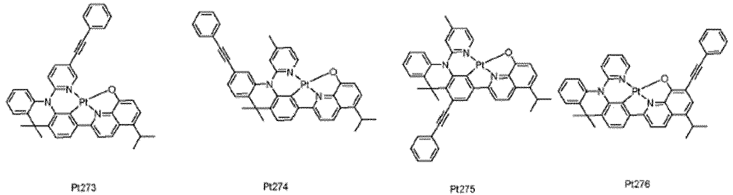

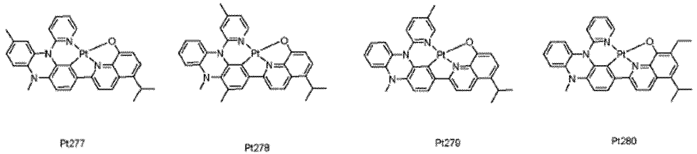

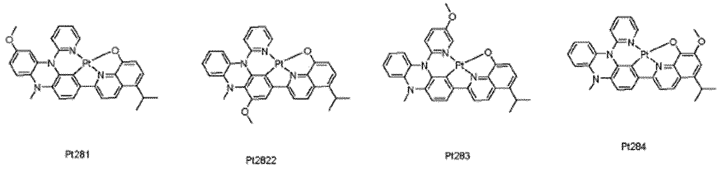

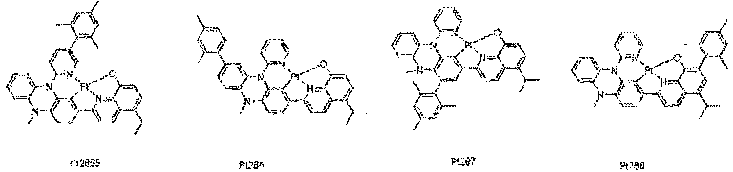

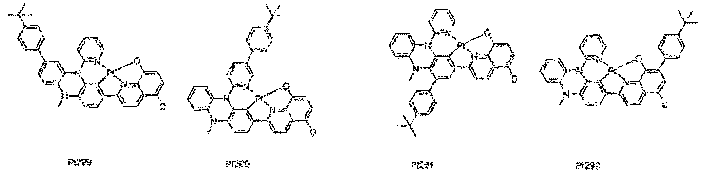

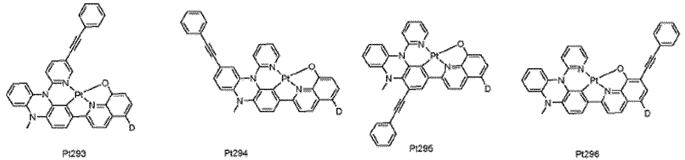

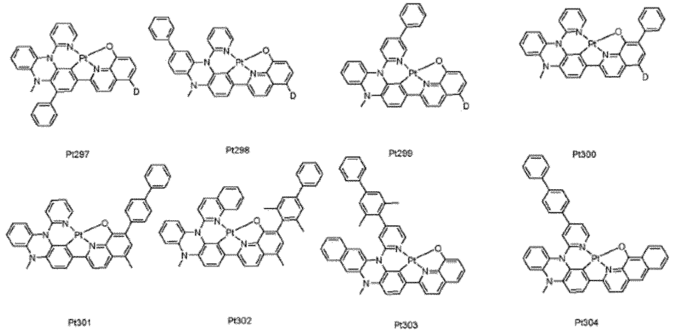

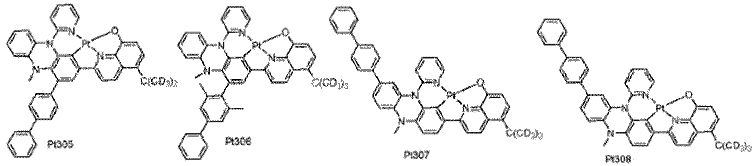

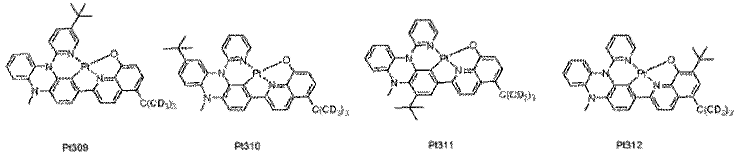

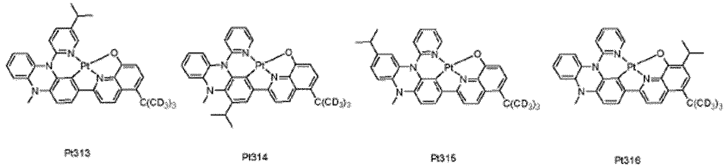

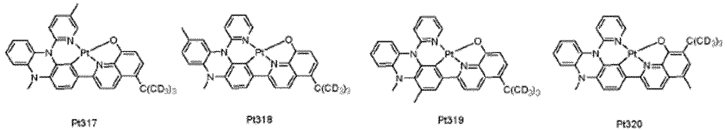

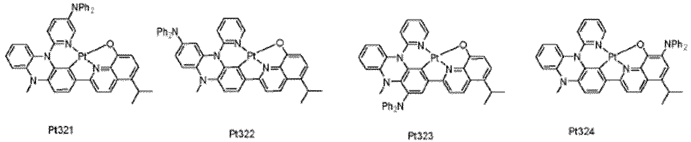

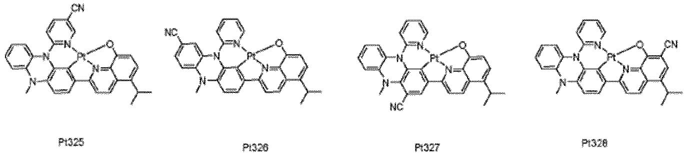

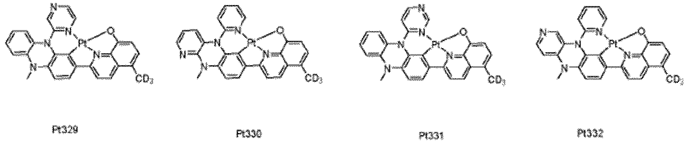

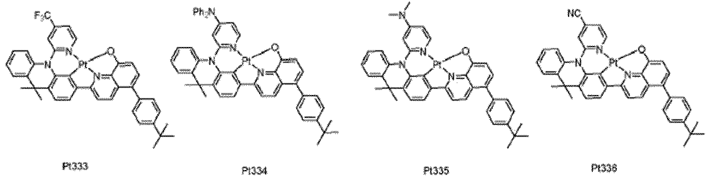

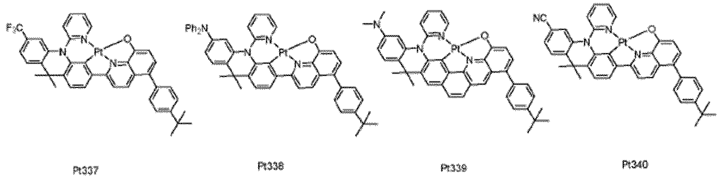

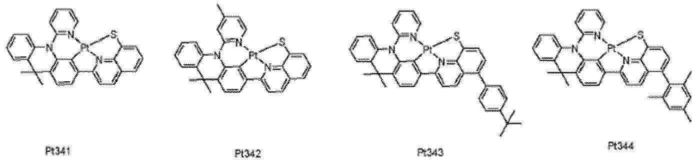

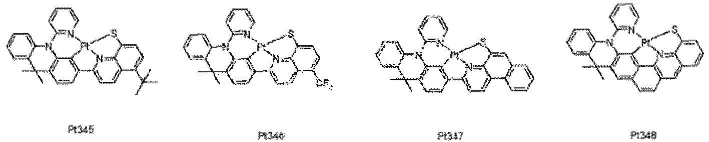

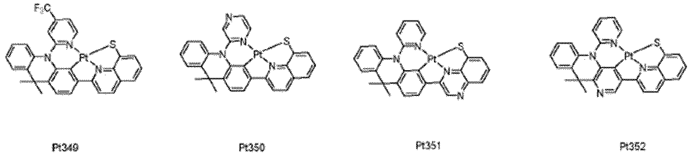

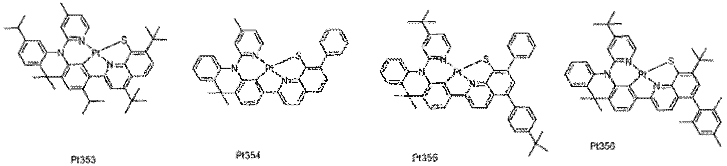

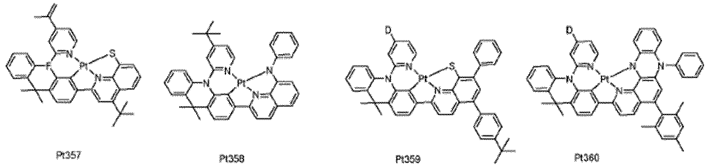

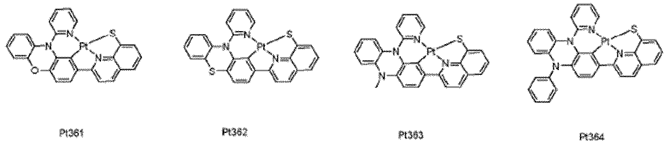

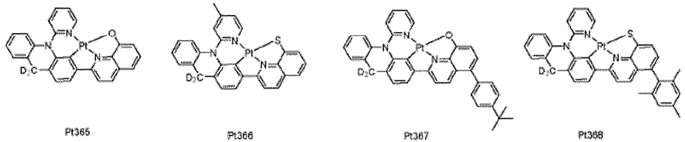

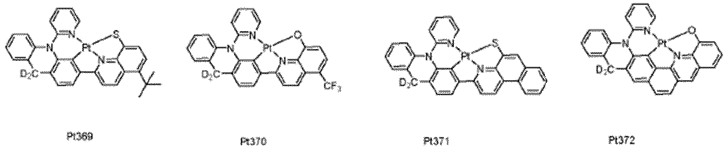

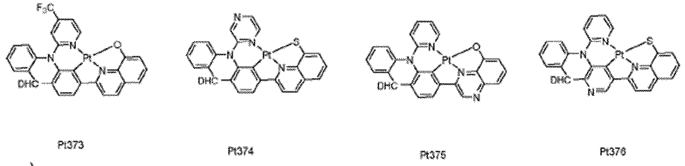

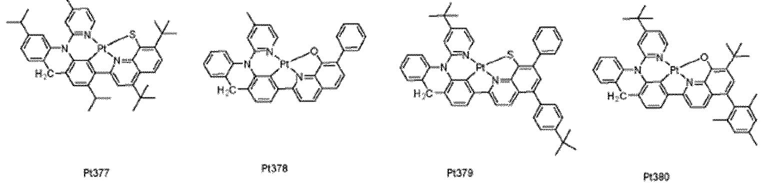

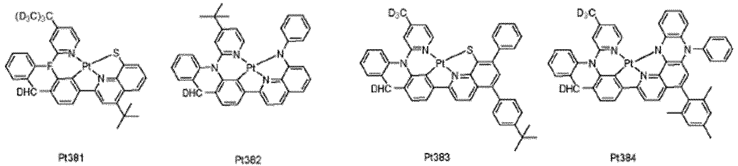

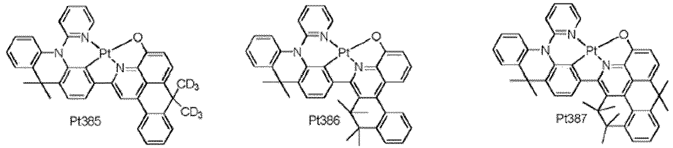

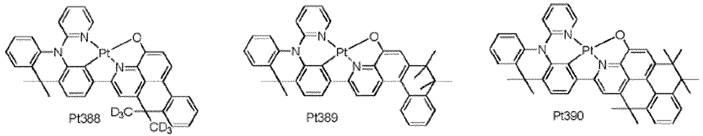

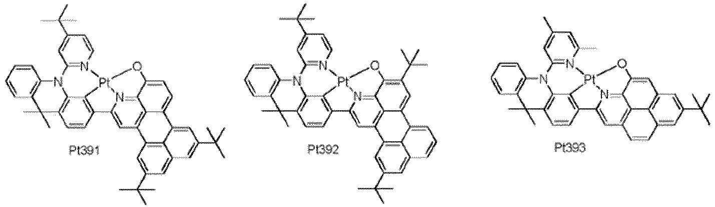

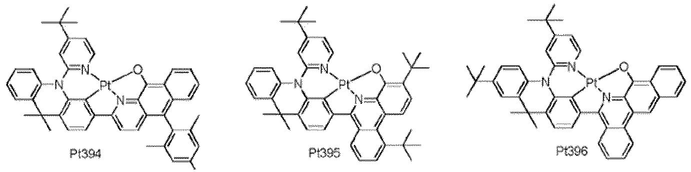

5. Application du complexe métallique de platine (II) ou de palladium (II) contenant des unités structurales de quinoléine selon n'importe laquelle des revendications I à 4 dans des dispositifs électroluminescents.

6. Dispositif comprenant le complexe métallique de platine (II) ou de palladium (II) contenant des unités structurales de quinoléine selon n'importe laquelle des revendications 1 à 4.

7. Dispositif selon la revendication 6, **caractérisé en ce que** le dispositif est un écran polychrome, un dispositif photovoltaïque, un dispositif d'affichage électroluminescent ou une diode électroluminescente organique.

8. Dispositif selon la revendication 6, comprenant au moins une cathode, au moins une anode et au moins une couche luminescente, au moins l'une des couches luminescentes comprenant un des complexes métalliques de platine (II) et de palladium (II) contenant des unités structurales de quinoléine selon n'importe laquelle des revendications 1 à 4.

9. Dispositif selon la revendication 6, **caractérisé en ce que** le dispositif est une diode électroluminescente organique, avec une couche électroluminescente contenant le complexe métallique de platine (II) ou de palladium (II) contenant des unités structurales quinoléiques et un matériau principal correspondant, un pourcentage en masse du complexe métallique de platine (II) ou de palladium (II) étant compris entre 1 % et 50 %, et un pourcentage en masse du matériau principal n'étant pas limité.

10. Dispositif d'affichage ou d'éclairage, comprenant le dispositif selon n'importe laquelle des revendications 6 à 9.
